# EUROPÄISCHE PATENTSCHRIFT

(11) **EP 1 513 834 B1**
(45) Veröffentlichungstag und Bekanntmachung des Hinweises auf die Patenterteilung: **15.03.2006**
(21) Anmeldenummer: 03740148.6
(22) Anmeldetag: 26.05.2003
(51) Int. Cl.: C07D 409/12, A61K 31/4168, A61P 11/00

(54) **SUBSTITUIERTE THIOPHENE, VERFAHREN ZU IHRER HERSTELLUNG, IHRE VERWENDUNG ALS MEDIKAMENT ODER DIAGNOSTIKUM SOWIE SIE ENTHALTENDES MEDIKAMENT**
SUBSTITUTED THIOPHENES, METHOD FOR THE PRODUCTION THEREOF, THEIR USE AS A MEDICAMENT OR DIAGNOSTIC REAGENT, AND A MEDICAMENT CONTAINING THE SAME
THIOPHENES SUBSTITUES, PROCEDES DE FABRICATION, UTILISATION EN TANT QUE MEDICAMENT OU AGENT DIAGNOSTIQUE ET MEDICAMENT CONTENANT CES THIOPHENES

(30) Priorität: 04.06.2002 DE 10224892
(43) Veröffentlichungstag der Anmeldung: 16.03.2005
(73) Patentinhaber: Sanofi-Aventis Deutschland GmbH, 65929 Frankfurt am Main (DE)
(72) Erfinder: LANG, Hans-Jochen, 65719 Hofheim (DE); HEINELT, Uwe, 65187 Wiesbaden (DE); HOFMEISTER, Armin, 55276 Oppenheim (DE); WIRTH, Klaus, 65830 Kriftel (DE); GEKLE, Michael, 97072 Würzburg (DE); BLEICH, Markus, 65597 Hünfelden-Dauborn (DE)
(86) Internationale Anmeldenummer: PCT/EP2003/005465
(87) Internationale Veröffentlichungsnummer: WO 2003/101984

(56) Entgegenhaltungen:
- US-A- 3 758 476

## Beschreibung

Die Erfindung betrifft Verbindungen vom Typ der substituierten Thiophen-Derivate der Formel I. Medikamente, die Verbindungen dieses Typs enthalten, sind nützlich bei der Prävention oder Behandlung diverser Erkrankungen. So lassen sich die Verbindungen unter anderem verwenden zur Behandlung von Atemstörungen und des Schnarchens sowie zur Verbesserung des Atemantriebes, zur Behandlung von akuten und chronischen Erkrankungen, durch ischämische und/oder Reperfusionsereignisse sowie durch proliferative oder durch fibrotische Ereignisse ausgelösten Erkrankungen, zur Behandlung oder Prophylaxe von Erkrankungen des zentralen Nervensystems, des Fettstoffwechsels und des Diabetes, der Blutgerinnung und des Befalls durch Parasiten.

Die Erfindung betrifft Verbindungen der Formel I, 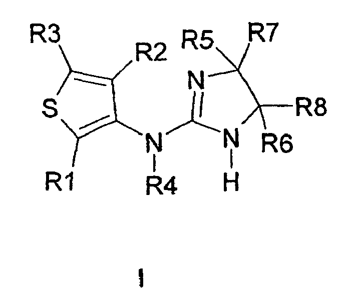
worin bedeuten:
- R1 und R2: unabhängig voneinander H, F, CI, Br, I, CN, NO₂, -(X)ₙ-C_{q}H_{2q}-Z, Cycloalkyl mit 3, 4, 5 oder 6 C-Atomen, Alkenyl mit 2, 3 oder 4 C-Atomen, Alkenylalkyl mit 3 oder 4 C-Atomen, Ethinyl oder Alkylalkinyl mit 3 oder 4 C-Atomen;
n Null oder 1;
X Sauerstoff, NH, N-CH₃, S(O)ₖ;
k Null, 1 oder 2;
q Null, 1, 2, 3, 4, 5 oder 6;
Z Wasserstoff oder CₘF_{2m+1;}
m Null, 1, 2, 3 oder 4;
- R3: Wasserstoff, Methyl, F, Cl, Br, I, CN, S(O)ₖ-CH₃, -NO₂, -O-CH₃;
k Null, 1 oder 2;
- R4: Wasserstoff, Cycloalkyl mit 3, 4, 5, oder 6 C-Atomen, Alkenyl mit 2, 3 oder 4 C-Atomen, Alkenylalkyl mit 3 oder 4 C-Atomen, Ethinyl oder Alkylalkinyl mit 3 oder 4 C-Atomen, -CᵣH₂ᵣ- Y;
r Null, 1, 2, 3 oder 4;
Y Wasserstoff oder Trifluormethyl;
- R5 und R6: Wasserstoff oder gemeinsam eine Bindung;
- R7 und R8: unabhängig voneinander (C₃-C₅)-Alkyl-, (C₂-C₅)-Alkenyl, (C₂-C₅)-Alkinyl, (C₃-C₆)-Cycloalkyl oder (C₄-C₆)-Cycloalkenyl

oder
- R7 und R8: gemeinsam eine Alkylenkette bestehend aus 3 bis 8 C-Atomen; wobei deren Wasserstoffatome nicht, teilweise oder vollständig durch Fluoratome ersetzt sein können;

oder
- R7 und R8: gemeinsam einen Rest 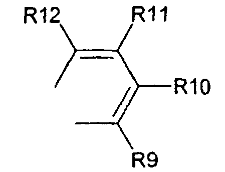
wobei R5 und R6 gemeinsam eine Bindung bilden;
R10 und R11 unabhängig voneinander Wasserstoff, Fluor, Chlor, Brom, Methyl, CN, OH, -O-CH₃, NO₂, NH₂ oder -CF₃;
- R9 und R12: Wasserstoff oder F; oder
- einer der Substituenten R9 und R12: Wasserstoff;
und der andere F, CI, Br, I, CN, NO₂, COOH, CO-NR13R14, SO₂₋NR13R14, Alkenyl mit 2, 3 oder 4 C-Atomen, Alkenylalkyl mit 3 oder 4 C-Atomen, Ethinyl, Alkylalkinyl mit 3 oder 4 C-Atomen oder -(X)ₙ₋C_{q}H_{2q}-Z;
R13 und R14 gleich oder verschieden Wasserstoff oder Alkyl mit 1, 2, 3 oder 4 C-Atomen;
oder
R13 und R14 bilden zusammen mit dem Stickstoff, an den sie gebunden sind einen gesättigten 5-, 6- oder 7-gliedrigen Ring;
n Null oder 1;
X Sauerstoff, NH, N-CH₃, S(O)ₖ;
k Null, 1 oder 2;
q Null, 1, 2, 3, 4, 5 oder 6;
und
Z Wasserstoff oder CₘF_{2m+1;}
m Null, 1, 2, 3 oder 4;

sowie deren pharmazeutisch verträgliche Salze, sowie deren Trifluoressigsäuresalze.

Eine Ausführungsform betrifft Verbindungen der Formel I, worin bedeuten
- R1 und R2: unabhängig voneinander H, F, Cl, Br, I, CN, NO₂, -(X)ₙ - C_{q}H_{2q} - Z, Cycloalkyl mit 3, 4, 5 oder 6 C-Atomen, Alkenyl mit 2, 3 oder 4 C-Atomen, Alkenylalkyl mit 3 oder 4 C-Atomen, Ethinyl oder Alkylalkinyl mit 3 oder 4 C-Atomen;
n Null oder 1;
X Sauerstoff, NH, N-CH₃, S(O)ₖ
k Null, 1 oder 2;
q Null, 1, 2, 3, 4, 5 oder 6;
Z Wasserstoff oder CₘF_{2m+1;}
m Null, 1, 2, 3 oder 4;
- R3: Wasserstoff, Methyl, F, Cl, Br, I, CN, S(O)ₖ-CH₃, -NO₂, -O- CH₃;
- R4: Wasserstoff, Cycloalkyl mit 3, 4, 5, oder 6 C-Atomen, Alkenyl mit 2, 3 oder 4 C-Atomen, Alkenylalkyl mit 3 oder 4 C-Atomen, Ethinyl oder Alkylalkinyl mit 3 oder 4 C-Atomen, -C_{q}H_{2q} - Z;
q Null, 1, 2, 3 oder 4;j
Z Wasserstoff oder Trifluormethyl;
- R5 und R6: Wasserstoff oder gemeinsam eine Bindung;
- R7 und R8: unabhängig voneinander (C₃-C₅)-Alkyl-, (C₂-C₅)-Alkenyl, (C₂-C₅)-Alkinyl, (C₃-C₆)-Cycloalkyl oder (C₄-C₆)-Cycloalkenyl

oder
- R7 und R8: gemeinsam eine Alkylenkette bestehend aus 3 bis 8 C-Atomen; wobei deren Wasserstoffatome nicht, teilweise oder vollständig durch Fluoratome ersetzt sein können;

oder
- R7 und R8: gemeinsam einen Rest 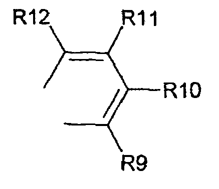
wobei R5 und R6 gemeinsam eine Bindung bilden;
R9, R10 und R11 unabhängig voneinander Wasserstoff, Fluor, Chlor, Brom, Methyl, CN, OH, -O-CH₃, NO₂, NH₂ oder -CF₃;
R9 und R12 Wasserstoff;
oder
einer der Substituenten R9 und R12 Wasserstoff;
und der andere F, Cl, Br, I, CN, NO₂, COOH, CO-NR13R14, SO₂-NR13R14, Alkenyl mit 2, 3 oder 4 C-Atomen, Alkenylalkyl mit 3 oder 4 C-Atomen, Ethinyl, Alkylalkinyl mit 3 oder 4 C-Atomen oder -(X)ₙ - CqH₂q-Z;
R13 und R14 gleich oder verschieden Wasserstoff oder Alkyl mit 1, 2, 3 oder 4 C-Atomen;
n Null oder 1;
X Sauerstoff, NH, N-CH₃, S(O)ₖ;
k Null, 1 oder 2;
q Null, 1, 2, 3, 4, 5 oder 6;
und
Z Wasserstoff oder CₘF₂ₘ₊₁;
m Null, 1, 2, 3 oder 4;
sowie deren pharmazeutisch verträgliche Salze, sowie deren Trifluoressigsäuresalze.

Bevorzugt sind Verbindungen der Formel I, worin bedeuten:
- R1 und R2: unabhängig voneinander H, F, CI, Br, CH₃, CF₃, SO₂CH₃, SO₂NH₂, wobei jedoch höchstens einer der Substituenten R1 und R2 Wasserstoff ist;
- R3: Wasserstoff, F oder Cl;
- R4: Wasserstoff, Alkyl mit 1, 2, 3 oder 4 C-Atomen, oder Cyclopropyl;
- R5 und R6: Wasserstoff oder gemeinsam eine Bindung;
- R7 und R8: gemeinsam eine Alkylenkette bestehend aus 3, 4, 5, 6, 7 oder 8 C-Atomen;

oder
- R7 und R8: gemeinsam einen Rest 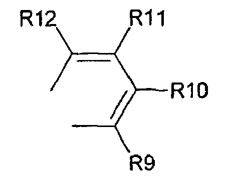
wobei R5 und R6 gemeinsam eine Bindung bilden;
R10 und R11 unabhängig voneinander Wasserstoff, OH, Fluor oder Chlor;
R9 und R12 Wasserstoff;
oder
einer der Substituenten R9 und R12 Wasserstoff;
und der andere F, CI, Br, CN, COOH, CO-NR13R14, SO₂-NR13R14 oder -(X)ₙ - CqH₂q-Z;
R13 und R14 gleich oder verschieden Wasserstoff oder Methyl;
n Null oder 1;
X Sauerstoff, NH, N-CH₃ oder S(O)ₖ;
k Null, 1 oder 2;
q Null, 1, 2, 3 oder 4;
Z Wasserstoff oder CF₃;

sowie deren pharmazeutisch verträgliche Salze, sowie deren Trifluoressigsäuresalze.

Besonders bevorzugt sind Verbindungen der Formel I, worin bedeuten:
- R1 und R2: unabhängig voneinander F, Cl, Br, CH₃ oder CF₃;
- R3: Wasserstoff;
- R4: Wasserstoff, Methyl, Ethyl;
- R5 und R6: Wasserstoff oder gemeinsam eine Bindung;
- R7 und R8: gemeinsam eine Alkylenkette bestehend aus 3, 4, 5, 6, 7 oder 8 C-Atomen;

oder
- R7 und R8: gemeinsam einen Rest 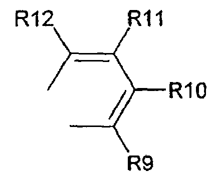
- wobei R5 und R6: gemeinsam eine Bindung bilden;
- R10 und R11: unabhängig voneinander Wasserstoff, OH oder Fluor;
- R9 und R12: Wasserstoff;
oder
- einer der Substituenten R9 und R12: Wasserstoff; und der andere F, Cl, Br oder -(X)ₙ - C_{q}H_{2q}-Z;
n Null oder 1;
X Sauerstoff, NH, N-CH₃ oder S(O)ₖ;
k Null, 1 oder 2;
q Null oder 1,
Z Wasserstoff oder CF₃;

sowie deren pharmazeutisch verträgliche Salze, sowie deren Trifluoressigsäuresalze.

Ganz besonders bevorzugt sind folgende Verbindungen der Formel I, ausgewählt aus der Gruppe:
trans-R,R-2-Chlor-3N-(3a,4,5,6,7,7a-hexahydro-1H-2-benzimidazolyl)-4-methyl-3-thienylamin,
trans-R,R-2-Brom-3N-(3a,4,5,6,7,7a-hexahydro-1H-2-benzimidazolyl)-4-methyl-3-thienylamin,
2-Chlor-3N-(2-benzimidazolyl)-4-methyl-3-thienylamin,
2-Brom-3N-(2-benzimidazolyl)-4-methyl-3-thienylamin,
2-Chlor-3N-(4-methyl-2-benzimidazolyl)-4-methyl-3-thienylamin,
2-Chlor-3N-(5-fluor-2-benzimidazolyl)-4-methyl-3-thienylamin,
2-Chlor-3N-(4-chlor-2-benzimidazolyl-amino)-4-methylthiophen,
2-Brom-3N-(4-chlor-2-benzimidazolyl-amino)-4-methylthiophen,
2-Brom-3N-(4-fluor-2-benzimidazolyl-amino)-4-methylthiophen,
2-Chlor-3N-(4-fluor-2-benzimidazolyl-amino)-4-methylthiophen,
2-Chlor-3N-(4-hydroxy-2-benzimidazolyl-amino)-4-methylthiophen,
(1 H-Benzimidazol-2-yl)-(2-chlor-4-methyl-thiophen-3-yi)-methyl-amin,
(2-Brom-4-methyl-thiophen-3-yl)-(5-fluor-1H-benzimidazol-2-yl)-amin,
2-Chlor-3N-(2-benzimidazolylamino)-4-methylthiophen
2,4-Dichlor-3N-(2-benzimidazolylamino)thiophen,
2-Brom-4-chlor-3N-(2-benzimidazolylamino)thiophen,
2,4-Dichlor-3N-(4-methyl-2-benzimidazolyl-amino)thiophen, trans-R,R-2,4-Dichfor-3N-(3a,4,5,6,7,7a-hexahydro-1H-2-benzimidazolyl)-3-thienylamin,
   oder
2,4-Dichlor-3N-(4-chlor-2-benzimidazolyl-amino)thiophen,

sowie deren pharmazeutisch verträgliche Salze, zum Beispiel das Hydrochlorid oder das Hydrobromid oder das Methansulfonat jeder der Verbindungen.

Die Verbindungen der Formel I können in Form ihrer Salze vorliegen. Als Säureadditionssalze kommen dabei Salze aller pharmakologisch verträglichen Säuren in Frage, beispielsweise Halogenide, insbesondere Hydrochloride, Hydrobromide, Lactate, Sulfate, Citrate, Tartrate, Acetate, Phosphate, Methylsulfonate, Benzolsulfonate, p-Toluolsulfonate, Adipinate, Fumarate, Gluconate, Glutamate, Glycerolphosphate, Maleinate, Benzoate, Oxalate und Pamoate. Diese Gruppe entspricht auch den physiologisch akzeptablen Anionen; aber auch Trifuoracetate. Enthalten die Verbindungen eine Säuregruppe können sie Salze mit Basen bilden, beispielsweise als Alkalimetallsalze, vorzugsweise Natrium- oder Kaliumsalze, oder als Ammoniumsalze, zum Beispiel als Salze mit Ammoniak oder organischen Aminen oder Aminosäuren. Sie können auch als Zitterion vorliegen.

Enthalten die Verbindungen der Formel I ein oder mehrere Asymmetriezentren, so können diese unabhängig voneinander sowohl S als auch R konfiguriert sein. Die Verbindungen können als optische lsomere, als Diastereomere, als Racemate oder als Gemische derselben vorliegen.

Die Verbindungen der Formel 1 können weiterhin als Tautomere oder als Gemisch tautomerer Strukturen vorliegen. Dabei ist zum Beispiel an folgende Tautomere gedacht:

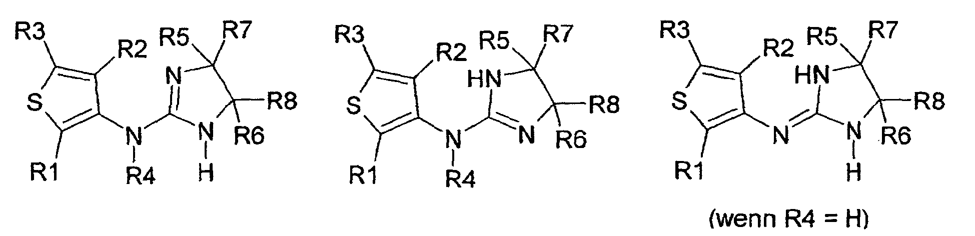

Alkylreste können geradkettig oder verzweigt sein. Dies gilt auch, wenn sie Substituenten tragen oder als Substituenten anderer Reste auftreten, beispielsweise in Fluoralkylresten oder Alkoxyresten. Beispiele für Alkylreste sind Methyl, Ethyl, n-Propyl, Isopropyl (= 1-Methylethyl), n-Butyl, Isobutyl (= 2-Methylpropyl), sec-Butyl (= 1-Methylpropyl), tert-Butyl (= 1,1-Dimethylethyl), n-Pentyl, Isopentyl, tert-Pentyl, Neopentyl oder Hexyl. Bevorzugte Alkylreste sind Methyl, Ethyl, n-Propyl, Isopropyl, n-Butyl, tert-Butyl, n-Pentyl, n-Hexyl. In Alkylresten können ein oder mehrere, zum Beispiel 1, 2, 3, 4, 5, 6, 7, 8 oder 9, Wasserstoffatome durch Fluoratome substituiert sein. Beispiele für solche Fluoralkylreste sind Trifluormethyl, 2,2,2-Trifluorethyl, Pentafluorethyl, Heptafluorisopropyl. Substituierte Alkylreste können in beliebigen Positionen substituiert sein.

Alkenylreste können geradkettig oder verzweigt sein. Dies gilt auch, wenn sie als Substituenten vorkommen, beispielsweise in Alkenylalkylen. Die Alkenylreste können in unterschiedlichen Positionen ungesättigt sein. Beispiele für Alkenylreste sind Ethenyl, Propenyl oder Butenyl.

Alkinylreste können geradkettig oder verzweigt sein. Dies gilt auch, wenn sie als Substituenten vorkommen, beispielsweise in Alkinylalkylen. Die Alkinylreste können in unterschiedlichen Positionen ungesättigt sein. Beispiele für Alkinylreste sind Ethinyl, Propinyl oder Butinyl.

Beispiele für Cycloalkylreste sind Cyclopropyl, Cyclobutyl, Cyclopentyl oder Cyclohexyl. Substituierte Cycloalkylreste können in beliebigen Positionen substituiert sein. Die Cycloalkylreste können auch verzeigt als Alkyl-cycloalkyl oder Cycloalkylalkyl vorliegen.

Beschrieben werden auch Methoden zur Herstellung erfindungsgemäßer Verbindungen. So lassen sich die durch Formel I beschriebenen Substanzen in dem Fachmann bekannter Weise aus den zugrundeliegenden Isothiocyanaten II und den entsprechenden Diaminen III herstellen.

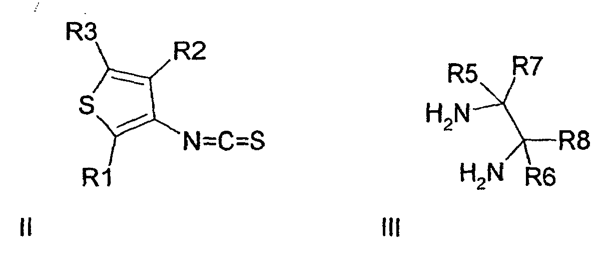

Das hierbei intermediär gebildete Thioharnstoff-Derivat wird mittels Methyliodid (Synthesis, 1974, 41 - 42) oder Carbodiimid (Synthesis, 1977, 864 - 865) oder mit p-Toluolsulfonsäurechlorid zur entsprechenden Verbindung der Formel I cyclisiert. Die hierbei verwendeten Isothiocyanate II können, falls nicht käuflich erhältlich, in literaturbekannter Weise aus den entsprechenden Aminothiophen-Derivaten durch dem Fachmann bekannte Methoden, z. B. durch Behandeln mit Thiophosgen (J. Med. Chem., 1975, 18, 90-99) oder Thiocarbonyldiimidazol (Justus Liebigs Ann. Chem., 1962, 657, 104) hergestellt werden.

Neben den oben beschriebenen Isothiocyanaten II lassen sich auch die Isocyanate IV 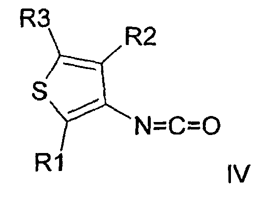 erfolgreich mit Aminen vom Typ der Formel III zu Verbindungen der Formel I umsetzen. Dabei wird das intermediär gebildete Hamstoffderivat mit Phosphoroxychlorid zu den entsprechenden Verbindungen der Formel I cyclisiert.

Bei der vorliegenden Erfindung konnte überraschend gezeigt werden, dass die beschriebenen Verbindungen potente Inhibitoren des Natrium-/ Protonen-Austauschers (NHE), insbesondere des Natrium-/ Protonen-Austauschers vom Subtyp 3 (NHE3), darstellen.

Die bisher bekannten NHE3-Inhibitoren leiten sich von Verbindungen des Acylguanidin-Typs (EP825178), Norbomylamin-Typs (DE199 60 204), 2-Guanidinochinazolin-Typs (WO 0179186) oder Benzamidin-Typs (WO0121582, WO0172742) ab. Das ebenfalls als NHE3-Inhibitor beschriebene Squalamin (M. Donowitz et al. Am. J. Physiol. 276 (Cell Physiol. 45): C136 - C144) wirkt nach derzeitigem Wissensstand nicht unmittelbar wie die Verbindungen nach Formel I, sondern über einen indirekten Mechanismus und erreicht so seine maximale Wirkstärke erst nach einer Stunde.

Solche mechanistisch andersartig wirkende NHE3-Inhibitoren eignen sich deshalb als Kombinationspartner der vorliegenden erfindungsgemäßen Verbindungen.

Das den hier beschriebenen Verbindungen ähnliche Clonidin ist als schwacher NHElnhibitor bekannt. Allerdings ist seine Wirkung auf den NHE3 der Ratte mit einer halbmaximalen Hemmkonzentration (IC₅₀) von 620 µM äußerst moderat. Stattdessen weist es eine gewisse Selektivität für den NHE2 auf (J. Orlowski et al J. Biol. Chem. 268, 25536). Es ist daher eher als NHE2-Inhibitor zu bezeichnen. Neben der schwachen NHE-Wirkung besitzt das Clonidin eine hohe Affinität zum adrenergen alpha2-Rezeptor und Imidazolin I1-Rezeptor, wodurch eine starke blutdrucksenkende Wirkung verursacht wird (Ernsberger et al Eur. J. Pharmacol. 134, 1, 1987).

Dem Clonidin ähnliche Verbindungen mit einem Thiophen- statt dem Phenylring sind aus der DE1941761 bekannt. Diese bekannten Verbindungen unterscheiden sich von den hier beschriebenen Strukturen der Formel I durch erheblich kleinere Reste R7 und R8 und insbesondere dadurch, dass R7 und R8 keinen gemeinsamen Ring bilden können.
Durch diese Unterschiede in den Substituenten R7 und R8 können die oben beschriebenen Clonidin-artigen, unerwünschten durch alpha-Adrenoceptorwirkung vermittelten Herz-Kreislaufeffekte dieser Thiophenderivate eliminiert werden. Gleichzeitig werden infolge dieser Substitutionsunterschiede die NHE-inhibierenden Eigenschaften der hier beschriebenen Verbindungen bis in den mikromolaren und submikromolaren Bereich verstärkt, während die aus DE1941761 bekannten Verbindungen keine oder nur sehr schwach ausgeprägte NHE-inhibierende Effekte zeigen. So besitzt der Hauptvertreter aus DE1941761, der als Entwicklungspräparat ausgewählte Blutdrucksenker Thiamenidin, bei jeweils 300 µM keine inhibierenden Effekte auf die untersuchten NHE-Subtypen NHE1, NHE2, NHE3 und NHE5.

Verbindungen der Formel I zeichnen sich durch Inhibition des zellulären Natrium-Protonen-Austauschers und insbesondere durch NHE3-inhibitorische Wirkung aus.

Der NHE3 wird im Körper verschiedener Spezies bevorzugt in der Galle, dem Darm und in der Niere gefunden (Larry Fliegel et al, Biochem. Cell. Biol. 76: 735 - 741, 1998), ließ sich aber auch im Gehirn nachweisen (E. Ma et al. Neuroscience 79: 591 - 603).

Aufgrund der NHE-inhibitorischen Eigenschaften eignen sich die Verbindungen der Formel I zur Prävention und Behandlung von Krankheiten, die durch eine Aktivierung des NHE bzw. durch einen aktivierten NHE verursacht werden, sowie von Krankheiten, die sekundär aus NHE-bedingten Schädigungen resultieren.

Da NHE Inhibitoren in überwiegender Weise über die Beeinflussung der zellulären pH-Regulation wirken, können diese in günstiger Weise mit anderen, den intrazellulären pH-Wert ebenfalls regulierenden Verbindungen kombiniert werden, wobei Inhibitoren der Enzymgruppe der Carboanhydratasen, Inhibitoren der Bicarbonationen transportierenden Systeme wie des Natrium-Bicarbonat-Cotransporters (NBC) oder des Natrium abhängigen Chlorid-Bicarbonat-Austauschers (NCBE), sowie NHE-Inhibitoren mit inhibitorischer Wirkung auf andere NHE-Subtypen, als Kombinationspartner infrage kommen, weil durch sie die pharmakologisch relevanten pH-regulierenden Effekte der hier beschriebenen NHE-inhibitoren verstärkt oder moduliert werden können.

Die Verwendung der erfindungsgemäßen Verbindungen betrifft die Prävention und die Behandlung akuter und chronischer Krankheiten in der Veterinär- und in der Humanmedizin.

Die pharmakologische Wirkung der Verbindungen der Formel I ist dadurch gekennzeichnet, dass sie zu einer Verbesserung des Atemantriebes führen. Sie können_deshalb zur Behandlung von gestörten Atmungszuständen herangezogen werden, wie sie beispielsweise bei folgenden klinischen Zuständen und Krankheiten auftreten können: Gestörter zentraler Atemantrieb (z. B. zentrale Schlafapnoen, plötzlicher Kindstod, postoperative Hypoxie), muskulär-bedingte Atemstörungen, Atemstörungen nach Langzeitbeatmung, Atemstörungen bei Adaptation im Hochgebirge, obstruktive und gemischte Form der Schlafapnoen, akute und chronische Lungenkrankheiten mit Hypoxie und Hyperkapnie.
Zusätzlich erhöhen die Verbindungen den Muskeltonus der oberen Atemwege, so dass das Schnarchen unterdrückt wird. Die genannten Verbindungen finden deshalb vorteilhaft Verwendung zur Herstellung eines Medikaments zur Prävention und Behandlung von Schlafapnoen und muskulär bedingter Atemstörungen, zur Herstellung eines Medikaments zur Prävention und Behandlung des Schnarchens.

Eine Kombination eines NHE-Inhibitors der Formel I mit einem Carboanhydrase-Hemmer (z. B. Acetazolamid) kann sich als vorteilhaft erweisen, wobei letzterer eine metabolische Azidose herbeiführt und dadurch bereits die Atmungstätigkeit steigert, so daß eine verstärkte Wirkung und verminderter Wirkstoffeinsatz erzielt werden können.

Die erfindungsgemäßen Verbindungen schonen infolge ihrer NHE3-inhibitorischen Wirkung die zellulären Energiereserven, die sich unter toxischen und pathogenen Ereignissen rasch erschöpfen und so zur Zellschädigung oder zum Zelluntergang führen. Dabei wird die energieaufwendige ATP-verbrauchende Natriumresorption im proximalen Tubulus unter dem Einfluß der Verbindungen der Formel I vorübergehend still gelegt und die Zelle kann so eine akute pathogene, ischämische oder toxische Situation überleben. Die Verbindungen eignen sich deshalb beispielsweise als Arzneimittel zur Behandlung ischämischer Noxen, zum Beispiel des akuten Nierenversagens.
Weiterhin eignen sich die Verbindungen auch zur Behandlung aller chronischen Nierenerkrankungen und Nephritisformen, die infolge vermehrter Proteinausscheidung zum chronischen Nierenversagen führen. Dementsprechend eignen sich die Verbindungen der Formel I zur Herstellung eines Medikaments zur Behandlung diabetischer Spätschäden, der diabetischen Nephropathie und chronischer Nierenerkrankungen, insbesondere von allen Nierenentzündungen (Nephritiden), die mit einer vermehrten Protein-/ Albuminausscheidung verbunden sind;

Es hat sich gezeigt, dass die erfindungsgemäß verwendeten Verbindungen eine milde abführende Wirkung besitzen und demzufolge auch vorteilhaft als Abführmittel oder bei drohender Darmverstopfung verwendet werden können.

Weiterhin können die erfindungsgemäßen Verbindungen vorteilhaft zur Prävention und Therapie von akuten und chronischen Erkrankungen des Darmtrakts verwendet werden, die beispielweise durch ischämische Zustände im Intestinalbereich und / oder durch nachfolgende Reperfusion oder durch entzündliche Zustände und Ereignisse ausgelöst werden. Solche Komplikationen können beispielweise durch mangelnde Darmperistaltik, wie sie z.B. häufig nach chirurgischen Eingriffen, bei Darmverstopfung oder stark verminderter Darmtätigkeit zu beobachten sind.

Mit den erfindungsgemäßen Verbindungen besteht die Möglichkeit, der Gallenstein-Bildung vorzubeugen.

Die erfindungsgemäßen NHE-Inhibitoren eignen sich generell zur Behandlung von Krankheiten, die durch Ischämie und durch Reperfusion hervorgerufen werden.

Die erfindungsgemäßen Verbindungen sind infolge ihrer pharmakologischen Eigenschaften als antiarrhythmische Arzneimittel geeignet.
Durch ihre cardioprotektive Komponente eignen sich die NHE-Inhibitoren der Formel I hervorragend zur lnfarktprophylaxe und der Infarktbehandlung sowie zur Behandlung der angina pectoris, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäß verwendeten Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden.

Dies betrifft ihre Verwendung als Arzneimittel für chirurgische Eingriffe. So können die erfindungsgemäßen Verbindungen bei Organ-Transplantationen verwendet werden, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den mit Verbindungen der Formel I vorbehandelten Empfängerorganismus verwendet werden können.

Die Verbindungen sind ebenfalls wertvolle, protektiv wirkende Arzneimittel bei der Durchführung angioplastischer operativer Eingriffe beispielsweise am Herzen wie auch an peripheren Organen und Gefäßen.

Da NHE-Inhibitoren menschliches Gewebe und Organe nicht nur effektiv gegen Schäden schützen, die durch Ischämie und Reperfusion verursacht werden, sondern auch gegen die cytotoxische Wirkung von Arzneimitteln, wie sie insbesondere in der Krebstherapie und der Therapie von Autoimmunerkrankungen, Anwendung finden, ist die kombinierte Verabreichung mit Verbindungen der Formel I geeignet, die cytotoxischen Effekte einer Therapie zu vermindern oder zu unterdrücken. Durch die Verminderung der cytotoxischen Effekte, insbesondere der Cardiotoxizität, infolge Ko-Medikation mit NHE-Inhibitoren kann außerdem die Dosis der cytotoxischen Therapeutika erhöht und / oder die Medikation mit solchen Arzneimitteln verlängert werden. Der therapeutische Nutzen einer solchen cytotoxischen Therapie kann durch die Kombination mit NHE-Inhibitoren erheblich gesteigert werden.
Die Verbindungen der Formel I eignen sich insbesondere zur Verbesserung der Therapie mit Arzneimitteln, die eine unerwünschte cardiotoxische Komponente besitzen.

Entsprechend ihrer protektiven Wirkung gegen ischämisch induzierte Schäden sind die erfindungsgemäßen Verbindungen auch als Arzneimittel zur Behandlung von Ischämien des Nervensystems, insbesondere des Zentralnervensystems, geeignet, wobei sie z. B. zur Behandlung des Schlaganfalls oder des Hirnödems geeignet sind.

Die Verbindungen der Formel I eignen sich auch zur Therapie und Prophylaxe von Erkrankungen und Störungen, die durch Übererregbarkeit des Zentralnervensystems ausgelöst werden, insbesondere zur Behandlung von Erkrankungen des epileptischen Formenkreises, zentral ausgelöster klonischer und tonischer Spasmen, psychische Depressionszustände, Angsterkrankungen und Psychosen. Dabei können die erfindungsgemäßen NHE-Inhibitoren allein angewandt werden oder in Kombination mit anderen antiepileptisch wirkenden Substanzen oder antipsychotischen Wirkstoffen, oder Carboanhydratasehemmern, beispielweise mit Acetazolamid, sowie mit weiteren Inhibitoren des NHE oder des natrium-abhängigen Chlorid-Bicarbonat-Austauschers (NCBE).

Darüber hinaus eignen sich die erfindungsgemäßen Verbindungen der Formel I ebenfalls zur Behandlung von Formen des Schocks, wie beispielweise des allergischen, cardiogenen, hypovolämischen und des bakteriellen Schocks.

Die Verbindungen der Formel I können ebenfalls zur Prävention und zur Behandlung thrombotischer Erkrankungen verwendet werden, da sie als NHE-Inhibitoren sowohl die Plättchenaggregation selbst inhibieren können. Darüberhinaus können sie die nach Ischämie und Reperfusion stattfindende überschießende Freisetzung von Entzündungs- und Gerinnungsmediatoren, insbesondere des *von Willebrand Faktors* und thrombogener Selectin-Proteine hemmen bzw. verhindern. Damit kann die pathogene Wirkung thrombogener und entzündungsrelevanter Faktoren vermindert und ausgeschaltet werden. Deshalb sind die NHE-Inhibitoren der vorliegenden Erfindung kombinierbar mit weiteren antikoagulativen und/oder thrombolytischen Wirkstoffen wie beispielsweise recombinantem oder natürlichen tissue plasminogen activator, Streptokinase, Urokinase, Acetylsalizylsäure, Thrombinantagonisten, Faktor Xa-Antagonisten, fibrinolytisch wirkenden Arzneistoffen, Thromboxan-RezeptorAntagonisten, Phosphodiesterase-Inhibitoren, Factor-VIIa-Antagonisten, Clopidogrel, Ticlopidin usw. Eine kombinierte Anwendung der vorliegenden NHE-Inhibitoren mit NCBE-Inhibitoren und/oder mit Inhibitoren der Carboanhydratase, wie beispielsweise mit Acetazolamide, ist besonders günstig.

Darüber hinaus zeichnen sich die erfindungsgemäßen Verbindungen der Formel I durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten in Frage, bei denen die Zellproliferation eine primäre oder sekundäre Ursache darstellt, und können deshalb als Antiatherosklerotika, Mittel gegen das chronische Nierenversagen, Krebserkrankungen, verwendet werden. Sie können somit zur Behandlung von Organhypertrophien und -hyperplasien, beispielsweise des Herzens und der Prostata verwendet werden. Verbindungen der Formel I sind deshalb zur Prävention und zur Behandlung der Herzinsuffizienz (congestive heart failure = CHF) wie auch bei der Behandlung und Prävention der Prostatahyperplasie bzw. Prostatahypertrophie geeignet.

Die Verbindungen der Formel I zeichnen sich weiterhin durch eine Verzögerung oder Verhinderung von fibrotischen Erkrankungen aus. Sie eignen sich somit als ausgezeichnete Mittel zur Behandlung von Fibrosen des Herzens, sowie der Lungenfibrose, Leberfibrose, der Nierenfibrose und anderer fibrotischer Erkrankungen.

Da der NHE bei essentiellen Hypertonikern signifikant erhöht ist, eignen sich die Verbindungen der Formel zur Prävention und zur Behandlung des Bluthochdrucks und von Herz-Kreislauferkrankungen.
Dabei können sie allein oder mit einem geeigneten Kombinations- und Formulierungspartner zur Bluthochdruckbehandlung und von Herz-Kreislauferkrankungen zur Anwendung kommen. So können beispielsweise ein oder mehrere thiazid-artig wirkende Diuretika, Loop-Diuretika, Aldosteron- und Pseudoaldosteron-Antagonisten, wie Hydrochlorothiazid, Indapamid, Polythiazid, Furosemid, Piretanid, Torasemid, Bumetanid, Amiloride, Triamteren, Spironolacton oder Epleron, mit Verbindungen der Formel I kombiniert werden. Weiterhin können die NHE-Inhibitoren der vorliegenden Erfindung in Kombination mit Calcium-Antagonisten, wie Verapamil, Diltiazem, Amlodipin, oder Nifedipin, sowie mit ACE-Hemmern, wie beispielsweise Ramipril, Enalapril, Lisinopril, Fosinopril oder Captopril verwendet werden. Weitere günstige Kombinationspartner sind auch β-Blocker wie Metoprolol, Albuterol usw., Antagonisten des Angiotensin-Rezeptors und seiner Rezeptor-Subtypen wie Losartan, Irbesartan, Valsartan, Omapatrilat, Gemopatrilat, Endothelin-Antagonisten, Renin-Inhibitoren, Adenosin- Rezeptoragonisten, Inhibitoren und Aktivatoren von Kaliumkanälen wie des Glibenclamide, Glimepirid, Diazoxide, Cromokalim, Minoxidil und deren Derivate, Aktivatoren des mitochondrialen ATPsensitiven Kaliumkanals (mitoK(ATP) Kanal), Inhibitoren weitere Kaliumkanäle, wie des Kv1.5 usw.

Infolge ihrer antiphlogistischen Wirkung haben können NHE-Inhibitoren als Antiinflammatorika verwendet werden. Mechanistisch fällt dabei die Inhibition der Freisetzung von Entzündungsmediatoren auf. Die Verbindungen können somit allein oder in Kombination mit einem Antiphlogistikum bei der Prävention oder Behandlung chronischer und akuter inflammatorischer Erkrankungen verwendet werden. Als Kombinationspartner werden vorteilhaft steroidale und nicht-steroidale Antiinflammatorika verwendet.

Es wurde außerdem gefunden, dass Verbindungen der Formel eine günstige Beeinflussung der Serumlipoproteine zeigen. Sie können daher zur Prophylaxe und zur Regression von atherosklerotischen Veränderungen herangezogen werden, indem sie einen kausalen Risikofaktor ausschalten. Hierzu zählen nicht nur die primären Hyperlipidämien, sondern auch gewisse sekundäre Hyperlipidämien, wie sie z.B. beim Diabetes vorkommen. Darüber hinaus führen die Verbindungen der Formel I zu einer deutlichen Reduktion der durch Stoffwechsetanomalien induzierten Infarkte und insbesondere zu einer signifikanten Verminderung der induzierten Infarktgröße und dessen Schweregrades.
Die genannten Verbindungen finden deshalb vorteilhaft Verwendung zur Herstellung eines Medikaments zur Behandlung von Hypercholesterinämie; zur Herstellung eines Medikaments zur Prävention der Atherogenese; zur Herstellung eines Medikaments zur Prävention und Behandlung der Atherosklerose, zur Herstellung eines Medikaments zur Prävention und Behandlung von Krankheiten, die durch erhöhte Cholesterinspiegel ausgelöst werden, zur Herstellung eines Medikaments zur Prävention und Behandlung von Krankheiten; die durch endotheliale Dysfunktion ausgelöst werden, zur Herstellung eines Medikaments zur Prävention und Behandlung von atherosklerose-induzierter Hypertonie, zur Herstellung eines Medikaments zur Prävention und Behandlung von atherosklerose-induzierter Thrombosen, zur Herstellung eines Medikaments zur Prävention und Behandlung von Hypercholesterinämie- und endothelialer Dysfunktion induzierter ischämischer Schäden und postischämischer Reperfusionsschäden, zur Herstellung eines Medikaments zur Prävention und zur Behandlung kardialer Hypertrophien und Cardiomyopathien und der congestiven Herzinsuffizienz (CHF), zur Herstellung eines Medikaments zur Prävention und Behandlung von Hypercholesterinämie- und endothelialer Dysfunktion induzierter koronarer Gefäßspasmen und myocardialer Infarkte, zur Herstellung eines Medikaments zur Behandlung der genannten Leiden in Kombinationen mit blutdrucksenkenden Stoffen, bevorzugt mit Angiotensin Converting Enzyme (ACE)-Hemmem und Angiotensin-Rezeptorantagonisten. Eine Kombination eines NHE-Inhibitors der Formel I mit einem blutfettspiegelsenkenden Wirkstoff, bevorzugt mit einem HMG-CoA-Reduktaseinhibitor (z.B. Lovastatin oder Pravastatin), wobei letzterer eine hypolipidämische Wirkung herbeiführt und dadurch die hypolipidämischen Eigenschaften des NHE-Inhibitors der Formel I steigert, erweist sich als günstige Kombination mit verstärkter Wirkung und vermindertem Wirkstoffeinsatz.

So führen Verbindungen der Formel I zu einen wirksamen Schutz gegen Endothelschädigungen unterschiedlicher Genese. Mit diesem Schutz der Gefäße gegen das Syndrom der endothelialen Dysfunktion sind Verbindungen der Formel I wertvolle Arzneimittel zur Prävention und zur Behandlung koronarer Gefäßspasmen, peripherer Gefäßkrankheiten, insbesondere von claudicatio intermittens, der Atherogenese und der Atherosklerose, der linksventrikulären Hypertrophie und der dilatierten Kardiomyopathie, und thrombotischer Erkrankungen.

Außerdem eignen sich NHE-Inhibitoren der Formel I zur Behandlung des nichtinsulinabhängigen Diabetes (NIDDM) sind, wobei beispielweise die Insulinresistenz zurückgedrängt wird. Dabei kann es zur Verstärkung von antidiabetischer Wirksamkeit und Wirkquaütät der erfindungsgemäßen Verbindungen günstig sein, diese mit einem Biguanid wie Metformin, mit einem antidiabetischen Sulfonylharnstoff, wie Glyburide, Glimepiride, Tolbutamid usw., einem Glucosidase Inhibitor, einem PPAR Agonisten, wie Rosiglitazone, Pioglitazone etc, mit einem Insulinpräparat unterschiedlicher Verabreichungsform, mit einem DB4 Inhibitor, mit einem Insulinsensitizer oder mit Meglitinide zu kombinieren.

Neben den akuten antidiabetischen Effekten wirken die Verbindungen der Formel I der Entstehung diabetischer Spätkomplikation entgegen und können deshalb als Arzneimittel zur Prävention und Behandlung diabetischer Spätschäden, wie der diabetischen Nephropathie, der diabetischen Neuropathie, der diabetischen Retinopathie, der diabetischen Cardiomyopathie und anderen, als Folge des Diabetes auftretenden Erkrankungen verwendet werden. Dabei können sie mit den oben unter NIDDM-Behandlung beschriebenen antidiabetischen Arzneimitteln vorteilhaft kombiniert werden. Der Kombination mit einer günstigen Darreichungsform von Insulin kann dabei eine besondere Bedeutung zukommen.

Die erfindungsgemäßen NHE-Inhibitoren der Formel I zeigen neben den protektiven Effekten gegen akute ischämische Ereignisse und die nachfolgenden ebenso akut belastenden Reperfusionsereignisse, auch noch direkte therapeutisch verwertbare Wirkungen gegen Erkrankungen und Störungen des gesamten Säugetierorganismus, die mit den Erscheinungen des chronisch ablaufenden Alterungsprozesses zusammenhängen und die auch unabhängig von akuten Mangeidurchblutungszuständen sind und auch bei normalen, nicht-ischämischen Bedingungen auftreten können. Bei diesen pathologischen, über die lange Zeit des Alterns ausgelösten altersbedingten Erscheinungen wie Krankheit, Siechtum und Tod, die neuerdings mit NHE-Inhibitoren einer Behandlung zugänglich gemacht werden können, handelt es sich um Erkrankungen und Störungen, die maßgeblich durch altersbedingte Veränderungen von lebensnotwendigen Organen und deren Funktion verursacht werden und im alternden Organismus zunehmend an Bedeutung gewinnen. Erkrankungen, die mit einer altersbedingten Funktionsstörung, mit altersbedingten Verschleißerscheinungen von Organen zusammenhängen, sind beispielsweise die ungenügende Ansprechbarkeit und Reagibilität der Blutgefäße gegenüber Kontraktions und Relaxationsreaktionen. Diese altersbedingte Abnahme der Gefäßreagibilität auf konstriktorische und relaxierende Reize, die ein essentieller Prozess des Herz-Kreislaufsystems und somit des Lebens und der Gesundheit sind, kann signifikant durch NHE-Inhibitoren aufgehoben bzw. verringert werden. Eine wichtige Funktion und ein Maß für die Aufrechterhaltung der Gefäßreagibilität ist die Blockade bzw. Retardierung der altersbedingt fortschreitenden endothelialen Dysfunktion, die hochsignifikant durch NHE-Inhibitoren aufgehoben werden kann. Die Verbindungen der Formel I eignen sich somit hervorragend zur Behandlung und Prävention der altersbedingt fortschreitenden endothelialen Dysfunktion, insbesondere von claudicatio intermittens. Außerdem eignen sich die Verbindungen der Formel I somit hervorragend zur Behandlung und Prävention der Herzinsuffizienz, des congestive heart failure (CHF) sowie zur Behandlung und insbesondere zur Prävention von altersbedingten Formen von Krebs.
Außerdem kann an eine Kombination mit blutdrucksenkenden Medikamenten, wie mit ACE-Hemmern, Angiotensinrezeptorantagonisten, Diuretika, Ca⁺²-Antagonisten etc. oder mit stoffwechselnormalisierenden Medikamenten, wie Cholesterinsenkern, gedacht werden. Die Verbindungen der Formel I eignen sich somit zur Prävention altersbedingter Gewebsveränderungen und zur Lebensverlängerung unter Erhalt einer hohen Lebensqualität.

Die erfindungsgemäßen Verbindungen sind wirkungsvolle Inhibitoren des zellulären Natrium-Protonen-Antiporters (Na/H-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäß verwendeten Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes und diabetischer Spätkomplikationen, proliferativer Erkrankungen usw.

Weiterhin sind NHE3 Inhibitoren zur Behandlung von durch Bakterien, sowie durch Protozoen ausgelöste Erkrankungen (human und veterinär) geeignet. Bei den durch Protozoen ausgelösten Erkrankungen sind insbesondere Malariaerkrankungen des Menschen und Hühnercoccidiose zu nennen.
Außerdem eignen sich die Verbindungen als Mittel zur Bekämpfung von saugenden Parasiten in der Human- und Veterinärmedizin sowie im Pflanzenschutz. Dabei ist die Verwendung als Mittel gegen blutsaugende Parasiten in der Human- und Veterinärmedizin bevorzugt.

Die Verbindungen der Formel I zeichnen sich neben ihren potenten NHE-Hemmwerten, ihren pharmakologischen Eigenschaften und der Abwesenheit unerwünschter biologischer Wirkungen auch durch günstige pharmakokinetische Eigenschaften aus, die ihre Verwendung als Arzneimittel in besonderer Weise günstig erscheinen lassen.

Die Erfindung betrifft somit Heilmittel für die humane, veterinäre oder phytoprotektive Anwendung enthaltend eine wirksame Menge einer Verbindung der Formel I und/oder ihrer pharmazeutisch verträglichen Salze allein oder in Kombination mit anderen pharmakologischen Wirkstoffen oder Arzneimitteln.

Arzneimittel, die eine Verbindung I enthalten, können zum Beispiel oral, parenteral, intramuskulär, intravenös, rektal, nasal, durch Inhalation, subkutan oder durch eine geeignete transkutane Darreichungsform appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin und im Pflanzenschutz.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen, perkutanen oder intravenösen Applikation werden die verwendeten aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.
Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,1 mg/kg, bis höchstens 30 mg/kg, vorzugsweise 1 mg/kg Körpergewicht. In akuten Situationen, etwa unmittelbar nach Erleiden apnoetischer Zustände im Hochgebirge, können auch noch höhere Dosen notwendig werden. Insbesondere bei i.v. Anwendung, etwa bei einem Infarktpatienten auf der Intensivstation können bis zu 200 mg/kg pro Tag notwendig werden. Die Tagesdosis kann auf eine oder mehrere, zum Beispiel bis zu 4, Einzeldosen verteilt werden.

### Versuchsbeschreibungen und Beispiele:

Handelt es sich um enantiomerenreine Verbindungen, so ist die Konfiguration und/oder das Vorzeichen der optischen Drehung angegeben. Fehlen diese Angaben, so handelt es sich um Racemate oder optisch nicht aktive Verbindungen.

Die im Folgenden angegebenen Retentionszeiten (Rt) beziehen sich auf LCMS-Messungen mit den folgenden Parametern:

### Analytische Methoden:

| A | |
|---|---|
| stationäre Phase: | Merck Purospher 3µ 2 x 55 mm |
| mobile Phase: | 95% H₂O (0,1 % HCOOH) → 95% Acetonitril (0, 1 % HCOOH); 5 min → 95% Acetonitril (0,1% HCOOH); 2 min → 95% H₂O (0,1% HCOOH); 1 min; 0,45 ml/min. |

| B | |
|---|---|
| stationäre Phase: | YMC J'sphere H80 ~4µ 2,1 x 33 mm |
| mobile Phase: | 95% H₂O (0,1% HCOOH) → 95% Acetonitril (0,08% HCOOH); 2,5 min → 95% Acetonitril (0,08% HCOOH); 0,5 min → 95% H₂O (0,1% HCOOH); 0,5 min; 1,3 ml/min. |

| C | |
|---|---|
| stationäre Phase: | YMC J'sphere H80, 4µ, 2,1 x 20 mm |
| mobile Phase: | 90% H₂O (0,05% TFA) → 95% Acetonitril; 1,9 min; → 95% Acetonitril 0,5 min; 1 ml/min. |

| D | |
|---|---|
| stationäre Phase: | Merck Purospher 3µ 2 x 55 mm |
| mobile Phase: | 95% H₂O (0,1 % HCOOH)→ 95% Acetonitril (0,1 % HCOOH); 3,4 min → 95% Acetonitril (0,1% HCOOH); 1 min → 95% H₂O (0,1% HCOOH); 0,2 min; 0,75 ml/min. |

| E | |
|---|---|
| stationäre Phase: | Merck Purospher 3µ 2 x 55 mm |
| mobile Phase: | 95% H₂O (0,05% CF3COOH)→ 95% Acetonitril (0,05% CF3COOH); 3,4 min → 95% Acetonitril (0,05% CF3COOH); 1 min; 0,75 ml/min. |

| F | |
|---|---|
| stationäre Phase: | YMC J'sphere H80, 4µ, 2,1 x 20 mm |
| mobile Phase: | 96% H₂O (0,05% CF3COOH) → 95% Acetonitril; 2 min; → 95% Acetonitril 0,4 min; 1 ml/min. |

| Die präparative HPLC wurde unter folgenden Bedingungen durchgeführt: | |
|---|---|
| stationäre Phase: | Merck Purospher RP18 (10µM) 250 x 25 mm |
| mobile Phase: | 90% H₂O (0,05% TFA)→ 90% Acetonitril; 40 min; 25 ml/min |

### Beispiel 1: 3N-(5-Fluor-2-benzimidazolyl)-4-methyl-3-thienylamin Hydrochlorid

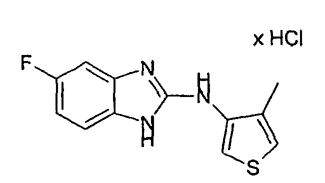

### a) 4-Methyl-3-thienyl-isothiocyanat

wird durch Umsetzung von äquimolaren Mengen von 3-Amino-4-methylthiophen und N,N'-Thiocarbonyldiimidazol in wasserfreiem Tetrahydrofuran (THF) durch Rühren des Reaktionsgemisches über 5 Stunden bei Raumtemperatur und weiterem Stehenlassens über Nacht bei Raumtemperatur erhalten. Die Isolierung von 4-Methyl-3-thienyl-isothiocyanat erfolgt durch Abdestillieren des Lösungsmittels unter vermindertem Druck am Rotavapor, Lösen des Rückstandes in Ethylacetat und mehrfachem Waschen der organischen Phase mit Wasser. Nach dem Trocknen der organischen Phase über Natriumsulfat wird das organische Lösungsmittel am Rotavapor unter vermindertem Druck abdestilliert und 4-Methyl-3-thienyl-isothiocyanat als brauner öliger Rückstand erhalten. Die Weiterverwendung von 4-Methyl-3-thienyl-isothiocyanat kann ohne weitere Reinigungsmaßnahmen erfolgen.

### b) N-(2-Amino-5-fluorphenyl)-N'-(4-methyl-3-thienyl)-thioharnstoff

Zu einer Lösung von 0,02 Mol 4-Methyl-3-thienyl-isothiocyanat in 60 ml wasserfreiem THF gibt man 0,02 Mol 4-Fluor-1 ,2-diaminobenzol. Nach Rühren des Reaktionsgemisches über 2 Stunden bei Raumtemperatur und Stehenlassen über Nacht wird das Lösungsmittel am Rotavapor unter vermindertem Druck abdestilliert und der ölige Rückstand an einer Kieselgelsäule mit einem Gemisch aus gleichen Anteilen von Toluol und Essigester gereinigt.
Braun gefärbter kristalliner Feststoff. Schmp. 180°C.

### c) 3N-(5-Fluor-2-benzimidazolyl)-4-methyl-3-thienylamin Hydrochlorid

1,5 g (0,0053 Mol) N-(2-Amino-5-fluorphenyl)-N'-(4-methyl-3-thienyl)-thioharnstoff werden in 50 ml wasserfreiem Ethanol mit der mehrfach molaren Menge (etwa 1,5 bis 4 Mol) Methyljodid versetzt und 5 Stunden am Rückflusskühler gekocht. Nach dem Stehenlassen über Nacht bei Raumtemperatur wird das Ethanol am Rotavapor unter vermindertem Druck abdestilliert, der Rückstand mit Wasser versetzt und mit gesättigter wässeriger Natriumhydrogencarbonat-Lösung auf pH 8 bis 9 gestellt. Man extrahiert mehrfach mit Ethylacetat, wäscht die organische Phase mit Wasser, trocknet über Natriumsulfat, destilliert das Lösungsmittel unter vermindertem Druck am Rotavapor ab und reinigt den Rückstand durch Säulenchromatografie an Kieselgel mit einem Lösungsmittelgemisch aus gleichen Volumenteilen Essigester und Toluol (nachfolgend als "Gemisch 2" bezeichnet) als Elutionsmittel. Das nach dem Abdestillieren des organischen Lösungsmittels erhaltene ölige Produkt wird in Ethylacetat gelöst und mit einer gesättigten Lösung von Chlorwasserstoff in trockenem Diethylether stark sauer gestellt und der kristallisierende Niederschlag nach längerem Stehenlassen abfiltriert. Kristalliner Feststoff, Schmp. 192 +/- 2°C.

### Beispiel 2: 2-Chlor-3N-(5-fluor-2-benzimidazolyl)-4-methyl-3-thienylamin Hydrochlorid und 2,5-Dichlor-3N-(5-fluor-2-benzimidazolyl)-4-methyl-3-thienylamin Hydrochlorid

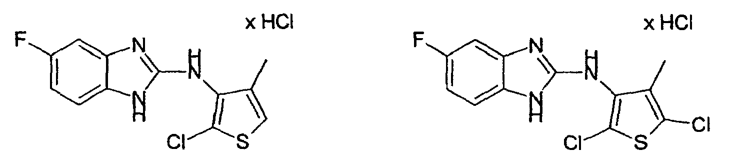

Zu einer Lösung aus 0,5g (0,0018 Mol) 3N-(5-Fluor-2-benzimidazolyl)-4-methyl-3-thienylamin Hydrochlorid in 25 ml Eisessig tropft man eine Lösung aus 0,24g (0,0018 Mol) N-Chlorsuccinimid in 15 ml Eisessig, rührt das Reaktionsgemisch etwa 2 bis 3 Stunden bei Raumtemperatur und destilliert das Lösungsmittel unter vermindertem Druck am Rotavapor ab. Nach Versetzen des Rückstandes mit Wasser wird mit 2N NaOH alkalisch gestellt, mit Ethylacetat extrahiert, die organische Phase mit Wasser gewaschen, über Natriumsulfat getrocknet und das Lösungsmittel am Rotavapor unter vermindertem Druck abdestilliert. Der so erhaltene ölige Rückstand wird auf der Säule durch Mitteldruck-Chromatographie und mit einem Lösungsmittelgemisch aus 20 Teilen Ethylacetat, 10 Teilen n-Heptan und 3 Teilen Eisessig (nachfolgend als "Gemisch 17" bezeichnet) als Elutionsmittel getrennt, und nach Behandlung mit einer Lösung von Chlorwasserstoffgas in Ether erhält man:
2-Chlor-3N-(5-fluor-2-benzimidazolyl)-4-methyl-3-thienylamin Hydrochlorid aus Fraktion 1 und 2: Farbloses bis leicht gelb gefärbtes kristallines Produkt, Schmp. 200 - 202 °C,
2,5-Dichlor-3N-(5-fluor-2-benzimidazolyl)-4-methyl-3-thienylamin Hydrochlorid aus Fraktion 3: Farbloses bis leicht gelb gefärbtes kristallines Produkt, Schmp. 286 - 288 °C.

### Beispiel 3: 3N-(5,6-dichlor-2-benzimidazolyl)-4-methyl-3-thienylamin Hydrochlorid

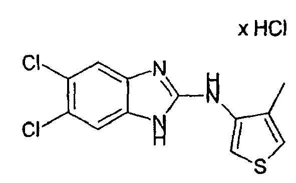

a) N-(2-Amino-4,5-dichlorphenyl)-N'-(4-methyl-3-thienyl)-thioharnstoff erhält man analog der in Beispiel 1, b) angegebenen Reaktion aus 4-Methyl-3-thienyl-isothiocyanat und 4,5-Dichlor-1,2-diaminobenzol. Kristalliner Feststoff, Schmp. 310-320°C.
b) 3N-(5,6-dichlor-2-benzimidazolyl)-4-methyl-3-thienylamin Hydrochlorid erhält man analog der in Beispiel 1, c) angegebenen Vorschrift aus N-(2-Amino-4,5-dichlorphenyl)-N'-(4-methyl-3-thienyl)-thioharnstoff und Methyljodid. Kristalliner Feststoff, Schmp. 290 - 294 °C

### Beispiel 4: 3N-(2-Benzimidazolyl)-4-methyl-3-thienylamin Hydrochlorid

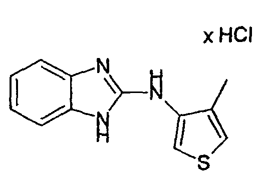

a) N-(2-Aminophenyl)-N'-(4-methyl-3-thienyl)-thioharnstoff erhält man analog der in Beispiel 1, b) angegebenen Reaktion aus 4-Methyl-3-thienyl-isothiocyanat und 1,2-Diaminobenzol. Kristalliner Feststoff mit 1. Schmp. 177-182°C, anschließend erneute Kristallisation und 2. Schmp.: 285-290 °C.
b) 3N-(2-Benzimidazolyl)-4-methyl-3-thienylamin Hydrochlorid erhält man analog der in Beispiel 1, c) angegebenen Vorschrift aus N-(2-Aminophenyl)-N'-(4-methyl-3-thienyl)-thioharnstoff und Methyljodid.
   Kristalliner Feststoff nach Umkristallisieren aus Essigester / Ethanol, Schmp. 194-200 °C

### Beispiel 5: 3N-(4-Fluor-2-benzimidazolyl)-4-methyl-3-thienylamin Hydrochlorid

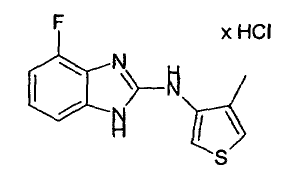

a) 3-Fluor-1,2-diaminobenzol erhält man als ölig amorphes Produkt durch Hydrierung von 3-Fluor-2-nitrophenylhydrazin (dargestellt durch Reaktion von 2,6-Difluomitrobenzol mit Hydrazin Hydrat) mit Wasserstoff und 10%igen Palladiumkatalysator auf Kohle in Methanol bei Raumtemperatur und Normaldruck.
b) N-(2-Amino-3-fluorphenyl)-N'-(4-methyl-3-thienyl)-thioharnstoff erhält man analog der in Beispiel 1, b) angegebenen Reaktion aus 4-Methyl-3-thienyl-isothiocyanat und 3-Fluor-1,2-diaminobenzol. Kristalliner Feststoff, Zersetzungspunkt > 240°C,
c) 3N-(4-Fluor-2-benzimidazolyl)-4-methyl-3-thienylamin Hydrochlorid erhält man analog der in Beispiel 1, c) angegebenen Vorschrift aus N-(2-Amino-3-fluorphenyl)-N'-(4-methyl-3-thienyl)-thioharnstoff und Methyljodid. Amorpher Niederschlag, der unter Aceton kristallisiert. Kristalliner Feststoff, Schmp. 220-230 °C

### Beispiel 6: 3N-(4,6-Difluor-2-benzimidazolyl)-4-methyl-3-thienylamin Hydrochlorid

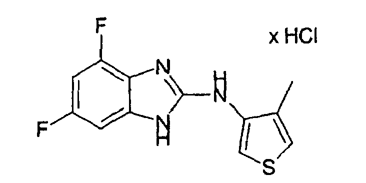

a) N-(2-Amino-3,5-difluorphenyl)-N`-(4-methyl-3-thienyl)-thioharnstoff erhält man analog der in Beispiel 1, b) angegebenen Reaktion aus 4-Methyl-3-thienyl-isothiocyanat und 3,5-Difluor-1,2-diaminobenzol. Kristalliner Feststoff, 1. Schmelzpunkt: 178-182°C, erneute Kristallisation mit 2. Schmp.: 299-301 °C
b) 3N-(4,6-Difluor-2-benzimidazolyl)-4-methyl-3-thienylamin Hydrochlorid erhält man analog der in Beispiel 1, c) angegebenen Vorschrift aus N-(2-Amino-3,5-difluorphenyl)-N'-(4-methyl-3-thienyl)-thioharnstoff und Methyljodid. Amorpher Niederschlag, der unter Ethylacetat kristallisiert. Kristalliner Feststoff, Schmp. 232-234 °C

### Beispiel 7: 3N-(4 ,5,6, 7- Tetrafluor-2-benzimidazolyl)-4-methyl-3-thienylamin Hydrochlorid

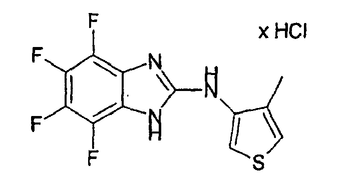

a) N-(2-Amino-3,4,5,6-tetrafluorphenyl)-N'-(4-methyl-3-thienyl)-thioharnstoff erhält man analog der in Beispiel 1, b) angegebenen Reaktion aus 4-Methyl-3-thienyl-isothiocyanat und 3,4,5,6-Tetrafluor-1,2-diaminobenzol. Kristalliner Feststoff, Schmp.: 286-290°C
b) 3N-(3,4,5,6,-Tetrafluor -2-benzimidazolyl)-4-methyl-3-thienylamin Hydrochlorid erhält man analog der in Beispiel 1, c) angegebenen Vorschrift aus N-(2-Amino-3,4,5,6-tetrafluorphenyl)-N'-(4-methyl-3-thienyl)-thiohamstoff und Methyljodid.
   Amorpher Niederschlag, der unter Ethylacetat kristallisiert. Kristalliner Feststoff, Schmp. 225-228°C

### Beispiel 8: 3N-(4-Methyl-2-benzimidazolyl)-4-methyl-3-thienylamin Hydrochlorid

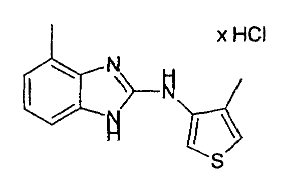

a) N-(2-Amino-3-methylphenyl)-N'-(4-methyl-3-thienyl)-thioharnstoff erhält man analog der in Beispiel 1, b) angegebenen Reaktion aus 4-Methyl-3-thienyl-isothiocyanat und 3-Methyl-1 ,2-diaminobenzol. Kristalliner Feststoff, Schmp. 184-186°C,
b) 3N-(4-Methyl-2-benzimidazolyl)-4-methyl-3-thienylamin Hydrochlorid erhält man analog der in Beispiel 1, c) angegebenen Vorschrift aus N-(2-Amino-3-methylphenyl)-N'-(4-methyl-3-thienyl)-thioharnstoff und Methyljodid. Amorpher Niederschlag, der unter Aceton kristallisiert. Kristalliner Feststoff, Zersetzungspunkt: 320 °C

### Beispiel 9: trans-3N-(3a,4,5,6,7,7a-Hexahydro-1H-2-benzimidazolyl)-4-methyl-3-thienylamin Hydrochlorid (Racemat)

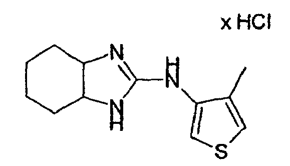

a) trans-N-(2-Amino-cyclohexyl)-N'-(4-methyl-3-thienyl)-thioharnstoff (Racemat) erhält man analog der in Beispiel 1, b) angegebenen Reaktion aus 4-Methyl-3-thienyl-isothiocyanat und racemischem trans-1,2-Diaminocyclohexan. Kristalliner Feststoff, Schmp. 205-210°C,
b) trans-3N-(3a,4,5,6,7,7a-Hexahydro-1H-2-benzimidazolyl)-4-methyl-3-thienylamin Hydrochlorid (Racemat)
   0,6g racemischer trans-N-(2-Amino-cyclohexyl)-N'-(4-methyl-3-thienyl)-thioharnstoff werden in 60 ml Toluol suspendiert und durch Erwärmen auf 90°C gelöst. Man lässt auf 70°C abkühlen, tropft eine Lösung von 0,376g Dicyclohexylcarbodiimid in 5 ml wasserfreiem Toluol zu, rührt insgesamt ca. 10 Stunden bei 70 °C und 2-3 Tage bei Raumtemperatur. Man filtriert den kristallinen Feststoff ab, entfernt das Lösungsmittel am Rotavapor unter vermindertem Druck und löst den so erhaltenen öligen Rückstand in wenig Ethylacetat. Nach Zugabe von wasserfreier Chlorwasserstoff-Lösung in Diethylether entsteht ein viskoser Niederschlag, der nach Zugabe von wenig Ethanol kristallisiert. Kristalliner Feststoff, Schmp.: 261-264 °C.

### Beispiel 10: trans- R,R-3N-(3a,4,5,6,7,7a-Hexahydro-1H-2-benzimidazolyl)-4-methyl-3-thienylamin Hydrochlorid

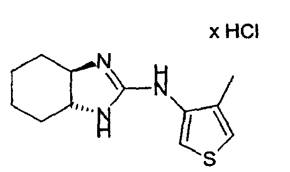

a) trans-R,R-N-(2-Amino-cyclohexyl)-N'-(4-methyl-3-thienyl)-thioharnstoff erhält man analog der in Beispiel 1, b) angegebenen Reaktion aus 4-Methyl-3-thienyl-isothiocyanat und trans-R,R-1,2-Diaminocyclohexan durch säulenchromatografische Trennung an Kieselgel durch Eluierung mit einem Lösungsmittelgemisch bestehend aus 10 Teilen Ethylacetat, 5 Teilen n-Heptan, 5 Teilen Methylenchlorid, 5 Teilen Methanol und 1 Teil 26 %iges wässeriges Ammoniak (nachfolgend als "Gemisch 4" bezeichnet) als amorphes öliges Produkt neben einem höhermolekularen kristallinen Produkt vom Schmp. 94-100°C.
   Das amorphe Produkt wird ohne weitere Aufreinigung weiterverarbeitet.
trans-R,R-N-(3a,4,5,6,7,7a-Hexahydro-1H-2-benzimidazolyl)-4-methyl-3-thienylamin Hydrochlorid
   erhält man analog der in Beispiel 1, c) angegebenen Vorschrift durch Umsetzung von R,R-N-(2-Amino-cyclohexyl)-N'-(4-methyl-3-thienyl)-thioharnstoff und Methyljodid in wasserfreiem Ethanol als Lösungs- und Reaktionsmedium. Amorpher Niederschlag der an Kieselgel mit Gemisch 4 als Elutionsmittel chromatografiert wird und unter Aceton kristallisiert. Kristalliner Feststoff, Schmp. 235 - 238 °C.

### Beispiel 11: trans-S,S-3N-(3a,4,5,6,7,7a-Hexahydro-1 H-2-benzimidazolyl)-4-methyl-3-thienylamin Hydrochlorid

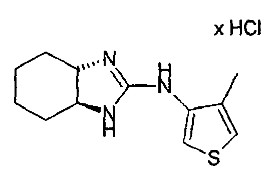

a) trans-S,S-3N-(2-Amino-cyclohexyl)-N'-(4-methyl-3-thienyl)-thiohamstoff erhält man analog der in Beispiel 1, b) angegebenen Reaktion aus 4-Methyl-3-thienyl-isothiocyanat und trans-S,S-1,2-Diaminocyclohexan durch säulenchromatografische Trennung an Kieselgel mit Gemisch 4 als Elutionsmittel als amorphes öliges Produkt neben einem höhermolekularen Produkt vom Schmp. 94-102°C.
   Das amorphe Produkt wird ohne weitere Aufreinigung weiterverarbeitet.
b) trans-S,S-N-(3a,4,5,6,7,7a-Hexahydro-1H-2-benzimidazolyl)-4-methyl-3-thienylamin Hydrochlorid
   erhält man analog der in Beispiel 1, c) angegebenen Vorschrift durch Umsetzung von S,S-N-(2-Amino-cyclohexyl)-N'-(4-methyl-3-thienyl)-thioharnstoff und Methyljodid in wasserfreiem Ethanol als Lösungs- und Reaktionsmedium. Amorpher Niederschlag der an Kieselgel mit Gemisch 4 als Elutionsmittel chromatografiert wird und unter Aceton kristallisiert. Kristalliner Feststoff, Schmp. 225 - 230 °C.

### Beispiel 12: 2-Chlor-3N-(2-benzimidazolyl)-4-methyl-3-thienylamin Hydrochlorid und 2,5-Dichlor-3N-(2-benzimidazolyl)-4-methyl-3-thienylamin Hydrochlorid

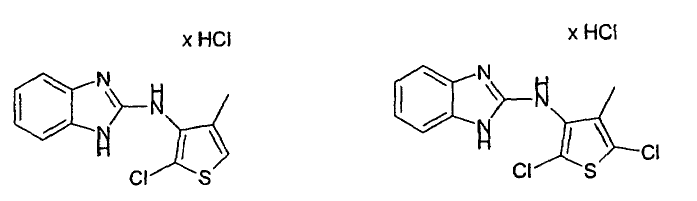

erhält man analog der in Beispiel 2 angegebenen Vorschrift aus 3N-(2-Benzimidazolyl)-4-methyl-3-thienylamin Hydrochlorid und N-Chlorsuccinimid in Eisessig. Die Säulenchromatografie an Kieselgel mit Gemisch 17 als Elutionsmittel führt zur Trennung des 2,5-Dichlor-3N-(2-benzimidazolyl)-4-methyt-3-thienylamin Hydrochlorid (farblose kristalline Verbindung, Schmp.: 291 °C) vom 2-Chlor-3N-(2-benzimidazolyl)-4-methyl-3-thienylamin Hydrochlorid (farblose kristalline Verbindung, Schmp. 257 - 259°C).

### Beispiel 13: 2-Chlor-3N-(4-methyl-2-benzimidazolyl)-4-methyl-3-thienylamin Hydrochlorid

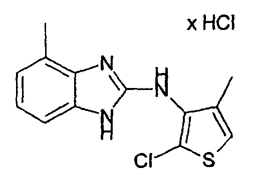

erhält man analog der in Beispiel 2 angegebenen Vorschrift aus 3N-(4-Methyl-2-benzimidazolyl)-4-methyl-3-thienylamin Hydrochlorid und N-Chlorsuccinimid in Eisessig. Nach Säulenchromatografie an Kieselgel mit Gemisch 17 als Elutionsmittel erhält man 2-Chlor-3N-(4-methyl-2-benzimidazolyl)-4-methyl-3-thienylamin Hydrochlorid als farbloses bis schwach gelblich gefärbtes kristallines Produkt. Schmp. 255-259°C.

### Beispiel 14: 2-Chlor-3N-(4,5,6,7-tetrafluor-2-benzimidazolyl)-4-methyl-3-thienylamin Hydrochlorid

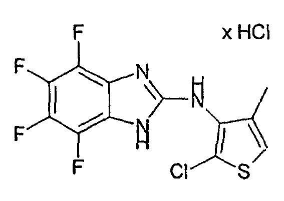

erhält man analog der in Beispiel 2 angegebenen Vorschrift: aus 3N-(4,5,6,7-Tetrafluor-2-benzimidazolyl)-4-methyl-3-thienylamin Hydrochlorid und N-Chlorsuccinimid in Eisessig. Kristallines Produkt. Schmp. 233-235°C.

### Beispiel 15: trans-2-Chlor-3N-(3a,4,5,6,7,7a-Hexahydro-1H-2-benzimidazolyl)-4-methyl-3-thienylamin Hydrochlorid (Racemat)

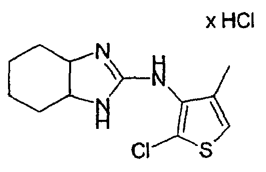

erhält man analog der in Beispiel 2 angegebenen Vorschrift: aus 3N-(3a,4,5,6,7,7a-Hexahydro-1H-2-benzimidazolyl)-4-methyl-3-thienylamin Hydrochlorid (Racemat) und N-Chlorsuccinimid in Eisessig. Kristallines Produkt. Schmp. 258-260°C.

### Beispiel 16: trans-R,R-2-Chlor-3N-(3a,4,5,6,7,7a,Hexahydro-1H-2-benzimidazolyl)-4-methyl-3-thienylamin Hydrochlorid und trans-R,R-2,5-Dichlor-3N-(3a,4,5,6,7,7a-Hexahydro-1H-2-benzimidazolyl)-4-methyl-3-thienylamin Hydrochlorid

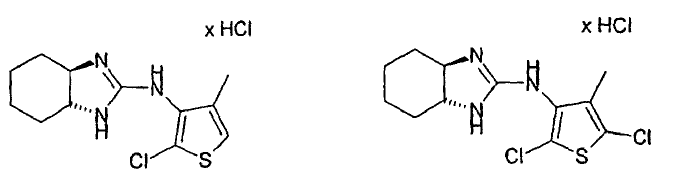

erhält man analog der in Beispiel 2 angegebenen Vorschrift: aus trans- R,R-3N-(3a,4,5,6,7,7a-Hexahydro-1H-2-benzimidazolyl)-4-methyl-3-thienylamin Hydrochlorid und N-Chlorsuccinimid in Eisessig nach chromatografischer Abtrennung die beiden kristallinen Produkte in nachfolgender Reihenfolge:
a) trans-R,R-2,5-Dichlor-3N-(3a,4,5,6,7,7a-Hexahydro-1H-2-benzimidazolyl)-4-methyl-3-thienylamin Hydrochlorid, Zersetzung unter Aufschäumen ab 80°C,
b) trans-R,R-2-Chlor-3N-(3a,4,5,6,7,7a-Hexahydro-1 H-2-benzimidazolyl)-4-methyl-3-thienylamin Hydrochlorid, kristallines Produkt. Schmp. 260-262°C.

### Beispiel 17: trans-S,S-2-Chlor-3N-(3a,4,5,6,7,7a-Hexahydro-1H-2-benzimidazolyl)-4-methyl-3-thienylamin Hydrochlorid

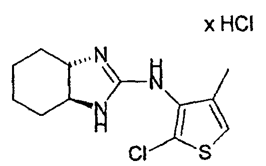

erhält man analog der in Beispiel 2 angegebenen Vorschrift: aus trans- S,S-3N-(3a,4,5,6,7,7a-Hexahydro-1 H-2-benzimidazolyl)-4-methyl-3-thienylamin Hydrochlorid und N-Chlorsuccinimid in Eisessig nach chromatografischer Abtrennung. Farbloses kristallines Produkt, Schmp. 258 - 260 °C

### Beispiel 18: 2-Brom-3N-(2-benzimidazolyl)-4-methyl-3-thienylamin Hydrochlorid und 2,5-Dibrom-3N-(2-benzimidazolyl)-4-methyl-3-thienylamin Hydrochlorid

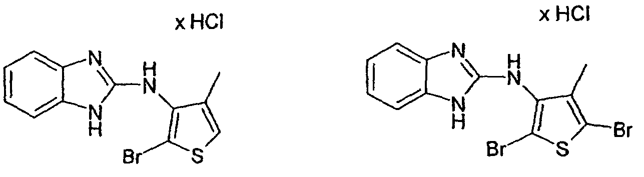

erhält man analog der in Beispiel 2 angegebenen Vorschrift: aus 3N-(2-Benzimidazolyl)-4-methyl-3-thienylamin Hydrochlorid und N-Bromsuccinimid (anstelle von N-Chlorsuccinimid) in Eisessig. Nach Säulenchromatografie an Kieselgel mit einem Gemisch aus 5 Teilen Dichlormethan, 3 Teilen n-Heptan, 1 Teil Eisessig und 1 Teil Ethanol (nachfolgend als "Gemisch 1" bezeichnet) als Elutionsmittel, und Behandlung mit einer Lösung von Chlorwasserstoffgas in Ether erhält man durch fraktionierter Kristallisation in Ethylacetat 2-Brom-3N-(2-benzimidazolyl)-4-methyl-3-thienylamin Hydrochlorid, kristallines Produkt vom Schmp. 228-231 °C, und 2,5-Dibrom-3N-(2-benzimidazolyl)-4-methyl-3-thienylamin Hydrochlorid, kristallines Produkt vom Schmp. 208-210°C.

### Beispiel 19: trans-R,R-2-Brom-3N-(3a,4,5,6,7,7a-Hexahydro-1H-2-benzimidazolyl)-4-methyl-3-thienylamin Hydrochlorid und trans-R,R-2,5-Dibrom-3N-(3a,4,5,6,7,7a-Hexahydro-1H-2-benzimidazolyl)-4-methyl-3-thienylamin Hydrochlorid

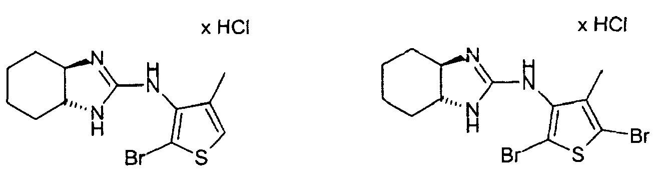

erhält man analog der in Beispiel 18 angegebenen Vorschrift: aus trans- R,R-3N-(3a,4,5,6,7,7a-Hexahydro-1H-2-benzimidazolyl)-4-methyl-3-thienylamin Hydrochlorid und N-Bromsuccinimid in Eisessig. Nach Säulenchromatografie an Kieselgel mit Gemisch 1 als Elutionsmittel, und Behandlung mit einer Lösung von Chlorwasserstoffgas in Ether erhält man nach fraktionierter Kristallisation in Ethylacetat
trans-R,R-2-Brom-3N-(3a,4,5,6,7,7a-Hexahydro-1H-2-benzimidazolyl)-4-methyl-3-thienylamin Hydrochlorid, kristallines Produkt, Schmp. 215-218°C, und trans-R,R-2,5-Dibrom-3N-(3a,4,5,6,7,7a-Hexahydro-1H-2-benzimidazolyl)-4-methyl-3-thienylamin Hydrochlorid, kristallines Produkt, Schmp. 218-220°C.

### Beispiel 20: 3N-(4-chlor-2-benzimidazolyl-amino)-4-methylthiophen Hydrochlorid

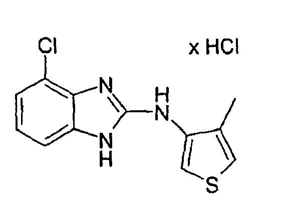

a) N-(2-Amino-3-chlorphenyl)-N'-(4-methyl-3-thienyl)-thioharnstoff
   erhält man analog der in Beispiel 1, b) angegebenen Reaktion aus 4-Methyl-3-thienyl-isothiocyanat und 3-Chlor-1,2-diaminobenzol (dargestellt durch katalytische Hydrierung von 3-Chlor-2-nitranilin mit Pt auf Aktivkohle unter Normaldruck bei Raumtemperatur). Kristalliner Feststoff, Schmp. 298-305°C,
b) 3N-(4-chlor-2-benzimidazolyl-amino)-4-methylthiophen Hydrochlorid
   erhält man analog der in Beispiel 1, c) angegebenen Vorschrift aus N-(2-Amino-3-chlorphenyl)-N'-(4-methyl-3-thienyl)-thioharnstoff und Methyljodid. Amorpher Niederschlag, der unter Ethylacetat kristallisiert. Kristalliner Feststoff, Zersetzungspunkt. 240 - 245 °C

### Beispiel 21: 2-Chlor-3N-(4-chlor-2-benzimidazolyl-amino)-4-methylthiophen Hydrochlorid

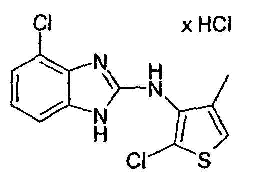

erhält man analog der in Beispiel 2 angegebenen Vorschrift: aus 3N-(4-Chlor-2-benzimidazolyl)-4-methyl-3-thienylamin Hydrochlorid und N-Chlorsuccinimid in Eisessig. Nach Säulenchromatografie an Kieselgel mit einem Gemisch aus 10 Teilen Methylenchlorid und 1 Teil Methanol als Elutionsmittel erhält man 2-Chlor-3N-(4-chlor-2-benzimidazolyl-amino)-4-methyl-thiophen Hydrochlorid als farblosen bis schwach gelblich gefärbten Feststoff nach Kristallisation unter Ethylacetat. Schmp. 270-272°C.

### Beispiel 22: 2-Brom-3N-(4-chlor-2-benzimidazolyl-amino)-4-methylthiophen Hydrochlorid

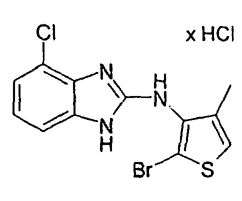

erhält man analog der in Beispiel 2 angegebenen Vorschrift: aus 3N-(4-Chlor-2-benzimidazolyl-amino)-4-methylthiophen Hydrochlorid und N-Bromsuccinimid (anstelle von N-Chlorsuccinimid) in Eisessig. Nach Säulenchromatografie an Kieselgel mit einem Gemisch aus 20 Teilen Ethylacetat, 10 Teilen n-Heptan und 3 Teilen Eisessig als Elutionsmittel, und Behandlung mit einer Lösung von Chlorwasserstoffgas in Ether erhält man durch fraktionierter Kristallisation in Ethylacetat in Gegenwart von Chlorwasserstoffgesättigtem Ether 2-Brom-3N-(4-chlor-2-benzimidazolyl-amino)-4-methylthiophen Hydrochlorid. Kristallines Produkt vom Schmp. 278-280°C

### Beispiel 23: (2-Brom-4-methyl-thiophen-3-yl)-(5-fluor-1 H-benzoimidazol-2-yl)-amin-Hydrochlorid

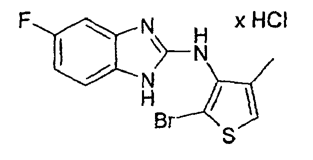

(5-Fluor-1H-benzoimidazol-2-yl)-(4-methyl-thiophen-3-yl)-amin (300mg) (Beispiel 1) wurde in Eisessig (50 ml) gelöst. Bei Raumtemperatur wurde unter kräftigem Rühren N - Bromsuccinimid (207 mg) gelöst in Eisessig (10 ml) langsam zugetropft. Nach beendeter Zugabe wurde noch 10 min nachgerührt, dann der Eisessig im Vakuum abdestilliert, der Rückstand in Essigester gelöst und mit gesättigter Kaliumcarbonat-Lösung gewaschen. Nach Trocknen über Magnesiumsulfat, wurde filtriert und eingeengt. Der Rückstand wurde mittels präparativer Chromatographie gereinigt, die Produkt-enthaltenden Fraktionen vereinigt, vom Acetonitril befreit, basisch gestellt und drei Mal mit Essigester extrahiert. Die organischen Phasen wurden vereinigt, getrocknet (MgSO₄) und filtriert. Nach Abziehen des Lösungsmittels wurde der Rückstand mit Wasser und 2 N Salzsäure versetzt und gefriergetrocknet. Es wurden 245 mg des gewünschten Produkts erhalten.
LCMS-Rt (B): 0,95 min
MS (ES⁺, M+H⁺): 326,09

### Beispiel 24: 2-Brom-3N-(4-fluor-2-benzimidazolyl-amino)-4-methylthiophen Hydrochlorid and 2,5-Dibrom-3N-(4-fluor-2-benzimidazolyl-amino)-4-methylthiophen Hydrochlorid

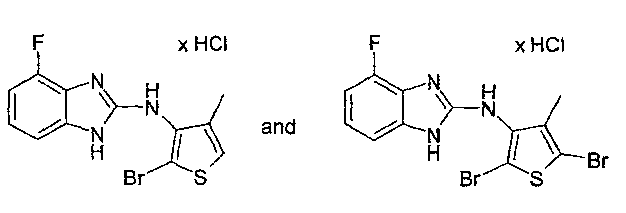

Zu einer Lösung aus 0,214 g 3N-(4-Fluor-2-benzimidazolyl-amino)-4-methylthiophen Hydrochlorid in 6 ml Eisessig wird eine Lösung von 0,161 g N-Bromsuccinimid in 6 ml Eisessig gegeben und die Mischung bei Raumtemperatur für 30 Minuten gerührt. Nach dem Abdestillieren des Lösungsmittels wird der Rückstand mit Wasser versetzt, mit zweinormaler NaOH alkalisch gestellt und mit Ethylacetat extrahiert. Die organische Phase wird getrocknet, das Lösungsmittel abdestilliert und der Rückstand durch Säulenchromatografie an Silicagel mit einem Gemisch aus 20 teilen Ethylacetat, 10 Teilen n-Heptan und 3 Teilen Eisessig aufgetrennt. Die Hydrochloride der beiden Verbindungen erhält man durch Abdestillieren der fraktionierten Lösungen, Auflösen in Ethylacetat und Fällung durch Zugabe von Chlorwasserstoff-gesättigtem Diethylether. Dabei wurde die Kristallisation durch leichtes Erwärmen gefördert.
Beispiel 24a: 2-Brom-3N-(4-fluor-2-benzimidazolyl-amino)-4-methylthiophen Hydrochlorid: Farblose Kristalle; Schmp. 212 °C (Zersetzung).
Beispiel 24b: 2,5-Dibrom-3N-(4-fluor-2-benzimidazolyl-amino)-4-methylthiophen Hydrochlorid: Farblose Kristalle; Schmp. 242 - 244 °C (Zersetzung).

### Beispiel 25: 3N-(5-Methoxy-2-benzimidazolyl-amino)-4-methylthiophen

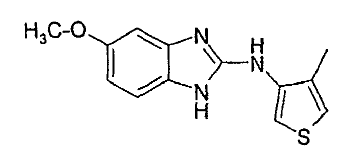

a) N-(2-Amino-4-methoxyphenyl)-N'-(4-methyl-3-thienyl)-thioharnstoff
   Eine Mischung aus 5,89g 4-Methylthiophen-3-isothiocyanat und 5g 4-Methoxy-1,2-diaminobenzol in 60ml wasserfreiem THF wird 2 Stunden bei Raumtemperatur gerührt und das Lösungsmittel abdestilliert. Man versetzt den Rückstand mit Wasser, extrahiert mit Ethylacetat, behandelt die dunkle Lösung mit Aktivkohle und verdampft erneut das organische Lösungsmittel. Der Rückstand wird mehrfach unter leichtem Erwärmen mit Diisopropylether behandelt und der Feststoff filtriert. Braun gefärbter kristalliner Feststoff, Schmp. 143 - 146°C.
b) Eine Mischung aus 2,83g N-(2-Amino-4-methoxyphenyl)-N'-(4-methyl-3-thienyl)-thioharnstoff, 8,5 g Methyljodid und 100ml wasserfreies Ethanol wird 5 Stunden unter Rückfluss gekocht, sodann das Lösungsmittel abdestilliert und der Rückstand mit Wasser versetzt. Man stellt mit 2N Natronlage alkalisch, extrahiert mit Ethylacetat, behandelt die organische Phase mit Wasser und anschließend mit Aktivkohle und reinigt durch Säulenchromatografie an Kieselgel mit einem Elutionsmittelgemisch aus 20 Teilen Essigester, 10 Teilen n-Heptan und 3-Teilen Eisessig. Man erhält 3N-(5-Methoxy-2-benzimidazolyl-amino)-4-methylthiophen als amorphes Produkt.

### Beispiel 26: 3N-(5-Methoxy-2-benzimidazolyl-amino)-4-methylthiophen Hydrochlorid

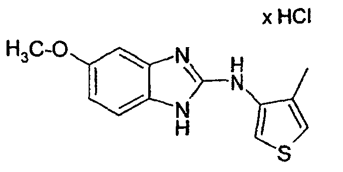

erhält man durch Fällung einer Lösung von 0,2g 3N-(5-Methoxy-2-benzimidazolyl-amino)-4-methylthiophen (Beispiel 25) in 10 ml Ethylacetat mit einer gesättigten Lösung von Chlorwasserstoffgas in Diethylether als kristallinen Niederschlag. Schmp.: 222 - 225°C

### Beispiel 27: 2-Chlor-3N-(5-Methoxy-2-benzimidazolyl-amino)-4-methylthiophen Hydrochlorid

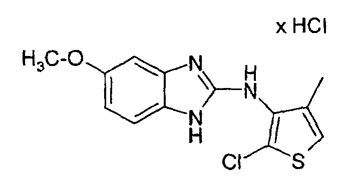

Eine Mischung aus 0,519 g 3N-(5-Methoxy-2-benzimidazolyl-amino)-4-methylthiophen Hydrochlorid, 0,046g N-Chlorsuccinimid und 10-15 ml Eisessig werden für ca 2 bis 2 ½ Stunden auf 45°C erwärmt. Sodann wird der Eisessig abdestilliert, zum Rückstand Wasser gegeben und mit 2N NaOH auf pH 9 - 10 gestellt. Man extrahiert mit Ethylacetat, verdampft das Lösungsmittel und chromatografiert den Rückstand an Kieselgel auf einer Mitteldrucksäule mit einer Mischung aus 20 Teilen Essigester, 10 Teilen n-Heptan und 3-Teilen Eisessig. Die nach Abdestillieren des Lösungsmittels erhaltene Base wird in Ethylacetat mit gesättigter etherischer Chlorwasserstofflösung in das Hydrochlorid umgewandelt und unter Ethylacetat zur Kristallisation gebracht. Kristalliner Feststoff, Schmp.: 182 - 186 °C.

### Beispiel 28: 2,5-Dichlor-3N-(5-methoxy-2-benzimidazolyl-amino)-4-methylthiophen Hydrochlorid

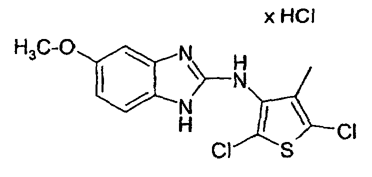

Bei analoger Aufarbeitung eines Reaktionsgemisches aus 3N-(5-Methoxy-2-benzimidazolyl-amino)-4-methylthiophen Hydrochlorid, N-Chlorsuccinimid und Eisessig über ca 2 bis 2 ½ Stunden bei 55°C, wurde 2,5-Dichlor-3N-(5-Methoxy-2-benzimidazolyl-amino)-4-methylthiophen erhalten. Kristalliner Feststoff, Schmp.: 278 - 282 °C.

### Beispiel 29: 3N-(4-Methoxy-2-benzimidazaly(-amino)-4-methylthiophen Hydrochlorid

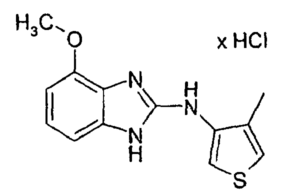

a) 3-Methoxy-1,2-diaminobenzol
   wurde als braunes Öl durch Hydrierung von 2-Methoxy-6-nitroanilin mit Wasserstoffgas und Raney-Nickel als Katalysator bei Raumtemperatur und 3 bar Druck erhalten. Das Produkt wurde ohne weitere Reinigung zum Thioharnstoff umgesetzt.
b) N-(2-Amino-3-methoxyphenyl)-N'-(4-methyl-3-thienyl)-thioharnstoff erhält man analog der in Beispiel 25 a) beschriebenen Vorschrift aus 3-Methoxy-1,2-diaminobenzol, 4-Methyl-3-thienylisothiocyanat in wasserfreiem THF und anschließender Mitteldruck-Chromatographie an Kieselgel mit einem Gemisch aus 1 Teil Toluol und 1 Teil Essigester. Kristalliner Feststoff, Schmp.:148-153°C. Erstarrung der Schmelze und nächster Schmp. bei 260°C.
c) 3N-(4-Methoxy-2-benzimidazolyl-amino)-4-methylthiophen Hydrochlorid erhält man analog der unter Beispiel 25 und 26 beschriebenen Vorschriften aus N-(2-Amino-3-methoxyphenyl)-N'-(4-methyl-3-thienyl)-thioharnstoff durch Erwärmen mit Methyljodid in THF, analoger Aufarbeitung und Behandlung des Benzimidazols mit HCl in Ether.
   Kristalliner Feststoff, Schmp.: 230 - 235 °C.

### Beispiel 30: 2-Chlor-3N-(4-methoxy-2-benzimidazolyl-amino)-4-methytthiophen Hydrochlorid

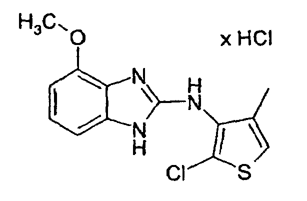

Eine Mischung aus 0,1 g 3N-(4-Methoxy-2-benzimidazolyl-amino)-4-methylthiophen Hydrochlorid, 0,046g N-Chlorsuccinimid und 10-15 ml Eisessig wird für ca 2 bis 2½ Stunden auf 40°C erwärmt. Sodann wird der Eisessig abdestilliert, zum Rückstand Wasser gegeben und mit 2N NaOH auf pH 9 - 10 gestellt. Man extrahiert mit Ethylacetat, verdampft das Lösungsmittel und chromatografiert den Rückstand an Kieselgel auf einer Mitteldrucksäule mit einer Mischung aus 20 Teilen Essigester, 10 Teilen n-Heptan und 3-Teilen Eisessig. Die so erhaltene Base wird in Ethylacetat mit gesättigter etherischer Chlorwasserstofflösung in das Hydrochlorid umgewandelt. Farbloser bis hellgelber kristalliner Feststoff, Schmp: 248 - 250 °C.

### Beispiel 31: 3N-(4-Chlor-6-trifluormethyl-2-benzimidazolyl-amino)-4-methylthiophen Hydrochlorid

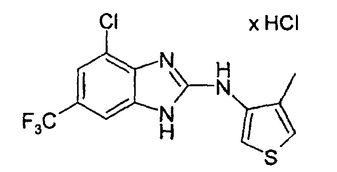

a) N-(2-Amino-3-chlor-5-trifluormethylphenyl)-N'-(4-methyl-3-thienyl)-thioharnstoff
   erhält man analog der in Beispiel 25 a) beschriebenen Vorschrift durch Reaktion von 3-Chlor-5-trifluormethyl-1,2-diaminobenzol und 4-Methyl-3-thienylisothiocyanat in wasserfreiem THF über 3 Tage bei Raumtemperatur. Nach Abdestillieren des Lösungsmittels und Zugabe von Wasser zum Rückstand extrahiert man mit Ethylacetat, destilliert erneut das Lösungsmittel ab und bringt den amorphen Rückstand unter Diisopropylether zur Kristallisation. Schmp.: > 310°C.
b) 3N-(4-Chlor-6-trifluormethyl-2-benzimidazolyl-amino)-4-methylthiophen Hydrochlorid
   erhält man analog der unter Beispiel 25 und 26 beschriebenen Vorschriften aus N-(2-Amino-3-chlor-5-trifluormethylphenyl)-N'-(4-methyl-3-thienyl)-thioharnstoff durch Kochen unter Rückflußbedingungen über 5 Stunden mit Methyljodid in THF, analoger Aufarbeitung und Reinigung durch Mitteldruck-Säulenchromatographie an Kieselgel mit einem Gemisch aus gleichen Volumenteilen Essigester und Toluol. Nach dem Verdampfen des Lösungsmittels löst man den Rückstand in Ethylacetat und erhält das 3N-(4-Chlor-6-trifluormethyl-2-benzimidazolyl-amino)-4-methylthiophen Hydrochlorid als kristallinen Niederschlag durch Zugabe einer gesättigten Lösung von Chlorwasserstoff in Diethylether. Feststoff, Fp.: 210 - 213°C.

### Beispiel 32: 2-Chlor-3N-(4-Chlor-6-trifluormethyl-2-benzimidazolyl-amino)-4-methylthiophen Hydrochlorid

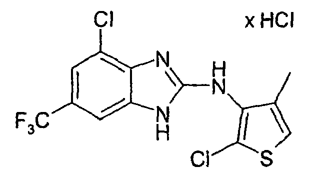

Eine Mischung aus 0,34 g 3N-(4-Methoxy-2-benzimidazolyl-amino)-4-methylthiophen Hydrochlorid, 0,151g N-Chlorsuccinimid und 20 ml Eisessig wird für ½ Stunde bei Raumtemperatur und eine Stunde auf 60°C erwärmt. Sodann wird der Eisessig abdestilliert, zum Rückstand Wasser gegeben und mit 2N NaOH auf pH 9 - 10 gestellt. Man extrahiert mit Ethylacetat, verdampft das Lösungsmittel und chromatografiert den Rückstand an Kieselgel auf einer Mitteldrucksäule mit einer Mischung aus gleichen Teilen Toluol und Ethylacetat. Nach Abdestillieren des Lösungsmittels wird die so erhaltene Base wird in Ethylacetat mit gesättigter etherischer Chlorwasserstofflösung in das Hydrochlorid umgewandelt. Farbloser bis hellgelber kristalliner Feststoff, Schmp.: 247 - 250 °C.

### Beispiel 33: 3N-(4-Carboxy-2-benzimidazolyl-amino)-4-methylthiophen Hydrochlorid

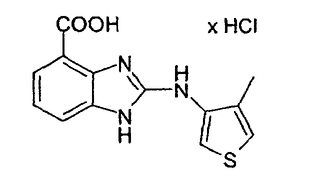

a) N-(2-Amino-3-carboxyphenyl)-N'-(4-methyl-3-thienyl)-thioharnstoff
   erhält man analog der in Beispiel 25 a) beschriebenen Vorschrift aus 3-Carboxy-1,2-diaminobenzol, 4-Methyl-3-thienylisothiocyanat in wasserfreiem THF und anschließender Mitteldruck-Chromatographie an Kieselgel mit einem Gemisch aus 12 Teilen Methylenchlorid und 1 Teil Methanol. Amorphes Produkt.
b) 3N-(4-Carboxy-2-benzimidazolyl-amino)-4-methylthiophen Hydrochlorid
   erhält man durch refluxierendes Kochen einer Lösung aus 1,12 g N-(2-Amino-3-carboxyphenyl)-N'-(4-methyl-3-thienyl)-thioharnstoff und 3,1 g Methyljodid in 60 ml Ethanol. Man verdampft das Lösungsmittel, versetzt den Rückstand mit Wasser, stellt mit 2N wässriger HCl auf pH 5 und filtriert den Niederschlag. Kristalliner Feststoff, Zersetzungspunkt: 265 - 285°C.

### Beispiel 34: 2-Chlor-3N-(4-Carboxy-2-benzimidazolyl-amino)-4-methylthiophen Hydrochlorid

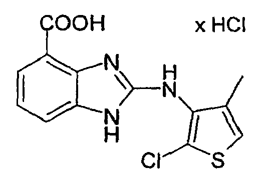

erhält man analog zu der in Beispiel 32 beschriebenen Vorschrift aus 0,2g 3N-(4-Carboxy-2-benzimidazolyl-amino)-4-methylthiophen Hydrochlorid und 0,103g N-Chlorsuccinimid in 20-25ml Eisessig und Fällung des entsprechenden Hydrochlorids mit HCI-gesättigtem Diethylether in Ethylacetat und anschließender Kristallisation unter Diisopropylether und Essigester. Zersetzungspunkt 170°C.

### Beispiel 35: 3N-[4-(1-Piperidinocarbonyl)-2-benzimidazolyl-amino]-4-methylthiophen Hydrochlorid

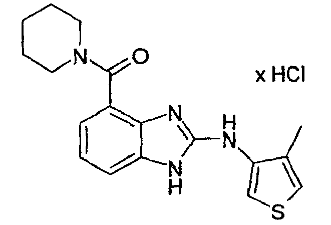

Zu einem Gemisch aus 0,330g 3N-(4-Carboxy-2-benzimidazolyl-amino)-4-methylthiophen Hydrochlorid, 30ml wasserfreiem THF und 5ml wasserfreiem Dimethylacetamid gibt man 0,215g N,N'-Carbonyldimidazol. Man rührt ca 4 Stunden bei Raumtemperatur, wobei die Kohlendioxidentwicklung beendet ist, und versetzt sodann mit 0,411g Piperidin. Die Lösung wird 2 Stunden bei Raumtemperatur gerührt und das Lösungsmittel nach weiterem Stehenlassen über Nacht unter vermindertem Druck abdestilliert. Man verrührt den Rückstand mit Wasser, filtriert den Feststoff ab, löst diesen in Ethylacetat, entfernt den unlöslichen Teil durch Filtration und destilliert das Lösungsmittel unter vermindertem Druck ab. Schaumiges amorphes Produkt.

### Beispiel 36: 2-Chlor-4-methyl-3N-[4-(1-piperidinocarbonyl)-2-benzimidazolyl-amino] thiophen Hydrochlorid

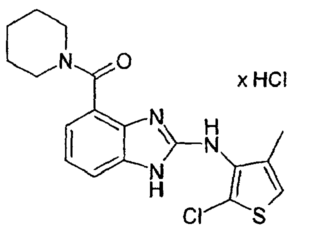

Eine Mischung aus 0,2g 3N-[4-(1-Piperidinocarbonyl)-2-benzimidazolyl-amino]-4-methylthiophen Hydrochlorid und 0,086g N-Chlorsuccinimid in ca 20ml Eisessig wird über 1 ½ Stunden bei Raumtemperatur und ca 30min bei 35°C gerührt, das Lösungsmittel abdestilliert und der Rückstand nach Zugabe von Wasser mit 2N NaOH alkalisch gestellt. Nach Extraktion mit Ethylacetat verdampft man das Lösungsmittel und reinigt durch Mitteldruck-Chromatographie auf der Säule an Kieselgel mit einem Gemisch aus 20 Teilen Ethylacetat, 10 Teilen n-Heptan und 3 Teilen Eisessig. Man destilliert das Lösungsmittel ab, löst in Ethylacetat und stellt mit Chlorwasserstoffgesättigter Etherlösung sauer. Der amorphe Rückstand wird unter einem Gemisch von Essigester mit wenig Aceton und wenig Ethanol zur Kristallisation gebracht. Amorpher Feststoff, Zersetzungspunkt ab 100°C.

### Beispiel 37: 2-Chlor-3N-(4-fluor-2-benzimidazolyl-amino)-4-methylthiophen Hydrochlorid

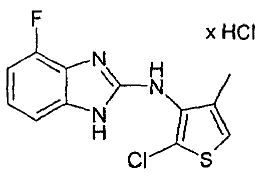

0,234g 3N-(4-Fluor-2-benzimidazolyl-amino)-4-methylthiophen Hydrochlorid werden in etwa 20ml Eisessig mit 0,132 g N-Chlorsuccinimid versetzt, 30 Minuten bei Raumtemperatur und weitere 1 % Stunden bei 50 - 60°C gerührt. Nach Abdestillieren der Essigsäure unter vermindertem Druck versetzt man den Rückstand mit Wasser und stellt mit 2N NaOH ungefähr auf pH 10 - 11, extrahiert mit Ethylacetat, das anschließend abdestilliert wird. Der Rückstand wird an Kieselgel auf einer Säule unter Mitteldruck-Bedingungen mit einem Gemisch aus 20 Teilen Ethylacetat, 10 Teilen n-Heptan und 3 Teilen Eisessig chromatographiert. Nach dem Einengen wird der Rückstand in wenig Ethylacetat gelöst und das Hydrochlorid durch Zugabe von Chlorwasserstoff-gesättigtem Diethylether ausgefällt.
Farbloser bis hellgelber kristalliner Feststoff. Schmp: 268 - 270 °C.

### Beispiel 38: 2-Chlor-3N-(4-hydroxy-2-benzimidazolyl-amino)-4-methylthiophen Hydrochlorid

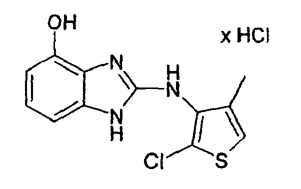

Zu einer Suspension von 0,29g aktivem wasserfreien Aluminiumchlorid in 10 ml wasserfreiem Methylenchlorid gibt man eine Suspension von 0,13g 2-Chlor-3N-(4-methoxy-2-benzimidazolyl-amino)-4-methylthiophen Hydrochlorid in etwa 20ml wasserfreiem Methylenchlorid und rührt das Reaktionsgemisch 2 Stunden bei 55°C. Nach dem Abkühlen gießt man das Reaktionsgemisch auf Eiswasser, extrahiert mit Ethylacetat, trocknet die organische Phase über Natriumsulfat und destilliert das Lösungsmittel ab. Der Rückstand wird auf der Säule an Kieselgel unter Mitteldruckbedingungen mit einem Gemisch aus 20 Teilen Essigester, 10 Teilen n-Heptan und 3 Teilen Eisessig chromatographiert und das Eluat unter vermindertem Druck eingedampft. Man löst den Rückstand mit Ethylacetat und fällt das Hydrochlorid durch Zugabe von Chiorwasserstoff-gesättigtem Diethylether. Kristalliner Feststoff. Schmp. 246-248°C.

### Beispiel 39: 2-Chlor-3N-(2-benzimidazolylamino)-4-methylthiophen

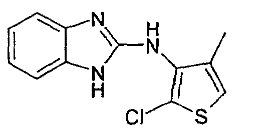

erhält man durch Zugabe von 2N NaOH zu einer Lösung von 3g 2-Chlor-3N-(2-benzimidazolylamino)-4-methylthiophen Hydrochlorid in 200ml Wasser, bis ein pH 10 eingestellt ist. Man filtriert die Kristalle ab und wäscht mehrfach mit Wasser nach. Ausbeute: 2,52g. Farbloses Kristallpulver. Schmp. 182-185°C.

### Beispiel 40: 2-Chlor-3N-(2-benzimidazolylamino)-4-methylthiophen Hydrobromid

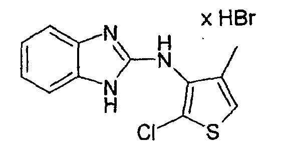

0,25g 2-Chlor-3N-(2-benzimidazolylamino)-4-methylthiophen werden in 10ml Ethanol gelöst, sodann mit 0,1 ml 48%iger HBr versetzt und kurze Zeit bei Raumtemperatur gerührt. Man destilliert das Lösungsmittel ab und bringt den Rückstand unter Ethylacetat zur Kristallisation. Ausbeute: 0,29g. Farblose Kristalle, Zersetzungspunkt: 252-254 °C.

### Beispiel 41: 2-Chlor-3N-(2-benzimidazolylamino)-4-rnethytthiophen Adipinsäuresatz

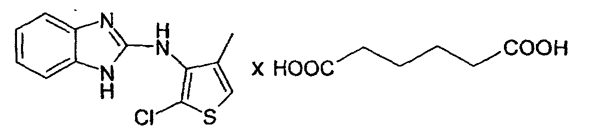

erhält man analog der in Beispiel 40 beschriebenen Vorschrift aus 2-Chlor-3N-(2-benzimidazolylamino)-4-methylthiophen mit einem Equivalent Adipinsäure. Farblose Kristalle. Schmp. 155 -157°C.

### Beispiel 42: 2-Chlor-3N-(2-benzimidazolylamino)-4-methylthiophen Oxalsäuresalz

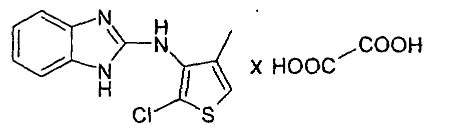

erhält man analog der in Beispiel 40 beschriebenen Vorschrift durch Umsetzung von 2-Chlor-3N-(2-benzimidazotylamino)-4-methylthiophen mit einem Equivalent Oxalsäure in Ethylacetat. Farblose Kristalle. Schmp: 220 - 222°C.

### Beispiel 43: 2-Chlor-3N-(2-benzimidazolylamino)-4-methylthiophen Phosphorsäuresalz

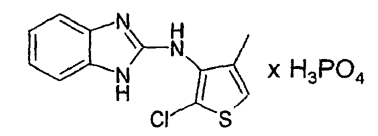

erhält man analog der in Beispiel 40 beschriebenen Vorschrift aus 2-Chlor-3N-(2-benzimidazolylamino)-4-methylthiophen mit einem Equivalent Phosphorsäure. Farblose Kristalle. Zersetzungsbereich: 113 - 175°C.

### Beispiel 44: (1H-Benzimidazol-2-yl)-(2-chlor-4-methyl-thiophen-3-yl)-methyl-amin

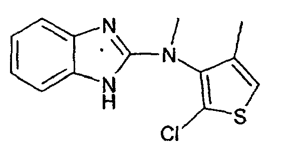

Zu einer Lösung aus 2-Chlor-3N-(2-benzimidazolyl)-4-methyl-3-thienylamin (125 mg, aus Beispiel 39) und trockenem Methanol (20 ml) wurde feingepulvertes, trockenes Kaliumcarbonat (66 mg) gegeben. Anschließend wurde unter Feuchtigkeitsausschluss und starkem Rühren Methyliodid (74 mg) zugetropft und das Gemisch drei Tagen unter Rückfluss gehalten. Nach Abziehen des Lösungsmittels wurde der Rückstand zwischen Essigester und Wasser verteilt, die Essigesterphase mit Magnesiumsulfat getrocknet, das Magnesiumsulfat abfiltriert und das Filtrat zur Trockne gebracht. Anschließend wurde mittels präparativer HPLC aufgereinigt. Die Produkt-enthaltenden Fraktionen wurden vereinigt und nach Abziehen des Acetonitrils gefriergetrocknet. Zur weiteren Aufreinigung wurde abschließend mit Essigester/Heptan (1/4) über Kieselgel chromatographiert. Nach Vereinigen der Produkt-enthaltenden Fraktionen wurde zur Trockne gebracht, mit HCl aufgenommen und gefriergetrocknet. Es wurden 5 mg Feststoff erhalten.
LCMS-Rt (A): 2,04 min
MS (ES⁺, M+H⁺): 278,05

### Beispiel 45: (5,6-Difluor-1H-benzimidazol-2-yl)-(4-methyl-thiophen-3-yl)-amin-Trifluoressigsäuresalz

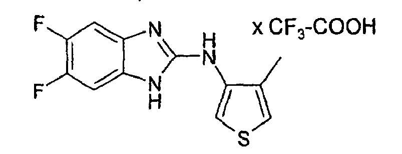

Zu einer Lösung aus 1,2-Diamino-4,5-Difluorbenzol (1 g) in absolutem Tetrahydrofuran (20 ml) wurde 3-Isothiocyanato-4-methylthiophen (1,08 g) gelöst in absolutem Tetrahydrofuran (30 ml) getropft. Anschließend wurde 2 Stunden bei Raumtemperatur gerührt und über Nacht stehen gelassen. Nach Zugabe von Methyliodid (0,44 ml) wurde 8 Stunden gerührt und über Nacht stehen gelassen. Danach wurde das Tetrahydrofuran abgezogen, der Rückstand zwischen Essigester und Wasser verteilt, die Phasen getrennt und die Essigesterphase über Magnesiumsulfat getrocknet. Der Rückstand wurde auf Kieselgel aufgezogen und über Kieselgel mit dem Laufmittel n-Heptan : Essigester = 1:1 chromatographiert. Es wurden 229 mg der gewünschten Verbindung als freie Base erhalten.
Eine verunreinigte Fraktion aus obiger Chromatographie wurde mittels präparativer HPLC gereinigt. Dabei wurden nach Gefriertrocknung 42,2 mg der gewünschten Verbindung als Trifluoressigsäuresalz isoliert.
LCMS-Rt (A): 1,98 min
MS (ES⁺, M+H⁺): 266,13

### Beispiel 46: (2-Chlor-4-methyl-thiophen-3-yl)-(5,6-difluor-1 H-benzoimidazol-2-yl)-amin-Hydrochlorid

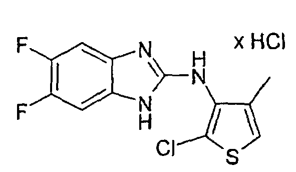

Zu einer Lösung von (5,6-Difluor-1 H-benzoimidazol-2-yl)-(4-methyl-thiophen-3-yl)-amin (225 mg) in Eisessig (5 ml) wurde bei Raumtemperatur eine Lösung aus N-Chlorsuccinimid (124,6 mg) in Eisessig (5 ml) getropft. Dann wurde 3,5 Stunden bei Raumtemperatur gerührt. Anschließend wurde der Eisessig entfernt, der Rückstand in Wasser aufgenommen und mit 2 M Natronlauge auf pH 10 gebracht. Die wässrige Phase wurde drei Mal mit Ethylacetat extrahiert, die vereinigten organischen Phasen über Magnesiumsulfat getrocknet und das Lösungsmittel entfernt. Der Rückstand wurde mittels präparativer Chromatographie gereinigt, die Produkt-enthaltenden Fraktionen vereinigt, vom Acetonitril befreit, basisch gestellt und drei Mal mit Essigester extrahiert. Die organischen Phasen wurden vereinigt, getrocknet (MgSO₄), filtriert und eingeengt. Der Rückstand wurde in Wasser aufgenommen, mit 10 % iger Salzsäure angesäuert und gefriergetrocknet. Es wurden 81 mg des gewünschten Produkts als Feststoff erhalten.
LCMS-Rt (A): 2,15 min
MS (ES⁺, M+H⁺): 300,11

### Beispiel 47: (2-Brom-4-methyl-thiophen-3-yl)-(5,6-difluor-1H-benzoimidazol-2-yl)-amin

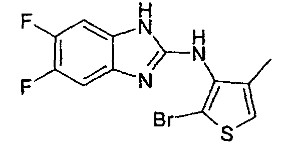

Bei Raumtemperatur wurde in einem ReactiVial zu einer Lösung aus (5,6-Difluor-1 H-benzoimidazol-2-yl)-(4-methyl-thiophen-3-yl)-amin-Trifluoressigsäuresalz (15 mg, Beispiel 45) in Eisessig (0,5 ml) eine Lösung aus N-Bromsuccinimid (8 mg) in Eisessig (0,5 ml) getropft und 0,5 h bei Raumtemperatur gerührt. Anschließend wurde die Essigsäure abgezogen und der Rückstand mit gesättigter Kaliumcarbonat-Lösung und Essigester versetzt. Nach Abtrennen der organischen Phase wurde zwei Mal mit Essigester extrahiert. Die vereinigten organischen Phasen wurden mit Magnesiumsulfat getrocknet und anschließend das Trockenmittel abfiltriert. Der nach Abziehen des Lösungsmittels verbliebene Rückstand wurde mittels präparativer Chromatographie gereinigt. Die Produkt-enthaltenden Fraktionen wurden vereinigt, vom Acetonitril befreit, mit gesättigter Natriumbicarbonat-Lösung versetzt und drei Mal mit Essigester extrahiert. Die organischen Phasen wurden vereinigt, getrocknet (MgSO₄) und filtriert. Nach Abziehen des Essigesters wurde der Rückstand mit Toluol abgezogen und dann im Hochvakuum getrocknet. Es wurden 8,1 mg der gewünschten Verbindung erhalten.
LCMS-Rt (D): 1,45 min
MS (ES⁺, M+H⁺): 343,96

### Beispiel 48: [(2-Chlor-4-methyl-thiophen-3-yl)-(4,5,6,7-tetrahydro-1H-benzoimidazol-2-yl]-amin-Hydrochlorid

### a) 1,4-Dioxa-spiro[4.5]dec-6-ylamin

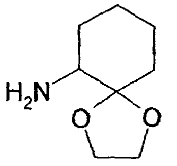

Das als Vorstufe benötigte Amin wurde entsprechend GB1131191 dargestellt. Dabei wurde 2-Chlorcyclohexanon mit Phthalimid zu 2-(2-Oxo-cyclohexyl)-isoindol-1,3-dion umgesetzt, welches mit Ethylenglykol in Gegenwart von para-Toluolsulfonsäure zu 2-(1,4-Dioxa-spiro[4.5]dec-6-yl)-isoindol-1,3-dion ketalisiert wurde. Nach Behandlung mit Hydrazin-Hydrat zur Abspaltung des Phthalimidrestes wurde das gewünschte 1,4-Dioxa-spiro[4.5]dec-6-ylamin erhalten.

### b) 1-(1,4-Dioxa-spiro[4.5]dec-6-yl)-3-(4-methyl-thiophen-3-yl)-thioharnstoff

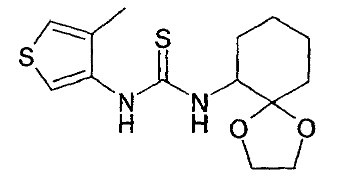

Zu einer Lösung aus 1,4-Dioxa-spiro[4.5]dec-6-ylamin (300 mg) in absolutem Tetrahydrofuran (10 ml) wurde eine Lösung aus 3-Isothiocyanato-4-methyl-thiophen (296,2 mg, siehe Beispiel 1a) in absolutem Tetrahydrofuran (10 ml) getropft, 2 Stunden bei Raumtemperatur gerührt und anschließend das Lösungsmittel abgezogen. Der Rückstand wurde mittels präparativer Chromatographie gereinigt, die Produkt-enthaltenden Fraktionen vereinigt, vom Acetonitril befreit, basisch gestellt und drei Mal mit Essigester extrahiert. Die organischen Phasen wurden vereinigt, getrocknet (MgSO4) und filtriert. Es wurden 428 mg des gewünschten Produkts erhalten.
LCMS-Rt (A): 3,57 min
MS (ES⁺, M+H⁺): 313,19

### c) 1-(1,4-Dioxa-spiro[4.5]dec-6-yl)-2-methyl-3-(4-methyl-thiophen-3-yl)-isothioharnstoff

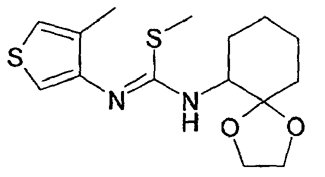

1-(1,4-Dioxa-spiro[4.5]dec-6-yl)-3-(4-methyl-thiophen-3-yl)-thiohamstoff (393 mg) wurde in absolutem Tetrahydrofuran (8,5 ml) gelöst und mit einer Lösung aus Methyliodid (179 mg) in absolutem Tetrahydrofuran (0,5 ml) versetzt. Anschließend wurde 2 Tage im Sandbad bei 70 ° C gerührt. Dann wurde das Reaktionsgemisch mit Essigester versetzt und zwei Mal mit Wasser gewaschen. Die organische Phase wurde über Magnesiumsulfat getrocknet und nach Filtrieren das Lösungsmittel entfernt. Der Rückstand wurde mittels präparativer Chromatographie gereinigt, die Produkt-enthaltenden Fraktionen vereinigt, vom Acetonitril befreit, basisch gestellt und drei Mal mit Essigester extrahiert. Die organischen Phasen wurden vereinigt, getrocknet (MgSO₄) und filtriert. Es wurden 59 mg des gewünschten Produkts erhalten, das direkt in der nächsten Stufe eingesetzt wurde.
LCMS-Rt (C): 1,05 min
MS (ES⁺, M+H⁺): 327,4

### d) N-(1,4-Dioxa-spiro[4.5]dec-6-yl)-N'-(4-methyl-thiophen-3-yl)-guanidin

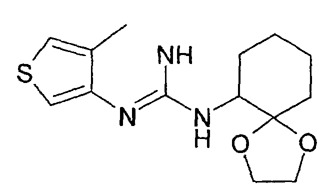

In einem ReactiVial wurde 1-(1,4-Dioxa-spiro[4.5]dec-6-yl)-2-methyl-3-(4-methylthiophen-3-yl)-isothioharnstoff (58,8 mg) mit 7 M ammoniakalischer Methanol-Lösung (2 ml) versetzt und 16 Stunden bei etwa 100 °C im Sandbad erwärmt. Nach entfernen des Lösungsmittels blieben 51 mg eines öligen Produkts zurück, welches direkt weiter umgesetzt wurde.
LCMS-Rt (C): 1,00 min
MS (ES⁺, M+H⁺): 296,4

### e) N-(2-Chlor-4-methyl-thiophen-3-yl)-N'-(1,4-dioxa-spiro[4,5]dec-6-yl)-guanidin

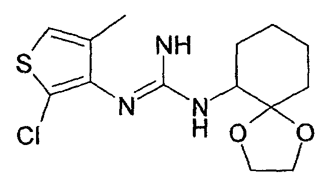

N-(1,4-Dioxa-spiro[4.5]dec-6-yl)-N'-(4-methyl-thiophen-3-yl)-guanidin (49 mg) wurde in Eisessig (3 ml) gelöst und langsam mit einer Lösung aus N-Chlorsuccinimid (20,3 mg) in Eisessig (5 ml) versetzt. Nach mehreren Stunden Rühren und Stehen übers Wochenende bei Raumtemperatur wurde der Eisessig abgezogen, der Rückstand mit Wasser aufgenommen und mit 2 N Natriumhydroxid-Lösung auf pH 10 gestellt. Die basische Phase wurde drei Mal mit Essigester extrahiert, die vereinigten organischen Phasen über Magnesiumsulfat getrocknet, filtriert und eingeengt. Der Rückstand wurde mittels präparativer Chromatographie gereinigt, die Produkt-enthaltenden Fraktionen vereinigt, vom Acetonitril befreit, basisch gestellt und drei Mal mit Essigester extrahiert. Die organischen Phasen wurden vereinigt, getrocknet (MgSO₄) und filtriert. Nach Abziehen des Lösungsmittels wurden 24 mg des gewünschten Produkts erhalten, das direkt in der nächsten Stufe eingesetzt wurde.
LCMS-Rt (C): 1,09 min
MS (ES⁺, M+H⁺): 330,4

### f) ((2-Chlor-4-methyl-thiophen-3-yl)-(4,5,6,7-tetrahydro-1H-benzoimidazo!-2-yl)-amin-Hydrochlorid

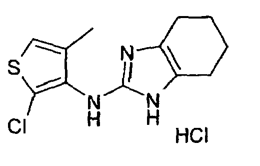

N-(2-Chlor-4-methyl-thiophen-3-yl)-N'-(1,4-dioxa-spiro[4.5]dec-6-yl)-guanidin (24 mg) wurde in 2 N Salzsäure (1 ml) gelöst und 30 min. bei Raumtemperatur gerührt. Anschließend wurde konzentrierte Salzsäure (1 ml) zugegeben und weitere zwei Stunden gerührt. Dann wurde mit wenig Wasser verdünnt und gefriergetrocknet. Der Rückstand wurde mit Toluol versetzt, das im Vakuum abdestilliert wurde. Nachdem dieser Vorgang zwei Mal wiederholt worden war, blieben 22 mg des gewünschten Produkts als Feststoffs zurück.
LCMS-Rt (B): 0,95 min
MS (ES⁺, M+H⁺): 268,07

### Beispiel 49: 2-Chlor-3N-(2-benzimidazolylamino)-4-methylthiophen Benzolsulfonat

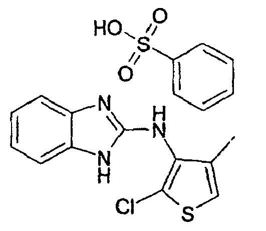

2-Chlor-3N-(2-benzimidazolylamino)-4-methylthiophen (250 mg) wurde in THF (5 ml) gelöst und mit Benzolsulfonsäure (150 mg), gelöst in THF (5 ml), unter Rühren versetzt. Nach 3 h wird das Reaktionsgemisch über Nacht in den Kühlschrank gestellt. Nach Absaugen des Niederschlags und Trockenen bei 75°C im Hochvakuum wurde das gewünschte Produkt erhalten. Farblose Kristalle. Fp: 235 °C

### Beispiel 50: 2-Chlor-3N-(2-benzimidazolylamino)-4-methylthiophen Methansulfonat

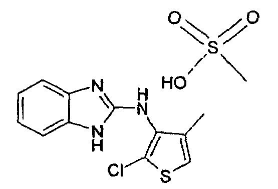

erhielt man analog der in Beispiel 49 beschriebenen Vorschrift aus 2-Chlor-3N-(2-benzimidazolylamino)-4-methylthiophen mit einem Equivalent Methansulfonsäure. Farblose Kristalle. Fp: 227°C

### Beispiel 51: 2-Chlor-3N-(2-benzimidazolylamino)-4-methylthiophen Benzoat

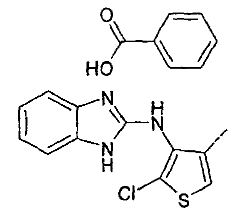

erhielt man analog der in Beispiel 49 beschriebenen Vorschrift aus 2-Chlor-3N-(2-benzimidazolylamino)-4-methylthiophen mit einem Equivalent Benzoesäure. Zur Fällung wurde das Reaktionsgemisch auf die Hälfte eingeengt und anschließend mit Ether (30 ml) versetzt. Farblose Kristalle, Fp: 198°C

### Beispiel 52: 2,4-Dichlor-3N-(2-benzimidazolylamino)thiophen Hydrochlorid

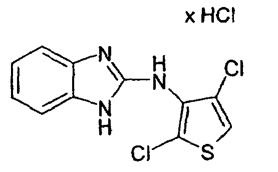

a) 3-Acetylaminothiophen-2-carbonsäuremethylester
   Zu einer Mischung aus 942 g 3-Aminothiophen-2-carbonsäuremethylester und 1000 ml Toluol tropft man unter gleichzeitigem Aufheitzen im Ölbad 567 ml Acetanhydrid, Kocht sodann über 1 ½ Stunden unter Rückflußbedingungen und kühlt sodann im Eisbad auf etwa 0°C ab. Die Kristalle werden abfiltriert , 2 mal mit wenig kaltem Isopropanol und 2 mal mit Diisopropylether gewaschen. Aus dem Filtrat kann durch weiteres Konzentrieren und Kristallisieren 3-Acetylaminothiophen-2-carbonsäuremethylester erhalten werden. Schmp. 93-95 °C.
b) 3-Acetylamino-4,5-dichlorthiophen-2-carbonsäuremethylester
   Zu einer Lösung von 19,9 g 3-Acetylaminothiophen-2-carbonsäuremethylester in 100ml Chloroform tropft man unter magnetischer Rührung bei einer Reaktionstemperatur von 20-30 °C 17,9g Sulfurylchorid SO₂Cl₂. Sodann rührt man weitere 2 Stunden bei 40 °C und kocht weitere 15 Minuten unter Rückflußbedingungen. Nach dem Abdestillieren des Lösungsmittels unter vermindertem Druck versetzt man den Rückstand mit Essigester und filtriert die Krastalle nach dem Stehewnlassen ab. Schmp. 136-138 °C.
c) 3-Acetylamino-4 chlorthiophen-2-carbonsäuremethylester
   Eine Mischung aus 25g 3-Acetylamino-4,5-dichlorthiophen-2-carbonsäuremethylester, ca 10g Triäthylamin, 300ml Methanol und 1g Paladium auf Kohle werden bei Raumtemperatur unter Normaldruck bis zum Stillstand der Wasserstoffaufnahme hydriert. Nach dem Abfiltrieren des Katalysators konzetriert man durch Abdestillieren unter vermindertem Druck bis zur beginnenden Kristallisation , versetzt sodann mit Wasser und filtriert den Feststoff ab. Farblose Kristalle aus Isopropanol. Schmp. 142-147°C.
d) 3-Amino-4-chlorthiophen-2-carbonsäuremethylester
   7 g 3-Acetylamino-4-chlorthiophen-2-carbonsäuremethylester werden in einer Mischung aus 50ml Methanol und 50ml konzentrierter Salzsäure 4 Stunden bei 60°C, 5 Stunden unter rückfließendem Sieden und weitere 3 Tage bei Raumtemperatur gerührt. Man filtriert eventuell entstandenen Niederschlag ab und entfernt etwa 1/3 des Lösungsmittelvolumens durch Destillation unter vermindertem Druck. Nach Zugabe von etwa 100ml Wasser rührt man weitere 15 Minuten bei Raumtemperatur, filtriert die farblosen Kristalle ab und trocknet sie im Luftstrom. Schmp.: 62 - 64 °C.
e) 3-Amino-4-chlorthiophen
   18,02 g 3-Amino-4-chlorthiophen-2-carbonsäuremethylester werden zu einer Lösung aus 11,1 g KOH und 160ml Wasser gegeben, sodann 3 Stunden am aufgesetzten Rückflußkühler gekocht und nach dem Abkühlen zu einer 60°C warmen Lösung aus 15ml konzentrierter Salzsäure 30ml Wasser tropfenweise zugegeben. Dabei kommt es zur deutlichen CO₂ -Entwicklung. Nach weiterem Rühren bei 60°C über ca 40 Minuten läßt man abkühlen, überschichtet sodann mit 50 - 100ml Methyl-tert.butylether und stellt mit konzentrierter Natronlauge alkalisch und extrahiert die wäßrige Phase im Scheidetrichter. Die wäßrige Phase wird 2 weitere Male mit Methyl-tert.butylether extrahiert und die vereinigten organischen Extraktionsphasen ein Mal im Scheidetrichter mit Wasser gewaschen. Man trocknet die organische Phase, destilliert das Lösungsmittel ab und chromatografiert den ölig-amorphen Rückstand auf einer Kieselgelsäule mit einem Gemisch aus 1 Teil Ethylacetat und 1 Teil Toluol.
f) 4-Chlor-3-thienylisothiocyanat
   Zu einer Lösung aus 1,1g 3-Amino-4-chlorthiophen in 20ml wasserfreiem THF gibt man 1,46g Thiocarbonyldiimidazol und rührt eine Stunde bei Raumtemperatur. Das Lösungsmittel wird unter vermindertem Druck abdestilliert, der Rückstand in Ethylacetat gelöst, die organische Phase zwei mal mit Wasser im Scheidetrichter behandelt, getrocknet und das Lösungsmittel erneut im Vakuum abdestilliert. Man erhält das 4-Chlor-3-thienylisothiocyanat als dunkles Öl, das anschließend ohne weitere Reinigungsoperationen weiter umgesetzt wird.
g) N-(2-Aminophenyl)-N'-(4-chlor-3-thienyl)thioharnstoff
   Zu einer Lösung aus 1,4g 4-Chlor-3-thienylisothiocyanat in 40ml wasserfreiem THF gibt man 0,86g 1,2-Diaminobenzol (o-Phenylendiamin), rührt ca 20 Stunden bei Raumtemperatur und destilliert das Lösungsmittel unter vermindertem Druck ab. Der Rückstand wird mit Wasser behandelt, mit Ethylacetat extrahiert, das Lösungsmittel erneut abdestilliert und der Rückstand unter Anwendung einer Mitteldruck-Säulenchromatografie an Kieselgel mit einer 1 : 1 Mischung aus Ethylacetat und Toluol gereinigt. Braungelber Feststoff.
h) 4-Chlor-3N-(2-benzimidazolylamino)thiophen
   Zu einer Lösung von 0,5g N-(2-Aminophenyl)-N'-(4-chlor-3-thienyl)thioharnstoff in 25ml wasserfreiem THF gibt man eine Lösung von 0,169g Natriumhydroxid in 5ml Wasser und sodann eine Lösung von 0,363 p-Toluolsulfonsäurechlorid in 10ml THF. Man rührt 3 Stunden bei Raumtemperatur, destilliert sodann das Lösungsmittel unter vermindertem Druck ab, behandelt den Rückstand mit Wasser und extrahiert mit Ethylacetat. Nach dem Abdestillieren des Lösungsmittels reinigt man das Produkt durch Mitteldruck-Chromatografie an Kieselgel mit einem Gemisch aus 20 Teilen Ethylacetat, 10 Teilen n-Heptan und 3 Teilen Eisessig als Elutionsmittel.
   Ein kleiner Teil des 4-Chlor-3N-(2-benzimidazolylamino)thiophens wurde zur Charakterisierung in Ethylacetat mit etherischer Chlorwasserstoff-Lösung in das 4-Chlor-3N-(2-benzimidazolylamino)thiophen Hydrochlorid übergeführt und charakterisiert. Farblose Kristalle. Fp.: 256 - 260°C.
i) 2,4-Dichlor-3N-(2-benzimidazolylamino)thiophen Hydrochlorid
   Eine Lösung von 0,3g 4-Chlor-3N-(2-benzimidazolylamino)thiophen in 10ml Eisessig versetzt man mit einer Lösung von 0,16g N-Chlorsuccinimid in 5ml Eisessig. Man rührt das Reaktionsgemisch 15 Minuten bei 40°C und etwa 4 Stunden bei Raumtemperatur, destilliert sodann die Essigsäure unter vermindertem Druck ab und behandelt den Rückstand mit Wasser. Nachdem mit Natronlauge alkalisch gestellt wurde, extrahiert man mit Ethylacetat, wäscht mit Wasser, trocknet die organische Phase und destilliert das Lösungsmittel unter vermindertem Druck ab. Der Rückstand wird unter Mitteldruckbedingung durch Säulenchromatografie mit einem Gemisch aus 20 Teilen Ethylacetat, 10 Teilen n-Heptan und 3 Teilen Eisessig als Elutionsmittel gereinigt und anschließend aus Ethylacetat durch Zugabe einer Chlorwasserstofflösung in Diethylether ausgefällt. Farbloses kristallines Produkt. Schmp. 264 - 268°C.

### Beispiel 53:

### 2-Brom-4-chlor-3N-(2-benzimidazolylamino)thiophen Hydrochlorid

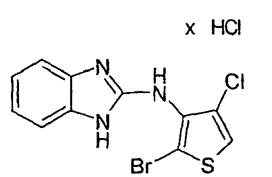

Zu einer Lösung aus 0,5g 4-Chlor-3N-(2-benzimidazolylamino)thiophen in 15ml Eisessig gibt man tropfenweise eine Lösung aus 0,356g N-Bromsuccinimid in 6ml Eisessig und rührt weitere 15 Minuten bei Raumtemperatur. Man destilliert das Lösungsmittel ab, behandelt den Rückstand mit Wasser und stellt mit Natronlauge alkalisch. Nach dem Extrahieren mit Ethylacetat wird die organische Phase mit Wasser gewaschen, getrocknet und unter vermindertem Druck abdestilliert. Man chromatografiert den Rückstand unter Mitteldruckbedingungen an Kieselgel mit einem Gemisch aus 20 Teilen Ethylacetat, 10 Teilen n-Heptan und 3 Teilen Eisessig als Elutionsmittel. Nach Abdestillieren des Lösungsmittels nimmt man den Rückstand in Ethylacetat auf und fällt 2-Brom-4-chlor-3N-(2-benzimidazolylamino)thiophen Hydrochlorid durch Zugabe einer Lösung von Chlorwasserstoffgas in Diethylether. Farbloses kristallines Produkt. Schmp.: 264 - 266°C.

### Beispiel 54: (2,4-Dichlor-thiophen-3-yl)-(5-fluor-1 H-benzoimidazol-2-yl)-amin-Hydrochlorid

### a) 1-(2-Amino-4-fluor-phenyl)-3-(4-chlor-thiophen-3-yl)-thioharnstoff

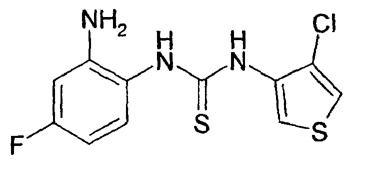

4-Fluor-1,2-phenylendiamin (900 mg) wurde in THF (25 ml) gelöst und 4-Chlor-3-thienylisothiocyanat (Beispiel 52c), gelöst in THF (15 ml), wurde unter Rühren zugetropft. Die Lösung wurde ca. 3 Stunden bei Raumtemperatur gerührt und stand über Nacht. Danach wurde das Reaktionsgemisch eingeengt und der Rückstand mittels präparativer HPLC gereinigt. Die Produkt-enthaltenden Fraktionen wurden vereinigt, vom Acetonitril befreit, basisch gestellt und drei Mal mit Essigester extrahiert. Die organischen Phasen wurden vereinigt, getrocknet (MgSO4) und filtriert. Nach Abziehen des Lösungsmittels wurde 625 mg des gewünschten Produkts erhalten.
LCMS-Rt (F): 1,28 min
MS (ES⁺, M+H⁺): 302,0

### b) (4-Chlor-thiophen-3-yl)-(5-fluor-1 H-benzoimidazol-2-yl)-amin

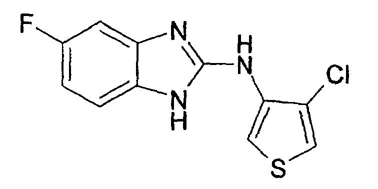

1-(2-Amino-4-fluor-phenyl)-3-(4-chlor-thiophen-3-yl)-thioharnstoff (625 mg) wurde in THF gelöst und mit einer Lösung aus NaOH (0,207 g) in Wasser (9 ml) versetzt. Innerhalb von 5 min. wurde eine Lösung aus p-Toluolsäurechlorid (0,395 g) in THF (10 ml) zugetropft. Nach beendeter Zugabe wurde eine Stunde bei Raumtemperatur gerührt. Zur Aufarbeitung wurde das Reaktionsgemisch mit Wasser und Essigester versetzt und im Scheidetrichter die Phasen getrennt. Die Wasserphase wurde drei Mal mit Essigester extrahiert, die vereinigten Essigester-Phasen mit Magnesiumsulfat getrocknet, mit Kohle geklärt, filtriert und eingeengt. Es wurden 135 mg des gewünschten Produkts erhalten.
LCMS-Rt (F): 0,90 min
MS (ES⁺, M+H⁺): 268,0

### c) (2,4-Dichlor-thiophen-3-yl)-(5-fluor-1H-benzoimidazol-2-yl)-amin-Hydrochlorid

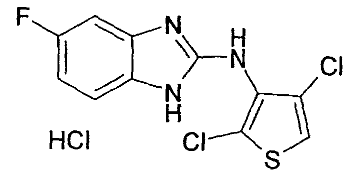

(4-Chlor-thiophen-3-yl)-(5-fluor-1H-benzoimidazol-2-yl)-amin (85 mg) wurde in Eisessig (4 ml) gelöst und bei Raumtemperatur unter kräftigem Rühren langsam mit N-Chlorsuccinimid (42 mg), gelöst in Eisessig (4 ml), versetzt. Nach beendeter Zugabe wurde 45 min. bei Raumtemperatur gerührt, gefolgt von 5 h bei 50°C. Nach Zugabe weiteren N-Chlorsuccinimids (4 mg) wurde noch eine Stunde bei 50°C gerührt. Danach wurde das Reaktionsgemisch mit Toluol (20 ml) versetzt und der Eisessig abdestilliert. Der Rückstand wurde in Essigester gelöst und mit gesättigter Kaliumcarbonat-Lösung eluiert. Die Essigesterphase wurde über Magnesiumsulfat getrocknet, filtriert und eingeengt. Der Rückstand wurde mittels präparativer HPLC gereinigt, die Produkt-enthaltenden Fraktionen vereinigt, vom Acetonitril befreit, basisch gestellt und drei Mal mit Essigester extrahiert. Die organischen Phasen wurden vereinigt, getrocknet (MgSO4) und filtriert. Nach Abziehen des Lösungsmittels wurde der Rückstand mit Wasser und 2 N Salzsäure versetzt und gefriergetrocknet. Es wurden 17 mg des gewünschten Produkts erhalten.
LCMS-Rt (E): 2,65 min
MS (ES⁺, M+H⁺): 301,93

### Beispiel 55: (2-Brom-4-chlor-thiophen-3-yl)-(5-fluor-1H-benzoimidazol-2-yl)-amin-Hydrochlorid

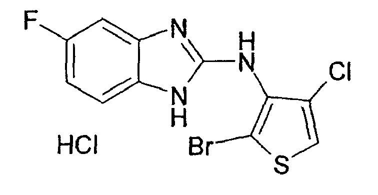

(4-Chlor-thiophen-3-yl)-(5-fluor-1H-benzoimidazol-2-yl)-amin (50 mg, Beispiel 54b) wurde in Eisessig (4 ml) gelöst und bei Raumtemperatur unter kräftigem Rühren langsam mit N-Bromsuccinimid (33 mg), gelöst in Eisessig (4 ml), versetzt. Nach beendeter Zugabe wurde 45 min. bei Raumtemperatur gerührt und dann das Reaktionsgemisch mit Toluol (20 ml) versetzt und der Eisessig abdestilliert. Der Rückstand wurde in Essigester gelöst und mit gesättigter Kaliumcarbonat-Lösung gewaschen. Die Essigesterphase wurde über Magnesiumsulfat getrocknet, filtriert und eingeengt. Der Rückstand wurde mittels präparativer Chromatographie gereinigt, die Produkt-enthaltenden Fraktionen vereinigt, vom Acetonitril befreit, basisch gestellt und drei Mal mit Essigester extrahiert. Die organischen Phasen wurden vereinigt, getrocknet (MgSO4) und filtriert. Nach Abziehen des Lösungsmittels wurde der Rückstand mit Wasser und 2 N Salzsäure versetzt und gefriergetrocknet. Es wurden 27 mg des gewünschten Produkts erhalten.
LCMS-Rt (E): 2,29 min
MS (ES⁺, M+H⁺): 347,87

### Beispiel 56: (2,4-Dichlor-thiophen-3-yl)-(5,6-difluor-1H-benzoimidazol-2-yl)-amin-Hydrochlorid

### a) 1-(2-Amino-4,5-difluor-phenyl)-3-(4-chlor-thiophen-3-yl)-thioharnstoff

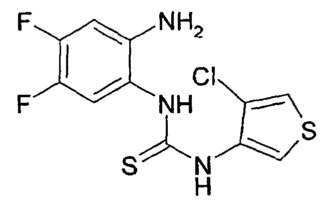

1,2-Diamino-4,5-difluorbenzof (1,02 g) wurde in THF abs. (15 ml) vorlegt und 4-Chlor-3-thienylisothiocyanat (1,25 g, Beispiel 52c), in THF abs. (15 ml) gelöst, zugetropft. Das weitere Vorgehen analog Beispiel 54a) lieferte 773 mg der gewünschten Verbindung.
LCMS-Rt (F): 1,32 min
MS (ES⁺, M+H⁺): 320,0

### b) (4-Chlor-thiophen-3-yl)-(5,6-difluor-1H-benzoimidazol-2-yl)-amin

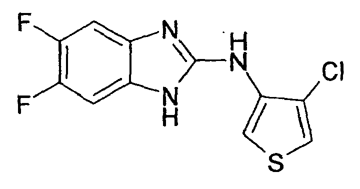

1-(2-Amino-4,5-difluor-phenyl)-3-(4-chlor-thiophen-3-yl)-thiohamstoft (773 mg) wurde in THF (20 ml) vorlegt und mit einer Lösung aus NaOH (240 mg) in Wasser (9 ml) versetzt und dann eine Lösung aus Tosylchlorid (528 mg) in THF (10 ml) zugegeben. Das weitere Vorgehen analog Beispiel 54b) lieferte 275 mg der gewünschten Verbindung.
LCMS-Rt (F): 0,95 min
MS (ES⁺, M+H⁺): 286

### c) (2,4-Dichlor-thiophen-3-yl)-(5,6-difluor-1H-benzoimidazol-2-yl)-amin-Hydrochlorid

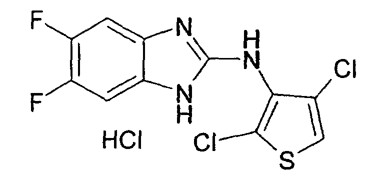

(4-Chlor-thiophen-3-yl)-(5,6-difluor-1 H-benzoimidazol-2-yl)-amin (125 mg) wurde in Essigsäure (8 ml) vorgelegt und eine Lösung aus Cl-Succinimid (59 mg) in Essigsäure (2 ml) zugetropft. Das weitere Vorgehen analog Beispiel 54c) lieferte 58 mg des gewünschten Produkts.
LCMS-Rt (E): 2,97 min
MS (ES⁺, M+H⁺): 319, 88

### Beispiel 57: (2-Brom-4-chlor-thiophen-3-yl)-(5,6-difluor-1H-benzoimidazol-2-yl)-amin-Hydrochlorid

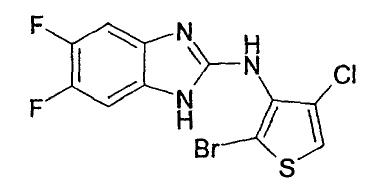

(4-Chlor-thiophen-3-yl)-(5,6-difluor-1 H-benzoimidazol-2-yl)-amin (125 mg, Beispiel 55b) wurde in Eisessig (8 ml) gelöst und bei Raumtemperatur unter kräftigem Rühren langsam mit N-Bromsuccinimid (78 mg), gelöst in Eisessig (2 ml), versetzt.
Das weitere Vorgehen analog Beispiel 55) lieferte 77 mg des gewünschten Produkts. LCMS-Rt (E): 2,39 min
MS (ES⁺, M+H⁺): 365,86

### Beispiel 58: 4-Chlor-3N-(4-methyl-2-benzimidazolyl-amino)thiophen Hydrochlorid

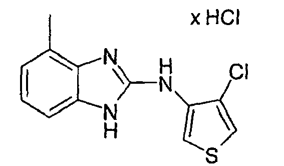

a) N-(2-Amino-3-methylphenyl)-N'-(4-chlor-3-thienyl)thioharnstoff
   erhält man analog der in Beispiel 52g) angegebenen Vorschrift aus 4-Chlor-3-thienylisothiocyanat und 1,2-Diamino-3-methyl-benzol und Anwendung einer Mitteldruck-Säulenchromatografie an Kieselgel mit einer Mischung aus 20 Teilen Essigester, 10 Teilen n-Heptan und 1 Teil Essigester. Braungelber Feststoff, Schmp.: 193-196°C.
b) e) 4-Chlor-3N-(4-methyl-2-benzimidazolylamino)thiophen
   erhält man analog der in Beispiel 52h) angegebenen Vorschrift aus N-(2-Amino-3-methylphenyl)-N'-(4-chlor-3-thienyl)thioharnstoff und Anwendung einer Mitteldruck-Säulenchromatografie an Kieselgel mit einer Mischung aus 10 Teilen Dichlormethan und 1 Teil Methanol als amorphes, schaumiges Produkt. Herstellung des 4-Chlor-3N-(4-methyl-2-benzimidazolylamino)thiophen Hydrochlorids in Ethylacetat mit Chlorwasseerstoffgas-gesättigtem Diethylether. Kristallines Produkt, Schmp. 325-327°C.

### Beispiel 59: 2,4-Dichlor-3N-(4-methyl-2-benzimidazolyl-amino)thiophen Hydrochlorid

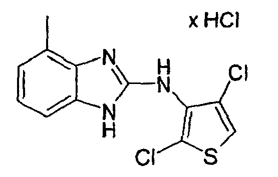

erhält man analog der in Beispiel 52i) angegebenen Vorschrift aus 4-Chlor-3N-(4-methyl-2-benzimidazolylamino)thiophen und N-Chlorsuccinimid in Eisessig bei analoger Aufarbeitung. Kristallines Produkt, Schmp. 296 - 298 °C.

### Beispiel 60: trans-(3aS, 7aS)-4-Chlor-3N-(3a,4,5,6, 7, 7a-hexahydro-1 H-2 benzimidazolyl)-3-thienylamin Hydrochlorid

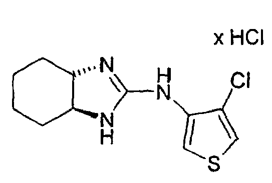

a) trans-S,S-3N-(2-Amino-cyclohexyl)-N'-(4-chlor-3-thienyl)-thioharnstoff erhält man analog der in Beispiel 1, b) angegebenen Reaktion aus 4-Chlor-3-thienyl-isothiocyanat und trans-S,S-1,2-Diaminocyclohexan durch säulenchromatografische Trennung an Kieselgel mit einem Gemisch aus 10 Teilen Ethylacetat, 5 Teilen Dichlormethan, 5-Teilen n-Heptan, 5 Teilen Methanol und 1 Teil Ammoniak (35-37%) als amorphes dunkles Produkt, das ohne weitere Aufreinigung weiterverarbeitet wird.
b) trans-(3aS, 7aS)-4-Chlor-3N-(3a,4,5,6,7,7a-hexahydro-1H-2-benzimidazolyl)-3-thienylamin
   erhält man analog der in Beispiel 52h) angegebenen Vorschrift aus trans-S,S-3N-(2-Amino-cyclohexyl)-N'-(4-chlor-3-thienyl)-thioharnstoff, p-Toluolsulfonsäurechlorid und anschließender Anwendung einer Mitteldruck-Säulenchromatografie an Kieselgel mit einer Mischung aus 10 Teilen Dichlormethan und 1 Teil Methanol als amorphes, schaumiges Produkt. Herstellung des trans-S,S-4-Chlor-3N-(3a,4,5,6,7,7a-hexahydro-1 H-2-benzimidazolyl)-3-thienylamin Hydrochlorids in Ethylacetat und wenig Ethanol mit Chlorwasserstoffgas-gesättigtem Diethylether. Kristallines Produkt, Schmp. 196-200°C.

### Beispiel 61: trans-(3aR,7aR)-4-Chlor-3N-(3a,4,5,6,7,7a-hexahydro-1H-2 benzimidazolyl)-3-thienylamin Hydrochlorid

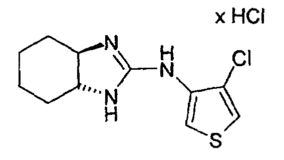

a) trans-(1R,2R)-3N-(2-Amino-cyclohexyl)-N'-(4-chlor-3-thienyl)-thioharnstoff erhält man analog der in Beispiel 1, b) angegebenen Reaktion aus 4-Chlor-3-thienyl-isothiocyanat und trans-(1R,2R)-(-)-1,2-Diaminocyclohexan durch nachfolgende säulenchromatografische Trennung an Kieselgel mit einem Gemisch aus 10 Teilen Ethylacetat, 5-Teilen n-Heptan, 5 Teilen Dichlormethan, 5 Teilen Methanol und 1 Teil Ammoniak (35-37%) als amorphes dunkles Produkt, das ohne weitere Aufreinigung weiterverarbeitet wird.
b) trans-(3aR, 7aR)-4-Chlor-3N-(3a,4,5,6,7,7a-hexahydro-1H-2-benzimidazolyl)-3-thienylamin
   erhält man analog der in Beispiel 52h) angegebenen Vorschrift aus trans-R,R-3N-(2-Amino-cyclohexyl)-N'-(4-chlor-3-thienyl)-thioharnstoff, p-Toluolsulfonsäurechlorid und anschließender Anwendung einer Mitteldruck-Säulenchromatografie an Kieselgel mit einer Mischung aus 10 Teilen Ethylacetat, 5-Teilen n-Heptan, 5 Teilen Dichlormethan, 5 Teilen Methanol und 1 Teil Ammoniak (35-37%) als amorphes, öliges Produkt. Herstellung des trans-R,R-4-Chlor-3N-(3a,4,5,6,7,7a-hexahydro-1H-2-benzimidazolyl)-3-thienylamin Hydrochlorids in Ethylacetat und wenig Ethanol mit Chlorwasserstoffgas-gesättigtem Diethylether. Kristallines Produkt, Schmp. 240-244°C.

### Beispiel 62: cis-4-Chlor-3N-(3a,4,5,6,7,7a-hexahydro-1H-2-benzimidazolyl)-3-thienylamin Hydrochlorid

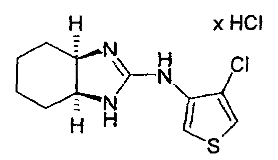

a) cis-3N-(2-Amino-cyclohexyl)-N'-(4-chlor-3-thienyl)-thioharnstoff erhält man analog der in Beispiel 1, b) angegebenen Reaktion aus 4-Chlor-3-thienyl-isothiocyanat und cis-1,2-Diaminocyclohexan durch nachfolgende säulenchromatografische Trennung an Kieselgel mit einem Gemisch aus 10 Teilen Ethylacetat, 5-Teilen n-Heptan, 5 Teilen Dichlormethan, 5 Teilen Methanol und 1 Teil Ammoniak (35-37%) als amorphes dunkles Produkt, das ohne weitere Aufreinigung weiterverarbeitet wird.
b) cis-4-Chlor-3N-(3a,4,5,6,7,7a-hexahydro-1H-2-benzimidazolyl)-3-thienylamin erhält man analog der in Beispiel 52h) angegebenen Vorschrift aus 3N-(cis- 2-Amino-cydohexyt)-N'-(4-chlor-3-thienyl)-thioharnstoff, p-Toluorsulfonsäurechlorid und anschließender Anwendung einer Mitteldruck-Säulenchromatografie an Kieselgel mit einer Mischung aus 10 Teilen Ethylacetat, 5-Teilen n-Heptan, 5 Teilen Dichlormethan, 5 Teilen Methanol und 1 Teil Ammoniak (35-37%) als braunes, öliges Produkt. Herstellung des 4-Chlor-3N-(cis-3a,4,5,6,7,7a-hexahydro-1H-2-benzimidazolyl)-3-thienylamin Hydrochlorids in Ethylacetat und wenig Ethanol mit Chlorwasserstoffgas-gesättigtem Diethylether. Kristallines Produkt, Schmp. 228-231°C.

### Beispiel 63: trans-R,R-2,4-Dichlor-3N-(3a,4,5,6,7,7a-hexahydro-1H-2-benzimidazolyl) 3-thienylamin Hydrochlorid

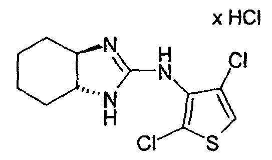

erhält man analog der in Beispiel 52i) angegebenen Vorschrift durch Umsetzung von trans-R,R-4-Chlor-3N-(3a,4,5,6,7,7a-hexahydro-1 H-2-benzimidazolyl)-3-thienylamin mit N-Chlorsuccinimid und analoger Aufarbeitung. Kristallines Produkt. Aufschäumen nter Sublimation ab 165°C.

### Beispiel 64: cis-2,4-Dichlor-3N-(3a,4,5,6,7,7a-hexahydro-1H-2-benzimidazolyl)-3-thienylamin Hydrochlorid

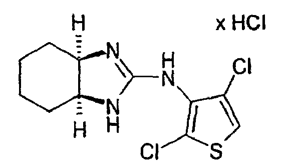

erhält man analog der in Beispiel 52i) angegebenen Vorschrift durch Umsetzung von cis-4-Chlor-3N-(3a,4,5,6,7,7a-hexahydro-1H-2-benzimidazolyl)-3-thienylamin mit N-Chlorsuccinimid und analoger Aufarbeitung. Kristallines Produkt. Schmp. 270-274°C.

### Beispiel 65: 4-Chlor-3N-(4-chlor-2-benzimidazolylamino)thiophen Hydrochlorid

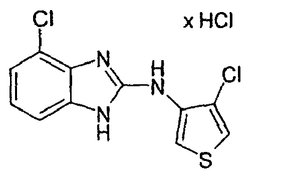

a) 1-N-(2-Amino-3-chlorpheny)-3-N-(4-chlor-3-thienyl)thioharnstoff erhält man aus 3-Chlor-1,2-diaminobenzol und 4-Chlor-3-thienylisothiocyanat analog der in Beispiel 1b) beschriebenen Vorschrift und Kristallisieren unter Diisopropylether als Feststoff mit zwei Schmelzpunkten: Schmp.1: 152-155°C unter Rekristallisation; Schmp.2: >310°C
b) 4-Chlor-3N-(4-chlor-2-benzimidazolylamino)thiophen erhält man analog der in Beispiel 52h) beschriebenen Vorschrift durch Umsetzung von 1-N-(2-Amino-3-chlorpheny)-3-N-(4-chlor-3-thienyl)thioharnstoff mit p-Toluolsufonsäurechlorid und Säulenchromatografie an Kieselgel mit einem Gemisch aus 3 Teilen Toluol und einem Teil Essigester als Elutionsmittel und anschließendem Abdestillieren des Lösungsmittels untervermindertem Druck als schaumiges amorphes Produkt, das weiter in das Hydrochlorid umgewandelt wird.
c) 4-Chlor-3N-(4-chtor-2-benzimidazolylamino)thiophen Hydrochlorid erhält man durch Behandeln einer Lösung von 4-Chlor-3N-(4-chlor-2-benzimidazolylamino)thiophen in Ethylacetat mit einer mit Chlorwasserstoffgas gesättigten Lösung in Diethylether als kristallinen Niederschlag. Schmp. 276 - 280 °C.

### Beispiel 66: 2,4-Dichlor-3N-(4-chlor-2-benzimidazolyl-amino)thiophen Hydrochlorid

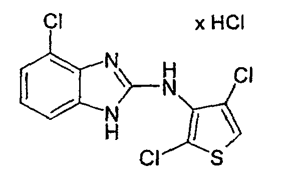

erhält man analog der in Beispiel 52i) angegebenen Vorschrift aus 4-Chlor-3N-(4-methyl-2-benzimidazolylamino)thiophen und N-Chlorsuccinimid in Eisessig bei analoger Aufarbeitung. Kristallines Produkt, Schmp. 294 - 297 °C.

Analog der in den Ausführungsbeispielen aufgeführten Verbindungen können die folgenden Thiophenderivate hergestellt werden:
2-Brom-4-chlor-3N-(4-methyl-2-benzimidazolyl-amino)thiophen Hydrochlorid, Schmp 284-286°C,
2-Brom-4-chlor-3N-(4-chlor-2-benzimidazolyl-amino)thiophen Hydrochlorid, Schmp 304-306°C,
trans-R,R-2-Brom-4-chlor-3N-(3a,4,5,6,7,7a-hexahydro-1H-2-benzimidazolyl)-3-thienylamin Hydrochlorid, Zersetzungspunkt: 215°C
trans-(3aS,7aS)-2-Brom-4-chlor-3N-(3a,4,5,6,7,7a-hexahydro-1H-2-benzimidazolyl)-3-thienylamin Hydrochlorid 243-254°C
2,4-Dibrom-3N-(2-benzimidazolyl)-3-thienylamin Hydrochlorid,
2,4- Dimethyl -3N-(2-benzimidazolyl)-3-thienylamin Hydrochlorid,
2,4- Dimethyl -3N-(4-methyl-2-benzimidazolyl)-3-thienylamin Hydrochlorid,
2,4- Dimethyl -3N-(5-fluor-2-benzimidazolyl)-3-thienylamin Hydrochlorid,
2- Chlor -3N-(4-butoxy-2-benzimidazolyl)-4-methyl-3-thienylamin Hydrochlorid,
2-Chlor-3N-(4-trifluormethyl-2-benzimidazolyl)-4-methyl-3-thienylamin Hydrochlorid,
2-Chlor-3N-(4-methylsulfonyl-2-benzimidazolyl)-4-methyl-3-thienylamin Hydrochlorid.

### Pharmakologische Daten:

### Testbeschreibung:

In diesem Test wurde die Erholung des intrazellulären pHs (pHᵢ) nach einer Ansäuerung ermittelt, die bei funktionsfähigem NHE auch unter bicarbonatfreien Bedingungen einsetzt. Dazu wurde der pHᵢ mit dem pH-sensitiven Fluoreszenzfarbstoff BCECF (Calbiochem, eingesetzt wird die Vorstufe BCECF-AM) bestimmt. Die Zellen wurden zunächst mit BCECF beladen. Die BCECF-Fluoreszenz wurde in einem "Ratio Fluorescence Spectrometer" (Photon Technology International, South Brunswick, N.J., USA) bei Anregungswellenlängen von 505 und 440 nm und einer Emissionswellenlänge von 535 nm bestimmt und mittels Kalibrierungskurven in den pHᵢ umgerechnet. Die Zellen wurden bereits bei der BCECF-Beladung in NH₄Cl-Puffer (pH 7,4) inkubiert (NH₄Cl-Puffer: 115 mM NaCl, 20 mM NH₄Cl, 5 mM KCl, 1 mM CaCl₂, 1 mM MgSO₄, 20 mM Hepes, 5 mM Glucose, 1 mg/ml BSA; mit 1 M NaOH wird ein pH von 7,4 eingestellt). Die intrazelluläre Ansäuerung wurde durch Zugabe von 975 µl eines NH₄Cl -freien Puffers (s. u.) zu 25 µl Aliquots der in NH₄Cl - Puffer inkubierten Zellen induziert. Die nachfolgende Geschwindigkeit der pH-Erholung wurde bei NHE1 zwei Minuten, bei NHE2 fünf Minuten und bei NHE3 drei Minuten registriert. Für die Berechnung der inhibitorischen Potenz der getesteten Substanzen wurden die Zellen zunächst in Puffern untersucht, bei denen eine vollständige bzw. überhaupt keine pH-Erholung stattfand. Zur vollständigen pH-Erholung (100%) wurden die Zellen in Na⁺-haltigem Puffer inkubiert (133,8 mM NaCl, 4,7 mM KCI, 1,25 mM CaCl₂, 1,25 mM MgCl₂, 0,97 mM Na₂HPO₄, 0,23 mM NaH₂PO₄, 5 mM Hepes, 5 mM Glucose, mit 1 M NaOH wird ein pH von 7,0 eingestellt). Für die Bestimmung des 0%-Wertes wurden die Zellen in einem Na⁺-freien Puffer inkubiert (133,8 mM Cholinchlorid, 4,7 mM KCI, 1,25 mM CaCl₂, 1,25 mM MgCl₂, 0,97 mM K₂HPO₄, 0,23 mM KH₂PO₄, 5 mM Hepes, 5 mM Glucose, mit 1 M KOH wird ein pH von 7,0 eingestellt). Die zu testenden Substanzen wurden in dem Na⁺-haltigem Puffer angesetzt. Die Erholung des intrazellulären pH's bei jeder getesteten Konzentration einer Substanz wurde in Prozent der maximalen Erholung ausgedrückt. Aus den Prozentwerten der pH-Erholung wurde mittels des Programms Sigma-Plot der IC₅₀₋Wert der jeweiligen Substanz für die einzelnen NHE-Subtypen berechnet.

### Ergebnisse:

| Beispiel | IC₅₀ [µM] |
|---|---|
| 16 | 0,27 (rNHE3) |
| trans-R,R-2-Chlor-3N-(3a,4,5,6,7,7a-Hexahydro-1 H-2-benzimidazolyl)-4-methyl-3-thienylamin Hydrochlorid | |
| 12 | 0,12 (rNHE3) |
| 2-Chlor-3N-(2-benzimidazolyl)-4-methyl-3-thienylamin Hydrochlorid | |
| 54: | 0,22 (hNHE3) |
| 2-Brom-4-chlor-3N-(2-benzimidazolylamino)thiophen Hydrochlorid | |
| 53: | 0,14 (hNHE3) |
| 2,4-Dichlor-3N-(2-benzimidazolylamino)thiophen Hydrochlorid | |
| 56: | 0,22 (hNHE3) |
| 2,4-Dichlor-3N-(4-methyl-2-benzimidazolylamino)thiophen Hydrochlorid | |
| 60: | 0,19 (hNHE3) |
| trans-R,R-2,4-Dichlor-3N-(3a,4,5,6,7,7a-hexahydro-1H-2-benzimidazolyl)-3-thienylamin Hydrochlorid | |
| 63: | 0,54 (hNHE3) |
| 2,4-Dichlor-3N-(4-chlor-2-benzimidazolyl-amino)thiophen Hydrochlorid | |
| 38: | |
| 2-Chlor-3N-(4-hydroxy-2-benzimidazolyl-amino)-4-methylthiophen Hydrochlorid | 0,84 (hNHE3) |
| 18: | 0,12 (hNHE3) |
| 2-Brom-3N-(2-benzimidazolyl)-4-methyl-3-thienylamin Hydrochlorid | |
| 19: | 0,56 (hNHE3) |
| trans-R,R-2-Brom-3N-(3a,4,5,6,7,7a-Hexahydro-1H-2-benzimidazolyl)-4-methyl-3-thienylamin Hydrochlorid | |
| 2: | 0,62 (hNHE3) |
| 2-Chlor-3N-(5-fluor-2-benzimidazolyl)-4-methyl-3-thienylamin Hydrochlorid | |
| 44: | 1,59 (hNHE3) |
| (1 H-Benzimidazol-2-yl)-(2-chlor-4-methyl-thiophen-3-yl)-methyl-amin | |

## Patentansprüche

1. Verbindungen der Formel I, 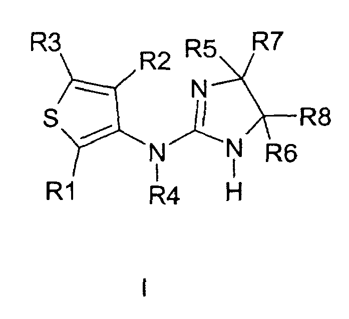
worin bedeuten:
R1 und R2 unabhängig voneinander H, F, CI, Br, I, CN, NO₂, -(X)ₙ-C_{q}H_{2q}-Z, Cycloalkyl mit 3, 4, 5 oder 6 C-Atomen, Alkenyl mit 2, 3 oder 4 C-Atomen, Alkenylalkyl mit 3 oder 4 C-Atomen, Ethinyl oder Alkylalkinyl mit 3 oder 4 C-Atomen;
n Null oder 1;
X Sauerstoff, NH, N-CH₃ oder S(O)ₖ;
k Null, 1 oder 2;
q Null, 1, 2, 3, 4, 5 oder 6;
Z Wasserstoff oder CₘF_{2m+1;}
m Null, 1, 2, 3 oder 4;
R3 Wasserstoff, Methyl, F, Cl, Br, I, CN, S(O)ₖ-CH₃, -NO₂ oder -O-CH₃;
k Null, 1 oder 2;
R4 Wasserstoff, Cycloalkyl mit 3, 4, 5, oder 6 C-Atomen, Alkenyl mit 2, 3 oder 4 C-Atomen, Alkenylalkyl mit 3 oder 4 C-Atomen, Ethinyl oder Alkylalkinyl mit 3 oder 4 C-Atomen oder -CᵣH₂ᵣ- Y;
r Null, 1, 2, 3 oder 4;
Y Wasserstoff oder Trifluormethyl;
R5 und R6 Wasserstoff oder gemeinsam eine Bindung;
R7 und R8 unabhängig voneinander (C₃-C₅)-Alkyl-, (C₂-C₅)-Alkenyl, (C₂-C₅)-Alkinyl, (C₃-C₆)-Cycloalkyl oder (C₄-C₆)-Cycloalkenyl
oder
R7 und R8 gemeinsam eine Alkylenkette bestehend aus 3 bis 8 C-Atomen; wobei deren Wasserstoffatome nicht, teilweise oder vollständig durch Fluoratome ersetzt sein können;
oder
R7 und R8 gemeinsam einen Rest 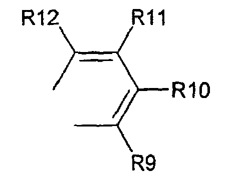
wobei R5 und R6 gemeinsam eine Bindung bilden;
R10 und R11 unabhängig voneinander Wasserstoff, Fluor, Chlor, Brom, Methyl, CN, OH, -O-CH₃, NO₂, NH₂ oder -CF₃;
R9 und R12 Wasserstoff oder F;
oder
einer der Substituenten R9 und R12 Wasserstoff;
und der andere F, Cl, Br, I, CN, NO₂, COOH, CO-NR13R14, SO₂₋NR13R14, Alkenyl mit 2, 3 oder 4 C-Atomen, Alkenylalkyl mit 3 oder 4 C-Atomen, Ethinyl, Alkylalkinyl mit 3 oder 4 C-Atomen oder -(X)ₙ₋CqH_{2q}-Z;
R13 und R14 gleich oder verschieden Wasserstoff oder Alkyl mit 1, 2, 3 oder 4 C-Atomen;
oder
R13 und R14 bilden zusammen mit dem Stickstoff, an den sie gebunden sind einen gesättigten 5-, 6- oder 7-gliedrigen Ring;
n Null oder 1;
X Sauerstoff, NH, N-CH₃ oder S(O)ₖ;
k Null, 1 oder 2;
q Null, 1, 2, 3, 4, 5 oder 6;
und
Z Wasserstoff oder CₘF₂ₘ₊₁;
m Null, 1, 2, 3 oder 4;
sowie deren pharmazeutisch verträgliche Salze, sowie deren Trifluoressigsäuresalze.

2. Verbindungen nach Anspruch 1, in denen bedeuten:
R1 und R2 unabhängig voneinander H, F, Cl, Br, CH₃, CF₃ ,SO₂CH₃ oder SO₂NH₂; wobei jedoch höchstens einer der Substituenten R1 und R2 Wasserstoff ist;
R3 Wasserstoff, F oder Cl;
R4 Wasserstoff, Alkyl mit 1, 2, 3 oder 4 C-Atomen, oder Cyclopropyl;
R5 und R6 Wasserstoff oder gemeinsam eine Bindung;
R7 und R8 gemeinsam eine Alkylenkette bestehend aus 3, 4, 5, 6, 7 oder 8 C-Atomen;
oder
R7 und R8 gemeinsam einen Rest 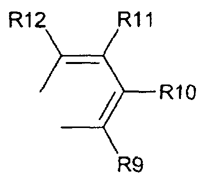
wobei R5 und R6 gemeinsam eine Bindung bilden;
R10 und R11 unabhängig voneinander Wasserstoff, OH, Fluor oder Chlor;
R9 und R12 Wasserstoff;
oder
einer der Substituenten R9 und R12 Wasserstoff; und der andere F, Cl, Br, CN, COOH, CO-NR13R14, SO₂-NR13R14 oder -(X)ₙ - CqH₂q-Z;
R13 und R14 gleich oder verschieden Wasserstoff oder Methyl;
n Null oder 1;
X Sauerstoff, NH, N-CH₃ oder S(O)ₖ;
k Null, 1 oder 2;
q Null, 1, 2, 3 oder 4;
Z Wasserstoff oder CF₃;
sowie deren pharmazeutisch verträgliche Salze, sowie deren Trifluoressigsäuresalze.

3. Verbindungen nach Anspruch 1 und/oder 2, in denen bedeuten:
R1 und R2 unabhängig voneinander F, Cl, Br, CH₃ oder CF₃;
R3 Wasserstoff;
R4 Wasserstoff, Methyl oder Ethyl;
R5 und R6 Wasserstoff oder gemeinsam eine Bindung;
R7 und R8 gemeinsam eine Alkylenkette bestehend aus 3, 4, 5, 6, 7 oder 8 C-Atomen;
oder
R7 und R8 gemeinsam einen Rest 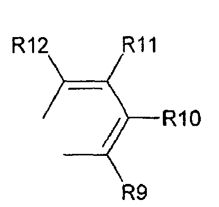
wobei R5 und R6 gemeinsam eine Bindung bilden;
R10 und R11 unabhängig voneinander Wasserstoff, OH oder Fluor;
R9 und R12 Wasserstoff;
oder
einer der Substituenten R9 und R12 Wasserstoff;
und der andere F, CI, Br oder -(X)ₙ - C_{q}H_{2q}-Z;
n Null oder 1;
X Sauerstoff, NH, N-CH₃ oder S(O)ₖ;
k Null, 1 oder 2;
q Null oder 1,
Z Wasserstoff oder CF₃;
sowie deren pharmazeutisch verträgliche Salze, sowie deren Trifluoressigsäuresalze.

4. Verbindungen der Formel I nach einem oder mehreren Ansprüchen 1,2 und 3, ausgewählt aus der Gruppe:
trans-R,R-2-Chlor-3N-(3a,4,5,6,7,7a-hexahydro-1H-2-benzimidazolyl)-4-methyl-3-thienylamin,
trans-R,R-2-Brom-3N-(3a,4,5,6,7,7a-hexahydro-1H-2-benzimidazolyl)-4-methyl-3-thienylamin,
2-Chlor-3N-(2-benzimidazolyl)-4-methyl-3-thienylamin,
2-Brom-3N-(2-benzimidazolyl)-4-methyl-3-thienylamin,
2-Chlor-3N-(4-methyl-2-benzimidazolyl)-4-methyl-3-thienylamin,
2-Chlor-3N-(5-fluor-2-benzimidazolyl)-4-methyl-3-thienylamin,
2-Chlor-3N-(4-chlor-2-benzimidazoly-amino)-4-methylthiophen,
2-Brom-3N-(4-chlor-2-benzimidazolyl-amino)-4-methylthiophen,
2-Brom-3N-(4-fluor-2-benzimidazolyl-amino)-4-methylthiophen,
2-Chlor-3N-(4-fluor-2-benzimidazolyl-amino)-4-methylthiophen,
2-Chlor-3N-(4-hydroxy-2-benzimidazolyl-amino)-4-methylthiophen,
(1 H-Benzimidazol-2-yl)-(2-chlor-4-methyl-thiophen-3-yl)-methyl-amin,
(2-Brom-4-methyl-thiophen-3-yl)-(5-fluor-1H-benzimidazot-2-yl)-amin,
2-Chlor-3N-(2-benzimidazolylamino)-4-methylthiophen,
2,4-Dichlor-3N-(2-benzimidazolylamino)thiophen,
2-Brom-4-chlor-3N-(2-benzimidazolylamino)thiophen,
2,4-Dichlor-3N-(4-methyl-2-benzimidazolyl-amino)thiophen,
trans-R,R-2,4-Dichlor-3N-(3a,4,5,6,7,7a-hexahydro-1 H-2-benzimidazolyl)-3-thienylamin,
und
2,4-Dichlor-3N-(4-chlor-2-benzimidazolyl-amino)thiophen,
sowie deren pharmazeutisch verträgliche Salze.

5. Verbindungen der Formel I und/oder deren pharmazeutisch verträgliche Salze nach einem oder mehreren der Ansprüche der Ansprüche 1 bis 4 zur Verwendung als Medikament.

6. Verwendung einer Verbindung der Formel I und/oder deren pharmazeutisch verträgliche Salze nach einem oder mehreren der Ansprüche der Ansprüche 1 bis 4 zur Herstellung eines Medikaments zur Behandlung oder Prophylaxe von Störungen des Atemantriebs, insbesondere von schlafbedingten Atemstörungen wie Schlafapnoen, oder des Schnarchens, zur Behandlung oder zur Prophylaxe von akuten und chronischen Nierenerkrankungen, besonders des akuten Nierenversagens oder des chronischen Nierenversagens, oder von Störungen der Darmfunktion, zur Behandlung oder Prophylaxe des Bluthochdrucks, insbesondere der essentiellen Hypertonie, von Erkrankungen des Zentralnervensystems, insbesondere von Erkrankungen, die durch ZNS-Übererregbarkeit resultieren, wie Epilepsie oder zentral ausgelöste Krämpfe, und von Erkrankungen des Zentralnervensystems, insbesondere von Angstzuständen, Depressionen und Psychosen, zur Behandlung oder Prophylaxe von akuten und chronischen Schäden, Erkrankungen und indirekte Folgeerkrankungen von Organen oder Geweben, die durch Ischämie- oder durch Reperfusionsereignisse verursacht werden, von Arrhythmien, von Atherosklerose, von Hypercholesterinämie, von Krankheiten, bei denen die Zellproliferation eine primäre oder sekundäre Ursache darstellt, von Krebs, von Prostata-Hypertrophie bzw. Prostata-Hyperplasie, von fibrotischen Erkrankungen innerer Organe, von Herzinsuffizienz oder von Congestive Heart Failure, von Thrombosen, von Störungen der Gallenfunktion, des Befalls durch Ektoparasiten, von Erkrankungen infolge endothelialer Dysfunktion, von claudicatio intermittens, von Protozoen-Erkrankungen, insbesondere der Malaria, zur Behandlung von Schockzuständen, der Zuckerkrankheit und diabetischer Spätschäden, akuter oder chronischer inflammatorischer Erkrankungen oder zur Herstellung eines Medikaments zur Konservierung und Lagerung von Transplantaten für chirurgische Maßnahmen, zur Herstellung eines Medikaments für den Einsatz bei chirurgischen Operationen und Organtransplantationen, zur Herstellung eines Medikaments zur Verhinderung altersbedingter Gewebsveränderung, zur Gesunderhaltung und Lebensverlängerung, zur Verminderung cytotoxischer Effekte oder zur Verwendung als Diagnostika.

7. Verwendung einer Verbindung der Formel I und/oder deren pharmazeutisch verträgliche Salze nach einem oder mehreren der Ansprüche der Ansprüche 1 bis 4 in Kombination mit anderen Arzneimitteln oder Wirkstoffen zur Herstellung eines Medikaments zur Behandlung oder Prophylaxe von Störungen des Atemantriebs, insbesondere von schlafbedingten Atemstörungen wie Schlafapnoen, oder des Schnarchens, zur Behandlung oder zur Prophylaxe von akuten und chronischen Nierenerkrankungen, besonders des akuten Nierenversagens oder des chronischen Nierenversagens, oder von Störungen der Darmfunktion, zur Behandlung oder Prophylaxe des Bluthochdrucks, insbesondere der essentiellen Hypertonie, von Erkrankungen des Zentralnervensystems, insbesondere von Erkrankungen, die durch ZNS-Übererregbarkeit resultieren, wie Epilepsie oder zentral ausgelöste Krämpfe, und von Erkrankungen des Zentralnervensystems, insbesondere von Angstzuständen, Depressionen und Psychosen, zur Behandlung oder Prophylaxe von akuten und chronischen Schäden, Erkrankungen und indirekte Folgeerkrankungen von Organen oder Geweben, die durch Ischämie- oder durch Reperfusionsereignisse verursacht werden, von Arrhythmien, von Atherosklerose, von Hypercholesterinämie, von Krankheiten, bei denen die Zellproliferation eine primäre oder sekundäre Ursache darstellt, von Krebs, von Prostata-Hypertrophie bzw. Prostata-Hyperplasie, von fibrotischen Erkrankungen innerer Organe, von Herzinsuffizienz oder von Congestive Heart Failure, von Thrombosen, von Störungen der Gallenfunktion, des Befalls durch Ektoparasiten, von Erkrankungen infolge endothelialer Dysfunktion, von claudicatio intermittens, von Protozoen-Erkrankungen, insbesondere der Malaria, zur Behandlung von Schockzuständen, der Zuckerkrankheit und diabetischer Spätschäden, akuter öder chronischer inflammatorischer Erkrankungen oder zur Herstellung eines Medikaments zur Konservierung und Lagerung von Transplantaten für chirurgische Maßnahmen, zur Herstellung eines Medikaments für den Einsatz bei chirurgischen Operationen und Organtransplantationen, zur Herstellung eines Medikaments zur Verhinderung altersbedingter Gewebsveränderung, zur Gesunderhaltung und Lebensverlängerung oder zur Verminderung cytotoxischer Effekte.

8. Verwendung einer Verbindung der Formel I und/oder deren pharmazeutisch verträgliche Salze nach einem oder mehreren der Ansprüche der Ansprüche 1 bis 4 allein oder in Kombination mit anderen Arzneimitteln oder Wirkstoffen zur Herstellung eines Medikaments zur Behandlung oder Prophylaxe von Störungen des Atemantriebs, insbesondere von Schlaf-bedingten Atemstörungen wie Schlafapnoen.

9. Verwendung einer Verbindung der Formel I und/oder deren pharmazeutisch verträgliche Salze nach einem oder mehreren der Ansprüche der Ansprüche 1 bis 4 allein oder in Kombination mit anderen Arzneimitteln oder Wirkstoffen zur Herstellung eines Medikaments zur Behandlung oder Prophylaxe des Schnarchens.

10. Verwendung einer Verbindung der Formel I und/oder deren pharmazeutisch verträgliche Salze nach einem oder mehreren der Ansprüche der Ansprüche 1 bis 4 allein oder in Kombination mit anderen Arzneimitteln oder Wirkstoffen zur Herstellung eines Medikaments zur Behandlung oder zur Prophylaxe von akuten und chronischen Nierenerkrankungen, besonders des akuten Nierenversagens oder des chronischen Nierenversagens.

11. Verwendung einer Verbindung der Formel I und/oder deren pharmazeutisch verträgliche Salze nach einem oder mehreren der Ansprüche der Ansprüche 1 bis 4 allein oder in Kombination mit anderen Arzneimitteln oder Wirkstoffen zur Herstellung eines Medikaments zur Behandlung oder Prophylaxe von Störungen der Darmfunktion.

12. Heilmittel für die humane, veterinäre oder phytoprotektive Anwendung enthaltend eine wirksame Menge einer Verbindung der Formel I und/oder deren pharmazeutisch verträgliche Salze nach einem oder mehreren der Ansprüche der Ansprüche 1 bis 4, zusammen mit pharmazeutisch annehmbaren Träger- und Zusatzstoffen.

13. Heilmittel für die humane, veterinäre oder phytoprotektive Anwendung enthaltend eine wirksame Menge einer Verbindung der Formel I und/oder deren pharmazeutisch verträgliche Salze nach einem oder mehreren der Ansprüche der Ansprüche 1 bis 4, zusammen mit pharmazeutisch annehmbaren Träger- und Zusatzstoffen, in Kombination mit anderen pharmakologischen Wirkstoffen oder Arzneimitteln.

## Claims

1. A compound of the formula I, 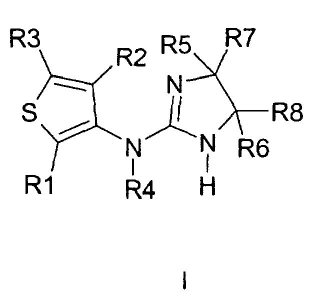
in which:
R1 and R2 independently of one another are H, F, CI, Br, I, CN, NO₂, -(X)ₙ-C_{q}H_{2q}-Z, cycloalkyl having 3, 4, 5 or 6 carbon atoms, alkenyl having 2, 3 or 4 carbon atoms, alkenylalkyl having 3 or 4 carbon atoms, ethynyl or alkylalkynyl having 3 or 4 carbon atoms;
n is zero or 1;
X is oxygen, NH, N-CH₃ or S(O)ₖ;
k is zero, 1 or 2;
q is zero, 1, 2, 3, 4, 5 or 6;
Z is hydrogen or CₘF₂ₘ₊₁;
m is zero, 1, 2, 3 or 4;
R3 is hydrogen, methyl, F, Cl, Br, I, CN, S(O)ₖ-CH₃, -NO₂ or -O-CH₃;
k is zero, 1 or 2;
R4 is hydrogen, cycloalkyl having 3, 4, 5, or 6 carbon atoms, alkenyl having 2, 3 or 4 carbon atoms, alkenylalkyl having 3 or 4 carbon atoms, ethynyl or alkylalkynyl having 3 or 4 carbon atoms or -CᵣH₂ᵣ-Y;
r is zero, 1, 2, 3 or 4;
Y is hydrogen or trifluoromethyl;
R5 and R6 are hydrogen or together are a bond;
R7 and R8 independently of one another are (C₃-C₅)-alkyl, (C₂-C₅)-alkenyl, (C₂-C₅)-alkynyl, (C₃-C₆)-cycloalkyl or (C₄-C₆)-cycloalkenyl
or
R7 and R8 together are an alkylene chain comprising 3 to 8 carbon atoms; where none, some or all of their hydrogen atoms may be replaced by fluorine atoms;
or
R7 and R8 together are a radical 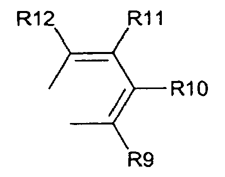
where R5 and R6 together form a bond;
R10 and R11 independently of one another are hydrogen, fluorine, chlorine, bromine, methyl, CN, OH, -O-CH₃, NO₂, NH₂ or -CF₃;
R9 and R12 are hydrogen or F;
or
one of the substituents R9 and R12 is hydrogen;
and the other is F, CI, Br, I, CN, NO₂, COOH, CO-NR13R14, SO₂-NR13R14, alkenyl having 2, 3 or 4 carbon atoms, alkenylalkyl having 3 or 4 carbon atoms, ethynyl, alkylalkynyl having 3 or 4 carbon atoms or -(X)ₙ - C_{q}H_{2q}-Z;
R13 and R14 are identical or different hydrogen or alkyl having 1, 2, 3 or 4 carbon atoms;
or
R13 and R14 together with the nitrogen to which they are attached form a saturated 5-, 6- or 7-membered ring;
n is zero or 1;
X is oxygen, NH, N-CH₃ or S(O)ₖ;
k is zero, 1 or 2;
q is zero, 1, 2, 3, 4, 5 or 6;
and
Z is hydrogen or CₘF_{2m+1;}
m is zero, 1, 2, 3 or 4;
and its pharmaceutically acceptable salts, and its trifluoroacetic acid salts.

2. A compound as claimed in claim 1, in which:
R1 and R2 independently of one another are H, F, CI, Br, CH₃, CF₃, SO₂CH₃ or SO₂NH₂;
but where at most one of the substituents R1 and R2 is hydrogen;
R3 is hydrogen, F or CI;
R4 is hydrogen, alkyl having 1, 2, 3 or 4 carbon atoms, or cyclopropyl;
R5 and R6 are hydrogen or together are a bond;
R7 and R8 together are an alkylene chain comprising 3, 4, 5, 6, 7 or 8 carbon atoms;
or
R7 and R8 together are a radical 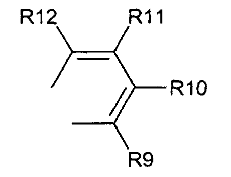
where R5 and R6 together form a bond;
R10 and R11 independently of one another are hydrogen, OH, fluorine or chlorine;
R9 and R12 are hydrogen;
or
one of the substituents R9 and R12 is hydrogen;
and the other is F, CI, Br, CN, COOH, CO-NR13R14, SO₂-NR13R14 or -(X)ₙ - C_{q}H_{2q}-Z;
R13 and R14 are identical or different hydrogen or methyl;
n is zero or 1;
X is oxygen, NH, N-CH₃ or S(O)ₖ;
k is zero, 1 or 2;
q
is zero, 1, 2, 3 or 4;
Z is hydrogen or CF₃;
and its pharmaceutically acceptable salts, and its trifluoroacetic acid salts.

3. A compound as claimed in claim 1 and/or 2, in which:
R1 and R2 independently of one another are F, Cl, Br, CH₃ or CF₃;
R3 is hydrogen;
R4 is hydrogen, methyl or ethyl;
R5 and R6 are hydrogen or together are a bond;
R7 and R8 together are an alkylene chain comprising 3, 4, 5, 6, 7 or 8 carbon atoms;
or
R7 and R8 together are a radical 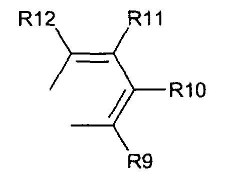
where R5 and R6 together form a bond;
R10 and R11 independently of one another are hydrogen, OH or fluorine;
R9 and R12 are hydrogen;
or
one of the substituents R9 and R12 is hydrogen;
and the other is F, Cl, Br or -(X)ₙ - CqH₂q-Z;
n is zero or 1;
X is oxygen, NH, N-CH₃ or S(O)ₖ;
k is zero, 1 or 2;
q is zero or 1;
Z is hydrogen or CF₃;
and its pharmaceutically acceptable salts, and its trifluoroacetic acid salts.

4. A compound of the formula I as claimed in one or more of claims 1, 2 and 3, selected from the group consisting of:
trans-R,R-2-chlorine-3N-(3a,4,5,6,7,7a-hexahydro-1H-2-benzimidazolyl)-4-methyl-3-thienylamine,
trans-R,R-2-bromo-3N-(3a,4,5,6,7,7a-hexahydro-1H-2-benzimidazolyl)-4-methyl-3-thienylamine,
2-chloro-3N-(2-benzimidazolyl)-4-methyl-3-thienylamine,
2-bromo-3N-(2-benzimidazolyl)-4-methyl-3-thienylamine,
2-chloro-3N-(4-methyl-2-benzimidazolyl)-4-methyl-3-thienylamine,
2-chloro-3N-(5-fluoro-2-benzimidazolyl)-4-methyl-3-thienylamine,
2-chloro-3N-(4-chloro-2-benzimidazolylamino)-4-methylthiophene,
2-bromo-3N-(4-chloro-2-benzimidazolylamino)-4-methylthiophene,
2-bromo-3N-(4-fluoro-2-benzimidazolylamino)-4-methylthiophene,
2-chloro-3N-(4-fluoro-2-benzimidazolylamino)-4-methylthiophene,
2-chloro-3N-(4-hydroxy-2-benzimidazolylamino)-4-methylthiophene,
(1 H-benzimidazol-2-yl)-(2-chloro-4-methylthiophen-3-yl)-methylamine,
(2-bromo-4-methylthiophen-3-yl)-(5-fluoro-1H-benzimidazol-2-yl)-amine,
2-chloro-3N-(2-benzimidazolylamino)-4-methylthiophene,
2,4-dichloro-3N-(2-benzimidazolylamino)thiophene,
2-bromo-4-chloro-3N-(2-benzimidazolylamino)thiophene,
2,4-dichloro-3N-(4-methyl-2-benzimidazolylamino)thiophene,
trans-R,R-2,4-dichloro-3N-(3a,4,5,6,7,7a-hexahydro-1H-2-benzimidazolyl)-3-thienylamine, and
2,4-dichloro-3N-(4-chloro-2-benzimidazolylamino)thiophene and their pharmaceutically acceptable salts.

5. A compound of the formula I and/or a pharmaceutically acceptable salt thereof as claimed in one or more of claims 1 to 4 for use as a medicament.

6. The use of a compound of the formula I and/or a pharmaceutically acceptable salt thereof as claimed in one or more of claims 1 to 4 for preparing a medicament for the treatment or prophylaxis of respiratory disorders, in particular respiratory disorders associated with sleeping, such as sleep apnea, or of snoring, for the treatment or prophylaxis of acute and chronic renal diseases, in particular acute kidney failure or chronic kidney failure, or of disorders of intestinal function, for the treatment or prophylaxis of high blood pressure, in particular essential hypertension, of disorders of the central nervous system, in particular disorders which are the result of CNS overexcitability, such as epilepsy or centrally induced spasms, and of disorders of the central nervous system, in particular states of anxiety, depressions and psychoses, for the treatment or prophylaxis of acute and chronic damage and disorders of organs or tissue caused by ischemic events or by reperfusion events and indirect sequelae thereof, of arrhythmias, of atherosclerosis, of hypercholesterolemia, of diseases in which cell proliferation is a primary or secondary cause, of cancer, of prostate hypertrophy or prostate hyperplasia, of fibrotic disorders of internal organs, of cardiac insufficiency or of congestive heart failure, of thromboses, of disorders of gall function, of infection by ectoparasites, of disorders which are the result of endothelial dysfunction, of intermittent claudication, of protozoa disorders, in particular malaria, for the treatment of states of shock, diabetes and late diabetic damage, acute or chronic inflammatory disorders or for preparing a medicament for the preservation and storage of transplants for surgical interventions, for preparing a medicament for use in surgical operations and organ transplantations, for preparing a medicament for preventing age-related tissue change, for maintaining health and prolonging life, for reducing cytotoxic effects or for use as diagnostics.

7. The use of a compound of the formula I and/or a pharmaceutically acceptable salt thereof as claimed in one or more of claims 1 to 4 in combination with other drugs or active compounds for preparing a medicament for the treatment or prophylaxis of respiratory disorders, in particular respiratory disorders associated with sleeping, such as sleep apnea, or of snoring, for the treatment or prophylaxis of acute and chronic renal diseases, in particular acute kidney failure or chronic kidney failure, or of disorders of intestinal function, for the treatment or prophylaxis of high blood pressure, in particular essential hypertension, of disorders of the central nervous system, in particular disorders which are the result of CNS overexcitability, such as epilepsy or centrally induced spasms, and of disorders of the central nervous system, in particular states of anxiety, depressions and psychoses, for the treatment or prophylaxis of acute and chronic damage and disorders of organs or tissues caused by ischemic events or by reperfusion events and indirect sequelae thereof, of arrhythmias, of atherosclerosis, of hypercholesterolemia, of diseases in which cell proliferation is a primary or secondary cause, of cancer, of prostate hypertrophy or prostate hyperplasia, of fibrotic disorders of internal organs, of cardiac insufficiency or of congestive heart failure, of thromboses, of disorders of gall function, of infection by ectoparasites, of disorders which are the result of endothelial dysfunction, of intermittent claudication, of protozoa disorders, in particular malaria, for the treatment of states of shock, diabetes and late diabetic damage, acute or chronic inflammatory disorders or for preparing a medicament for the preservation and storage of transplants for surgical interventions, for preparing a medicament for use in surgical operations and organ transplantations, for preparing a medicament for preventing age-related tissue change, for maintaining health and prolonging life or for reducing cytotoxic effects.

8. The use of a compound of the formula I and/or a pharmaceutically acceptable salt thereof as claimed in one or more of claims 1 to 4 alone or in combination with other drugs or active compounds for preparing a medicament for the treatment or prophylaxis of respiratory disorders, in particular respiratory disorders associated with sleeping, such as sleep apnea.

9. The use of a compound of the formula I and/or a pharmaceutically acceptable salt thereof as claimed in one or more of claims 1 to 4 alone or in combination with other drugs or active compounds for preparing a medicament for the treatment of prophylaxis or snoring.

10. The use of a compound of the formula I and/or a pharmaceutically acceptable salt thereof as claimed in one or more of claims 1 to 4 alone or in combination with other drugs or active compounds for preparing a medicament for the treatment or prophylaxis of acute and chronic renal diseases, in particular acute kidney failure or chronic kidney failure.

11. The use of a compound of the formula I and/or a pharmaceutically acceptable salt thereof as claimed in one or more of claims 1 to 4 alone or in combination with other drugs or active compounds for preparing a medicament for the treatment or prophylaxis of disorders of intestinal function.

12. A medicament for human, veterinary or phytoprotective use, comprising an effective amount of a compound of the formula I and/or a pharmaceutically acceptable salt thereof as claimed in one or more of claims 1 to 4, together with pharmaceutically acceptable carriers and additives.

13. A medicament for human, veterinary or phytoprotective use, comprising an effective amount of a compound of the formula I and/or a pharmaceutically acceptable salt thereof as claimed in one or more of claims 1 to 4, together with pharmaceutically acceptable carriers and additives, in combination with other pharmacologically active compounds or drugs.

## Revendications

1. Composés de formule I, 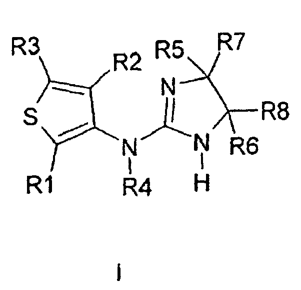
dans laquelle :
R1 et R2 représentent indépendamment l'un de l'autre H, F, CI, Br, I, CN, NO₂, -(X)ₙ-C_{q}H_{2q}-Z, un groupe cycloalkyle ayant 3, 4, 5 ou 6 atomes de carbone, alcényle ayant 2, 3 ou 4 atomes de carbone, alcénylalkyle ayant 3 ou 4 atomes de carbone, éthynyle ou alkylalcynyle ayant 3 ou 4 atomes de carbone ;
n est zéro ou 1 ;
X représente un atome d'oxygène, NH, N-CH₃ ou S(O)ₖ ;
k est zéro, 1 ou 2 ;
q est zéro, 1, 2, 3, 4, 5 ou 6 ;
Z représente un atome d'oxygène ou CₘF₂ₘ₊₁ ;
m est zéro, 1, 2, 3 ou 4 ;
R3 représente un atome d'hydrogène, un groupe méthyle, F, CI, Br, I, CN, S(O)ₖ-CH₃, -NO₂ ou -O-CH₃ ;
k est zéro, 1 ou 2 ;
R4 représente un atome d'hydrogène, un groupe cycloalkyle ayant 3, 4, 5 ou 6 atomes de carbone, alcényle ayant 2, 3 ou 4 atomes de carbone, alcénylalkyle ayant 3 ou 4 atomes de carbone, éthynyle ou alkylalcynyle ayant 3 ou 4 atomes de carbone ou -CᵣH₂ᵣY ;
r est zéro, 1, 2, 3 ou 4 ;
Y représente un atome d'hydrogène ou le groupe trifluorométhyle ;
R5 et R6 représentent des atomes d'hydrogène ou forment ensemble une liaison ;
R7 et R8 représentent, indépendamment l'un de l'autre, un groupe alkyle en C₃-C₅, alcényle en C₂-C₅, alcynyle en C₂-C₅, cycloalkyle en C₃-C₆ ou cycloalcényle en C₄-C₆
ou
R7 et R8 représentent une chaîne alkylène constituée de 3 à 8 atomes de carbone, dont les atomes d'hydrogène peuvent être partiellement ou totalement remplacés par des atomes de fluor;
ou
R7 et R8 forment ensemble un radical 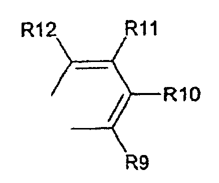
R5 et R6 formant ensemble une liaison ;
R10 et R11 représentent, indépendamment l'un de l'autre, un atome d'hydrogène, de fluor, chlore, brome, un groupe méthyle, CN, OH, -O-CH₃, NO₂, NH₂ ou -CF₃ ;
R9 et R12 représentent un atome d'hydrogène ou de fluor ;
ou
l'un des substituants R9 et R12 représente un atome d'hydrogène ;
et l'autre représente F, Cl, Br, I, CN, NO₂, COOH, CO-NR13R14, SO₂-NR13R14, un groupe alcényle ayant 2, 3 ou 4 atomes de carbone, alcénylalkyle ayant 3 ou 4 atomes de carbone, éthynyle, alkylalcynyle ayant 3 ou 4 atomes de carbone ou -(X)ₙ-CqH₂q-Z ;
R13 et R14 sont identiques ou différents et représentent un atome d'hydrogène ou un groupe alkyle ayant 1, 2, 3 ou 4 atomes de carbone ;
ou
R13 et R14 peuvent former ensemble, avec l'atome d'azote auquel ils sont fixés, un cycle saturé à 5, 6 ou 7 chaînons ;
n est zéro ou 1 ;
X représente un atome d'oxygène, NH, N-CH₃ ou S(O)ₖ ;
k est zéro, 1 ou 2 ;
q est zéro, 1, 2, 3, 4, 5 ou 6 ;
et
Z représente un atome d'hydrogène ou CₘF₂ₘ₊₁ ;
m est zéro, 1, 2, 3 ou 4 ;
ainsi que leurs sels pharmaceutiquement acceptables et leurs sels avec l'acide trifluoroacétique.

2. Composés selon la revendication 1, dans lesquels :
R1 et R2 représentent indépendamment l'un de l'autre H, F, Cl, Br, CH₃, CF₃, SO₂CH₃ ou SO₂NH₂ ; au maximum un des substituants R1 et R2 étant un atome d'hydrogène ;
R3 représente un atome d'hydrogène, F ou Cl ;
R4 représente un atome d'hydrogène, un groupe alkyle ayant 1, 2, 3 ou 4 atomes de carbone ou cyclopropyle ;
R5 et R6 représentent des atomes d'hydrogène ou forment ensemble une liaison ;
R7 et R8 représentent une chaîne alkylène constituée de 3, 4, 5, 6, 7 ou 8 atomes de carbone ;
ou
R7 et R8 forment ensemble un radical 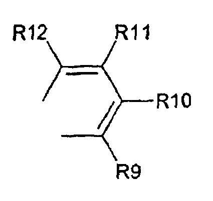
R5 et R6 formant ensemble une liaison ;
R10 et R11 représentent, indépendamment l'un de l'autre, un atome d'hydrogène, OH, un atome de fluor ou de chlore ;
R9 et R12 représentent un atome d'hydrogène ;
ou
l'un des substituants R9 et R12 représente un atome d'hydrogène;
et l'autre représente F, CI, Br, CN, COOH, CO-NR13R14, SO₂-NR13R14 ou -(X)ₙ-C_{q}H_{2q}-Z ;
R13 et R14 sont identiques ou différents et représentent un atome d'hydrogène ou le groupe méthyle ;
n est zéro ou 1 ;
X représente un atome d'oxygène, NH, N-CH₃ ou S(O)ₖ ;
k est zéro, 1 ou 2 ;
q est zéro, 1, 2, 3 ou 4 ;
Z représente un atome d'hydrogène ou CF₃ ;
ainsi que leurs sels pharmaceutiquement acceptables et leurs sels avec l'acide trifluoroacétique.

3. Composés selon la revendication 1 et/ou la revendication 2, dans lesquels :
R1 et R2 représentent indépendamment l'un de l'autre F, CI, Br, CH₃ ou CF₃ ;
R3 représente un atome d'hydrogène ;
R4 représente un atome d'hydrogène, le groupe méthyle ou éthyle ;
R5 et R6 représentent des atomes d'hydrogène ou forment ensemble une liaison ;
R7 et R8 représentent une chaîne alkylène constituée de 3, 4, 5, 6, 7 ou 8 atomes de carbone ;
ou
R7 et R8 forment ensemble un radical 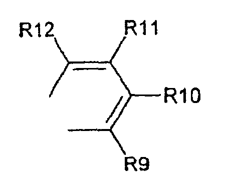
R5 et R6 formant ensemble une liaison ;
R10 et R11 représentent, indépendamment l'un de l'autre, un atome d'hydrogène, OH ou un atome de fluor ;
R9 et R12 représentent un atome d'hydrogène ;
ou
l'un des substituants R9 et R12 représente un atome d'hydrogène ; et l'autre représente F, CI, Br ou -(X)ₙ-C_{q}H_{2q}-Z ;
n est zéro ou 1 ;
X représente un atome d'oxygène, NH, N-CH₃ ou S(O)ₖ ;
k est zéro, 1 ou 2 ;
q est zéro ou 1 ;
Z représente un atome d'hydrogène ou CF₃ ;
ainsi que leurs sels pharmaceutiquement acceptables et leurs sels avec l'acide trifluoroacétique.

4. Composés de formule I selon une ou plusieurs des revendications 1, 2 et 3, choisis dans le groupe constitué par
la trans-R,R-2-chloro-3N-(3a,4,5,6,7,7a-hexahydro-1H-2-benzimidazolyl)-4-méthyl-3-thiénylamine,
la trans-R,R-2-bromo-3N-(3a,4,5,6,7,7a-hexahydro-1H-2-benzimidazolyl)-4-méthyl-3-thiénylamine,
la 2-chloro-3N-(2-benzimidazolyl)-4-méthyl-3-thiénylamine,
la 2-bromo-3N-(2-benzimidazolyl)-4-méthyl-3-thiénylamine,
la 2-chloro-3N-(4-méthyl-2-benzimidazolyl)-4-méthyl-3-thiénylamine,
la 2-chloro-3N-(5-fluoro-2-benzimidazolyl)-4-méthyl-3-thiénylamine,
le 2-chloro-3N-(4-chloro-2-benzimidazolyl-amino)-4-méthylthiophène,
le 2-bromo-3N-(4-chloro-2-benzimidazolyl-amino)-4-méthylthiophène,
le 2-bromo-3N-(4-fluoro-2-benzimidazolyl-amino)-4-méthylthiophène,
le 2-chloro-3N-(4-fluoro-2-benzimidazolyl-amino)-4-méthylthiophène,
le 2-chloro-3N-(4-hydroxy-2-benzimidazolyl-amino)-4-méthylthiophène,
la (1H-benzimidazol-2-yl)-2-chloro-4-méthyl-thiophén-3-yl)-méthylamine,
la (2-bromo-4-méthyl-thiophén-3-yl)-(5-fluoro-1 H-benzimidazol-2-yl)-amine,
le 2-chloro-3N-(2-benzimidazolylamino)-4-méthylthiophène,
le 2,4-dichloro-3N-(2-benzimidazolylamino)thiophène,
le 2-bromo-4-chloro-3N-(2-benzimidazolyl-amino)thiophène,
le 2,4-dichloro-3N-(4-méthyl-2-benzimidazolylamino)thiophène,
la trans-R,R-2,4-dichloro-3N-(3a,4,5,6,7,7a-hexahydro-1H-2-benzimidazolyl)-3-thiénylamine,
et
le 2,4-dichloro-3N-(4-chloro-2-benzimidazolylamino)thiophène,
ainsi que leurs sels pharmaceutiquement acceptables.

5. Composés de formule I et/ou leurs sels pharmaceutiquement acceptables selon une ou plusieurs des revendications 1 à 4, pour utilisation en tant que médicament.

6. Utilisation d'un composé de formule I et/ou de ses sels pharmaceutiquement acceptables selon une ou plusieurs des revendications 1 à 4, pour la fabrication d'un médicament destiné à la prévention et au traitement de troubles de l'impulsion respiratoire, en particulier de troubles respiratoires dus au sommeil, tels que les apnées du sommeil, ou du ronflement, à la prévention ou au traitement de néphropathies aiguës et chroniques, en particulier de l'insuffisance rénale aiguë ou de l'insuffisance rénale chronique, ou de troubles de la fonction intestinale, à la prévention ou au traitement de l'hypertension artérielle, en particulier de l'hypertonie essentielle, de maladies du système nerveux central, en particulier de maladies qui résultent d'une hyperexcitabilité du SNC, telles que l'épilepsie ou les convulsions d'origine centrale, et de maladies du système nerveux central, en particulier d'états anxieux, de dépressions et de psychoses, à la prévention ou au traitement de lésions, maladies et séquelles indirectes, aiguës et chroniques, d'organes et de tissus, qui sont provoquées par des incidents d'ischémie et des incidents de reperfusion, d'arythmies, de l'athérosclérose, de l'hypercholestérolémie, de maladies dans lesquelles la prolifération cellulaire représente une cause primaire ou secondaire, du cancer, de l'hypertrophie de la prostate ou de l'hyperplasie de la prostate, de maladies fibreuses d'organes internes, de l'insuffisance cardiaque ou de la Congestive Heart Failure, de thromboses, de troubles de la fonction biliaire, de l'attaque par des ectoparasites, de maladies faisant suite à un dysfonctionnement endothélial, de la claudication intermittente, de maladies qui sont provoquées par des protozoaires, notamment du paludisme, au traitement d'états de choc, du diabète et de lésions tardives diabétiques, de maladies inflammatoires aiguës ou chroniques, ou pour la fabrication d'un médicament destiné à la conservation et au stockage de transplants pour des gestes chirurgicaux, pour la fabrication d'un médicament destiné à l'utilisation dans des interventions chirurgicales et des greffes d'organes, pour la fabrication d'un médicament destiné à empêcher une altération tissulaire due à l'âge, pour le maintien de la santé et le prolongement de la vie, pour éviter des effets cytotoxiques ou pour l'utilisation en tant qu'agents de diagnostic.

7. Utilisation d'un composé de formule I et/ou de ses sels pharmaceutiquement acceptables selon une ou plusieurs des revendications 1 à 4, en association avec d'autres principes actifs ou substances actives pour la fabrication d'un médicament destiné à la prévention et au traitement de troubles de l'impulsion respiratoire, en particulier de troubles respiratoires dus au sommeil, tels que les apnées du sommeil ou du ronflement, à la prévention ou au traitement de néphropathies aiguës et chroniques, en particulier de l'insuffisance rénale aiguë ou de l'insuffisance rénale chronique, ou de troubles de la fonction intestinale, à la prévention ou au traitement de l'hypertension artérielle, en particulier de l'hypertonie essentielle, de maladies du système nerveux central, en particulier de maladies qui résultent d'une hyperexcitabilité du SNC, telles que l'épilepsie ou les convulsions d'origine centrale, et de maladies du système nerveux central, en particulier d'états anxieux, de dépressions et de psychoses, à la prévention ou au traitement de lésions, maladies et séquelles indirectes, aiguës et chroniques, d'organes et de tissus, qui sont provoquées par des incidents d'ischémie ou par des incidents de reperfusion, d'arythmies, de l'athérosclérose, de l'hypercholestérolémie, de maladies dans lesquelles la prolifération cellulaire représente une cause primaire ou secondaire, du cancer, de l'hypertrophie de la prostate ou de l'hyperplasie de la prostate, de maladies fibreuses d'organes internes, de l'insuffisance cardiaque ou de la *Congestive Heart Failure,* de thromboses, de troubles de la fonction biliaire, de l'attaque par des ectoparasites, de maladies faisant suite à un dysfonctionnement endothélial, de la claudication intermittente, de maladies qui sont provoquées par des protozoaires, notamment du paludisme, au traitement d'états de choc, du diabète et de lésions tardives diabétiques, de maladies inflammatoires aiguës ou chroniques, ou pour la fabrication d'un médicament destiné à la conservation et au stockage de transplants pour des gestes chirurgicaux, pour la fabrication d'un médicament destiné à l'utilisation dans des interventions chirurgicales et des greffes d'organes, pour la fabrication d'un médicament destiné à empêcher une altération tissulaire due à l'âge, pour le maintien de la santé et le prolongement de la vie, pour éviter des effets cytotoxiques ou pour l'utilisation en tant qu'agents de diagnostic.

8. Utilisation d'un composé de formule I et/ou de ses sels pharmaceutiquement acceptables selon une ou plusieurs des revendications 1 à 4, seul ou en association avec d'autres principes actifs ou substances actives, pour la fabrication d'un médicament destiné à la prévention ou au traitement de troubles de l'impulsion respiratoire, en particulier de troubles respiratoires dus au sommeil, tels que les apnées du sommeil.

9. Utilisation d'un composé de formule I et/ou de ses sels pharmaceutiquement acceptables selon une ou plusieurs des revendications 1 à 4, seul ou en association avec d'autres principes actifs ou substances actives, pour la fabrication d'un médicament destiné à la prévention ou au traitement du ronflement.

10. Utilisation d'un composé de formule I et/ou de ses sels pharmaceutiquement acceptables selon une ou plusieurs des revendications 1 à 4, seul ou en association avec d'autres principes actifs ou substances actives, pour la fabrication d'un médicament destiné à la prévention ou au traitement de néphropathies aiguës et chroniques, en particulier de l'insuffisance rénale aiguë ou de l'insuffisance rénale chronique.

11. Utilisation d'un composé de formule I et/ou de ses sels pharmaceutiquement acceptables selon une ou plusieurs des revendications 1 à 4, seul ou en association avec d'autres principes actifs ou substances actives, pour la fabrication d'un médicament destiné à la prévention ou au traitement de troubles de la fonction intestinale.

12. Médicament pour l'utilisation humaine, vétérinaire ou phytoprotectrice, contenant une quantité efficace d'un composé de formule I et/ou de ses sels pharmaceutiquement acceptables selon une ou plusieurs des revendications 1 à 4, conjointement avec des additifs et véhicules pharmaceutiquement acceptables.

13. Médicament pour l'utilisation humaine, vétérinaire ou phytoprotectrice, contenant une quantité efficace d'un composé de formule I et/ou de ses sels pharmaceutiquement acceptables selon une ou plusieurs des revendications 1 à 4, conjointement avec des additifs et véhicules pharmaceutiquement acceptables, en association avec d'autres principes actifs ou substances actives.
